# EUROPEAN PATENT APPLICATION

(11) **EP 4 831 202 A1**
(43) Date of publication of application: **28.10.2026**
(21) Application number: 24906548.3
(22) Date of filing: 20.12.2024
(51) Int. Cl.: C07K 16/28, C12N 5/10, A61K 39/395, A61P 35/00, C07K 16/46

(54) **ANTI-CD3 AND ANTI-CD3 MULTISPECIFIC ANTIBODIES, AND USE**

(30) Priority: 21.12.2023 CN 202311778073
(71) Applicant: Shanghai Junshi Biosciences Co., Ltd., Shanghai 201203 (CN); Shanghai Junshi Biotechnology Co., Ltd., Shanghai 201400 (CN)
(72) Inventor: LI, Shuai, Shanghai 201203 (CN); XU, Weili, Shanghai 201203 (CN); LI, Li, Shanghai 201203 (CN); LI, Xueting, Shanghai 201203 (CN)
(74) Representative: Zanoli, Enrico
(86) International application number: PCT/CN2024/141038
(87) International publication number: WO 2025/131075

(57) **Abstract**

Provided are an antibody specifically binding to human CD3 or an antigen-binding fragment thereof, and a multispecific antibody targeting CD3 and other antigens (e.g. tumor-associated antigens). Also provided are nucleic acid molecules encoding the antibodies, vectors, and host cells for expressing the antibodies, and treatment and diagnosis methods and uses of the antibodies or the antigen-binding fragment thereof.

## Description

### Cross-Reference to Related Application

The present application claims priority to the Chinese patent application with application number CN 202311778073.9 (filed on December 21, 2023). The entire contents of the patent application are incorporated into the present application by reference.

### Technical Field

The present invention relates to a class of antibodies that specifically bind to human CD3, and multispecific antibodies that simultaneously bind to CD3 and tumor-associated antigens.

### Background Art

T cells are a very important type of immune cell in the human body and play a critical role in anti-tumor immunity. The activation of T cells requires the involvement of various cell membrane surface molecules, such as the T cell receptor (TCR), CD3, and CD28 and so on. The TCR is responsible for receiving antigen stimulation but lacks the functional structure to transmit signals into the cell. Therefore, the TCR must form a complex with CD3 through non-covalent binding (the TCR/CD3 complex) to jointly accomplish T cell activation. The intracellular region of the CD3 molecule contains an immunoreceptor tyrosine-based activation motif (ITAM), which mediates T cell activation, proliferation, and survival.

Bispecific antibodies (BsAbs) are a class of bifunctional antibodies that can specifically bind to two different antigens or two distinct epitopes on the same antigen. Bispecific T-cell engagers (BiTEs) are representative non-IgG-like bispecific antibodies, formed by linking two single-chain variable fragments (scFvs), one recognizing the T-cell CD3 molecule and the other recognizing a target cell antigen. By recruiting T cells near the tumor tissue, BiTEs create an immune synapse between tumor cells and T cells, thereby mediating T-cell killing of tumor cells. Based on this drug design concept, the US-based company Amgen developed Blinatumomab, the world's first dual-targeting antibody drug for treating acute lymphoblastic leukemia (ALL). Since its approval in 2014, Blinatumomab has achieved remarkable clinical outcomes. Subsequently, several CD3-based bispecific antibodies have been approved, including Mosunetuzumab (a CD3xCD20 bispecific antibody by Roche), Teclistamab (a CD3xBCMA bispecific antibody by Johnson & Johnson), and Talquetamab (a CD3xGPRC5D bispecific antibody by Johnson & Johnson).

Although CD3 bispecific antibodies have demonstrated excellent therapeutic effects in hematologic malignancies, the incidence of cytokine release syndrome (CRS) remains excessively high, sometimes leading to severe adverse reactions. The high incidence of CRS is associated with excessively strong activity of CD3 antibodies. Therefore, it is essential to select a CD3 antibody with an appropriate level of activity for the development of bispecific antibodies.

### Contents of the Invention

The purpose of the present application is to provide an antibody or antigen-binding fragment thereof capable of specifically binding to CD3. Based on this, further provided are a multispecific antibody targeting CD3 and other antigens (such as CD19), an isolated nucleic acid molecule encoding the antibody or antigen-binding fragment thereof or the multispecific antibody of the present application, an expression vector and a host cell for expressing the antibody or antigen-binding fragment thereof or the multispecific antibody of the present application, as well as use of the antibody or antigen-binding fragment thereof or the multispecific antibody of the present application.

The multispecific antibody (e.g., bispecific antibody) of the present application exhibits activation of T cells, thereby specifically killing tumor cells, and have significantly reduced cytokine release, thus demonstrating significantly improved safety.

In one aspect, the present invention provides an antibody or antigen-binding fragment thereof capable of specifically binding to CD3, wherein the antibody or antigen-binding fragment thereof comprises: HCDR1 with an amino acid sequence as set forth in SEQ ID NO: 16, 54, 1, 9, or 29; HCDR2 with an amino acid sequence as set forth in SEQ ID NO: 55, 56, 17, 2, 10, 24, 30, or 37; HCDR3 with an amino acid sequence as set forth in SEQ ID NO: 18, 3, 11, 25, 31, or 38; LCDR1 with an amino acid sequence as set forth in SEQ ID NO: 42, 43, 20, 5, 13, or 33; LCDR2 with an amino acid sequence as set forth in SEQ ID NO: 21, 45, 46, 6, 14, 27, 34, or 44; and LCDR3 with an amino acid sequence as set forth in SEQ ID NO: 22, 7, 35, or 40.

In one aspect, the present invention provides an antibody or antigen-binding fragment thereof capable of specifically binding to CD3, wherein the antibody or antigen-binding fragment thereof comprises a heavy chain variable region and/or a light chain variable region:
the heavy chain variable region comprises:
   (I) HCDR1, HCDR2, and HCDR3 with amino acid sequences as set forth in SEQ ID NO: 16, SEQ ID NO: 55, and SEQ ID NO: 18, respectively; or HCDR1, HCDR2, and HCDR3 having 1, 2, or 3 amino acid differences from the amino acid sequences as set forth in SEQ ID NO: 16, SEQ ID NO: 55, and SEQ ID NO: 18, respectively;
   (II) HCDR1, HCDR2, and HCDR3 with amino acid sequences as set forth in SEQ ID NO: 16, SEQ ID NO: 56, and SEQ ID NO: 18, respectively; or HCDR1, HCDR2, and HCDR3 having 1, 2, or 3 amino acid differences from the amino acid sequences as set forth in SEQ ID NO: 16, SEQ ID NO: 56, and SEQ ID NO: 18, respectively;
   (III) HCDR1, HCDR2, and HCDR3 with amino acid sequences as set forth in SEQ ID NO: 54, SEQ ID NO: 56, and SEQ ID NO: 18, respectively; or HCDR1, HCDR2, and HCDR3 having 1, 2, or 3 amino acid differences from the amino acid sequences as set forth in SEQ ID NO: 54, SEQ ID NO: 56, and SEQ ID NO: 18, respectively;
   (IV) HCDR1, HCDR2, and HCDR3 with amino acid sequences as set forth in SEQ ID NO: 16, SEQ ID NO: 17, and SEQ ID NO: 18, respectively; or HCDR1, HCDR2, and HCDR3 having 1, 2, or 3 amino acid differences from the amino acid sequences as set forth in SEQ ID NO: 16, SEQ ID NO: 17, and SEQ ID NO: 18, respectively;
   (V) HCDR1, HCDR2, and HCDR3 with amino acid sequences as set forth in SEQ ID NO: 1, SEQ ID NO: 2, and SEQ ID NO: 3, respectively; or HCDR1, HCDR2, and HCDR3 having 1, 2, or 3 amino acid differences from the amino acid sequences as set forth in SEQ ID NO: 1, SEQ ID NO: 2, and SEQ ID NO: 3, respectively;
   (VI) HCDR1, HCDR2, and HCDR3 with amino acid sequences as set forth in SEQ ID NO: 9, SEQ ID NO: 10, and SEQ ID NO: 11, respectively; or HCDR1, HCDR2, and HCDR3 having 1, 2, or 3 amino acid differences from the amino acid sequences as set forth in SEQ ID NO: 9, SEQ ID NO: 10, and SEQ ID NO: 11, respectively;
   (VII) HCDR1, HCDR2, and HCDR3 with amino acid sequences as set forth in SEQ ID NO: 9, SEQ ID NO: 24, and SEQ ID NO: 25, respectively; or HCDR1, HCDR2, and HCDR3 having 1, 2, or 3 amino acid differences from the amino acid sequences as set forth in SEQ ID NO: 9, SEQ ID NO: 24, and SEQ ID NO: 25, respectively;
   (VIII) HCDR1, HCDR2, and HCDR3 with amino acid sequences as set forth in SEQ ID NO: 29, SEQ ID NO: 30, and SEQ ID NO: 31, respectively; HCDR1, HCDR2, and HCDR3 having 1, 2, or 3 amino acid differences from the amino acid sequences as set forth in SEQ ID NO: 29, SEQ ID NO: 30, and SEQ ID NO: 31, respectively; or
   (IX) HCDR1, HCDR2, and HCDR3 with amino acid sequences as set forth in SEQ ID NO: 9, SEQ ID NO: 37, and SEQ ID NO: 38, respectively; or HCDR1, HCDR2, and HCDR3 having 1, 2, or 3 amino acid differences from the amino acid sequences as set forth in SEQ ID NO: 9, SEQ ID NO: 37, and SEQ ID NO: 38, respectively;
the light chain variable region comprises:
   (I) LCDR1, LCDR2, and LCDR3 with amino acid sequences as set forth in SEQ ID NO: 42, SEQ ID NO: 21, and SEQ ID NO: 22, respectively; or LCDR1, LCDR2, and LCDR3 having 1, 2, or 3 amino acid differences from the amino acid sequences as set forth in SEQ ID NO: 42, SEQ ID NO: 21, and SEQ ID NO: 22, respectively;
   (II) LCDR1, LCDR2, and LCDR3 with amino acid sequences as set forth in SEQ ID NO: 42, SEQ ID NO: 44, and SEQ ID NO: 22, respectively; or LCDR1, LCDR2, and LCDR3 having 1, 2, or 3 amino acid differences from the amino acid sequences as set forth in SEQ ID NO: 42, SEQ ID NO: 44, and SEQ ID NO: 22, respectively;
   (III) LCDR1, LCDR2, and LCDR3 with amino acid sequences as set forth in SEQ ID NO: 43, SEQ ID NO: 45, and SEQ ID NO: 22, respectively; or LCDR1, LCDR2, and LCDR3 having 1, 2, or 3 amino acid differences from the amino acid sequences as set forth in SEQ ID NO: 43, SEQ ID NO: 45, and SEQ ID NO: 22, respectively;
   (IV) LCDR1, LCDR2, and LCDR3 with amino acid sequences as set forth in SEQ ID NO: 43, SEQ ID NO: 46, and SEQ ID NO: 22, respectively; or LCDR1, LCDR2, and LCDR3 having 1, 2, or 3 amino acid differences from the amino acid sequences as set forth in SEQ ID NO: 43, SEQ ID NO: 46, and SEQ ID NO: 22, respectively;
   (V) LCDR1, LCDR2, and LCDR3 with amino acid sequences as set forth in SEQ ID NO: 20, SEQ ID NO: 21, and SEQ ID NO: 22, respectively; or LCDR1, LCDR2, and LCDR3 having 1, 2, or 3 amino acid differences from the amino acid sequences as set forth in SEQ ID NO: 20, SEQ ID NO: 21, and SEQ ID NO: 22, respectively;
   (VI) LCDR1, LCDR2, and LCDR3 with amino acid sequences as set forth in SEQ ID NO: 5, SEQ ID NO: 6, and SEQ ID NO: 7, respectively; or LCDR1, LCDR2, and LCDR3 having 1, 2, or 3 amino acid differences from the amino acid sequences as set forth in SEQ ID NO: 5, SEQ ID NO: 6, and SEQ ID NO: 7, respectively;
   (VII) LCDR1, LCDR2, and LCDR3 with amino acid sequences as set forth in SEQ ID NO: 13, SEQ ID NO: 14, and SEQ ID NO: 7, respectively; or LCDR1, LCDR2, and LCDR3 having 1, 2, or 3 amino acid differences from the amino acid sequences as set forth in SEQ ID NO: 13, SEQ ID NO: 14, and SEQ ID NO: 7, respectively;
   (VIII) LCDR1, LCDR2, and LCDR3 with amino acid sequences as set forth in SEQ ID NO: 13, SEQ ID NO: 27, and SEQ ID NO: 7, respectively; or LCDR1, LCDR2, and LCDR3 having 1, 2, or 3 amino acid differences from the amino acid sequences as set forth in SEQ ID NO: 13, SEQ ID NO: 27, and SEQ ID NO: 7, respectively;
   (IX) LCDR1, LCDR2, and LCDR3 with amino acid sequences as set forth in SEQ ID NO: 33, SEQ ID NO: 34, and SEQ ID NO: 35, respectively; or LCDR1, LCDR2, and LCDR3 having 1, 2, or 3 amino acid differences from the amino acid sequences as set forth in SEQ ID NO: 33, SEQ ID NO: 34, and SEQ ID NO: 35, respectively; or
   (X) LCDR1, LCDR2, and LCDR3 with amino acid sequences as set forth in SEQ ID NO: 13, SEQ ID NO: 6, and SEQ ID NO: 40, respectively; or LCDR1, LCDR2, and LCDR3 having 1, 2, or 3 amino acid differences from the amino acid sequences as set forth in SEQ ID NO: 13, SEQ ID NO: 6, and SEQ ID NO: 40, respectively.

In some embodiments, the antibody or antigen-binding fragment thereof according to the present invention comprises:
(I) a heavy chain variable region, which comprises HCDR1, HCDR2, and HCDR3 with amino acid sequences as set forth in SEQ ID NO: 16, SEQ ID NO: 55, and SEQ ID NO: 18, respectively; and a light chain variable region, which comprises LCDR1, LCDR2, and LCDR3 with amino acid sequences as set forth in SEQ ID NO: 42, SEQ ID NO: 21, and SEQ ID NO: 22, respectively;
(II) a heavy chain variable region, which comprises HCDR1, HCDR2, and HCDR3 with amino acid sequences as set forth in SEQ ID NO: 16, SEQ ID NO: 56, and SEQ ID NO: 18, respectively; and a light chain variable region, which comprises LCDR1, LCDR2, and LCDR3 with amino acid sequences as set forth in SEQ ID NO: 43, SEQ ID NO: 45, and SEQ ID NO: 22, respectively;
(III) a heavy chain variable region, which comprises HCDR1, HCDR2, and HCDR3 with amino acid sequences as set forth in SEQ ID NO: 16, SEQ ID NO: 56, and SEQ ID NO: 18, respectively; and a light chain variable region, which comprises LCDR1, LCDR2, and LCDR3 with amino acid sequences as set forth in SEQ ID NO: 43, SEQ ID NO: 46, and SEQ ID NO: 22, respectively;
(IV) a heavy chain variable region, which comprises HCDR1, HCDR2, and HCDR3 with amino acid sequences as set forth in SEQ ID NO: 16, SEQ ID NO: 17, and SEQ ID NO: 18, respectively; and a light chain variable region, which comprises LCDR1, LCDR2, and LCDR3 with amino acid sequences as set forth in SEQ ID NO: 20, SEQ ID NO: 21, and SEQ ID NO: 22, respectively;
(V) a heavy chain variable region, which comprises HCDR1, HCDR2, and HCDR3 with amino acid sequences as set forth in SEQ ID NO: 1, SEQ ID NO: 2, and SEQ ID NO: 3, respectively; and a light chain variable region, which comprises LCDR1, LCDR2, and LCDR3 with amino acid sequences as set forth in SEQ ID NO: 5, SEQ ID NO: 6, and SEQ ID NO: 7, respectively;
(VI) a heavy chain variable region, which comprises HCDR1, HCDR2, and HCDR3 with amino acid sequences as set forth in SEQ ID NO: 9, SEQ ID NO: 10, and SEQ ID NO: 11, respectively; and a light chain variable region, which comprises LCDR1, LCDR2, and LCDR3 with amino acid sequences as set forth in SEQ ID NO: 13, SEQ ID NO: 14, and SEQ ID NO: 7, respectively;
(VII) a heavy chain variable region, which comprises HCDR1, HCDR2, and HCDR3 with amino acid sequences as set forth in SEQ ID NO: 9, SEQ ID NO: 24, and SEQ ID NO: 25, respectively; and a light chain variable region, which comprises LCDR1, LCDR2, and LCDR3 with amino acid sequences as set forth in SEQ ID NO: 13, SEQ ID NO: 27, and SEQ ID NO: 7, respectively;
(VIII) a heavy chain variable region, which comprises HCDR1, HCDR2, and HCDR3 with amino acid sequences as set forth in SEQ ID NO: 29, SEQ ID NO: 30, and SEQ ID NO: 31, respectively; and a light chain variable region, which comprises LCDR1, LCDR2, and LCDR3 with amino acid sequences as set forth in SEQ ID NO: 33, SEQ ID NO: 34, and SEQ ID NO: 35, respectively; or
(IX) a heavy chain variable region, which comprises HCDR1, HCDR2, and HCDR3 with amino acid sequences as set forth in SEQ ID NO: 9, SEQ ID NO: 37, and SEQ ID NO: 38, respectively; and a light chain variable region, which comprises LCDR1, LCDR2, and LCDR3 with amino acid sequences as set forth in SEQ ID NO: 13, SEQ ID NO: 6, and SEQ ID NO: 40, respectively.

In some embodiments, the antibody of the present invention is a murine antibody or a chimeric antibody.

In some embodiments, the antibody or antigen-binding fragment thereof according to the present invention comprises:
(I) a heavy chain variable region, which comprises an amino acid sequence as set forth in any one of SEQ ID NOs: 19, 4, 12, 26, 32, and 39; and a light chain variable region, which comprises an amino acid sequence as set forth in any one of SEQ ID NOs: 23, 8, 15, 28, 36, and 41; or
(II) a heavy chain variable region, which comprises an amino acid sequence having at least 95%, 96%, 97%, 98%, or 99% sequence identity to the amino acid sequence as set forth in any one of SEQ ID NOs: 19, 4, 12, 26, 32, and 39; and a light chain variable region, which comprises an amino acid sequence having at least 95%, 96%, 97%, 98%, or 99% sequence identity to the amino acid sequence as set forth in any one of SEQ ID NOs: 23, 8, 15, 28, 36, and 41.

In some embodiments, the antibody or antigen-binding fragment thereof according to the present invention comprises a heavy chain variable region and a light chain variable region, wherein:
(I) the heavy chain variable region comprises an amino acid sequence as set forth in SEQ ID NO: 19, or comprises an amino acid sequence having at least 95%, 96%, 97%, 98%, or 99% sequence identity to the amino acid sequence as set forth in SEQ ID NO: 19; and the light chain variable region comprises an amino acid sequence as set forth in SEQ ID NO: 23, or comprises an amino acid sequence having at least 95%, 96%, 97%, 98%, or 99% sequence identity to the amino acid sequence as set forth in SEQ ID NO: 23;
(II) the heavy chain variable region comprises an amino acid sequence as set forth in SEQ ID NO: 4, or comprises an amino acid sequence having at least 95%, 96%, 97%, 98%, or 99% sequence identity to the amino acid sequence as set forth in SEQ ID NO: 4; and the light chain variable region comprises an amino acid sequence as set forth in SEQ ID NO: 8, or comprises an amino acid sequence having at least 95%, 96%, 97%, 98%, or 99% sequence identity to the amino acid sequence as set forth in SEQ ID NO: 8;
(III) the heavy chain variable region comprises an amino acid sequence as set forth in SEQ ID NO: 12, or comprises an amino acid sequence having at least 95%, 96%, 97%, 98%, or 99% sequence identity to the amino acid sequence as set forth in SEQ ID NO: 12; and the light chain variable region comprises an amino acid sequence as set forth in SEQ ID NO: 15, or comprises an amino acid sequence having at least 95%, 96%, 97%, 98%, or 99% sequence identity to the amino acid sequence as set forth in SEQ ID NO: 15;
(IV) the heavy chain variable region comprises an amino acid sequence as set forth in SEQ ID NO: 26, or comprises an amino acid sequence having at least 95%, 96%, 97%, 98%, or 99% sequence identity to the amino acid sequence as set forth in SEQ ID NO: 26; and the light chain variable region comprises an amino acid sequence as set forth in SEQ ID NO: 28, or comprises an amino acid sequence having at least 95%, 96%, 97%, 98%, or 99% sequence identity to the amino acid sequence as set forth in SEQ ID NO: 28;
(V) the heavy chain variable region comprises an amino acid sequence as set forth in SEQ ID NO: 32, or comprises an amino acid sequence having at least 95%, 96%, 97%, 98%, or 99% sequence identity to the amino acid sequence as set forth in SEQ ID NO: 32; and the light chain variable region comprises an amino acid sequence as set forth in SEQ ID NO: 36, or comprises an amino acid sequence having at least 95%, 96%, 97%, 98%, or 99% sequence identity to the amino acid sequence as set forth in SEQ ID NO: 36; or
(VI) the heavy chain variable region comprises an amino acid sequence as set forth in SEQ ID NO: 39, or comprises an amino acid sequence having at least 95%, 96%, 97%, 98%, or 99% sequence identity to the amino acid sequence as set forth in SEQ ID NO: 39; and the light chain variable region comprises an amino acid sequence as set forth in SEQ ID NO: 41, or comprises an amino acid sequence having at least 95%, 96%, 97%, 98%, or 99% sequence identity to the amino acid sequence as set forth in SEQ ID NO: 41.

In some embodiments, the antibody described in the present invention is a humanized antibody.

In some embodiments, the antibody or antigen-binding fragment thereof according to the present invention comprises:
(I) a heavy chain variable region, which comprises an amino acid sequence as set forth in any one of SEQ ID NOs: 57, 58, 59, 60, and 61; and
   a light chain variable region, which comprises an amino acid sequence as set forth in any one of SEQ ID NOs: 47, 48, 49, 50, 51, 52, and 53;
(II) a heavy chain variable region, which comprises an amino acid sequence having at least 95%, 96%, 97%, 98% or 99% sequence identity to the amino acid sequence as set forth in any one of SEQ ID NOs: 57, 58, 59, 60, and 61; and
   a light chain variable region, which comprises an amino acid sequence having at least 95%, 96%, 97%, 98% or 99% sequence identity to the amino acid sequence as set forth in any one of SEQ ID NOs: 47, 48, 49, 50, 51, 52, and 53; or
(III) a heavy chain variable region with an amino acid sequence as set forth in SEQ ID NO: 57 or 58, and a light chain variable region with an amino acid sequence as set forth in SEQ ID NO: 47;
   a heavy chain variable region with an amino acid sequence as set forth in SEQ ID NO: 58, and a light chain variable region with an amino acid sequence as set forth in SEQ ID NO: 51;
   a heavy chain variable region with an amino acid sequence as set forth in SEQ ID NO: 60, and a light chain variable region with an amino acid sequence as set forth in SEQ ID NO: 52 or 53; or
   a heavy chain variable region with an amino acid sequence as set forth in SEQ ID NO: 61, and a light chain variable region with an amino acid sequence as set forth in SEQ ID NO: 52 or 53.

In some embodiments, in the antibody or antigen-binding fragment thereof according to the present invention, the antibody is selected from the group consisting of murine antibody, chimeric antibody, humanized antibody, or fully human antibody.

In some embodiments, the antigen-binding fragment of the present invention is selected from the group consisting of Fab, Fab', F(ab')₂, Fv, scFv, and sdAb.

In some embodiments, the antibody or antigen-binding fragment thereof according to the present invention is any IgG subtype, such as IgG1, IgG2, IgG3, or IgG4, preferably IgG4 subtype.

In some embodiments, in the antibody or antigen-binding fragment thereof according to the present invention, the antibody comprises a heavy chain constant region with an amino acid sequence as set forth in SEQ ID NO: 63 and/or a light chain constant region with an amino acid sequence as set forth in SEQ ID NO: 62.

In another aspect, the present invention provides a multispecific antibody, which comprises an antigen-binding domain targeting CD3 and at least one antigen-binding domain targeting other antigen, wherein the antigen-binding domain targeting CD3 is selected from the antibody or antigen-binding fragment thereof as described in the present application, and the other antigen is selected from a tumor-associated antigen (TAA) or a tumor-specific antigen (TSA).

In some embodiments, the multispecific antibody of the present invention is a bispecific antibody, a trispecific antibody, or a tetraspecific antibody.

In some embodiments, the multispecific antibody of the present invention comprises the antigen-binding domain targeting CD3 and the antigen-binding domain targeting the tumor-associated antigen or tumor-specific antigen.

In another aspect, in the multispecific antibody of the present invention, the tumor-associated antigen or tumor-specific antigen is selected from the group consisting of CD19, CD20, CD22, EGFR, HER2, HER3, PSMA, EpCAM, EphA2, CD30, CD33, CD38, CD79b, CD123, CLDN18.2, MSLN, GUCY2C, AFP, PAP, TROP2, LRRC15, gp100, 5T4, CEA, UPK2, DLL3, PRAME, CDH17, CDH19, GPA33, FAP, GPRC5D, GPC3, B7-H3, CLL-1, LIV-1, ACPP, CLDN6, PSCA, ENPP3, PRLR, MUC16, MUC17, CCR5, GD2, GD3, Ras, LewisY, BORIS, NY-ESO-1, OY-TES1, TSHR, LY6K, FLt3, IGFR-1, CAIX, c-MET, TROP2, and any combination thereof.

In some embodiments, in the multispecific antibody of the present invention, the tumor-associated antigen is selected from CD19.

In some embodiments, the multispecific antibody of the present invention comprises the antigen-binding domain targeting CD3 and an antigen-binding domain targeting CD19; the antigen-binding domain targeting CD19 comprises a light chain variable region and a heavy chain variable region, wherein the heavy chain variable region comprises a sequence as set forth in SEQ ID NO: 70 or a variant thereof, and the light chain variable region comprises a sequence as set forth in SEQ ID NO: 71 or a variant thereof; preferably, the variant has at least 95%, 96%, 97%, 98%, or 99% sequence identity to the sequence from which it is derived.

In some embodiments, the antigen-binding domain targeting CD3 comprises HCDR1, HCDR2, and HCDR3 with amino acid sequences as set forth in SEQ ID NO: 16, SEQ ID NO: 56, and SEQ ID NO: 18, respectively, and LCDR1, LCDR2, and LCDR3 with amino acid sequences as set forth in SEQ ID NO: 43, SEQ ID NO: 46, and SEQ ID NO: 22, respectively.

In some embodiments, the multispecific antibody of the present invention is a bispecific antibody in the form of a common light chain. In some embodiments, the common light chain comprises LCDR1, LCDR2, and LCDR3 with amino acid sequences as set forth in SEQ ID NO: 42, SEQ ID NO: 21, and SEQ ID NO: 22, respectively; or LCDR1, LCDR2, and LCDR3 with amino acid sequences as set forth in SEQ ID NO: 43, SEQ ID NO: 45, and SEQ ID NO: 22, respectively; or LCDR1, LCDR2, and LCDR3 with amino acid sequences as set forth in SEQ ID NO: 43, SEQ ID NO: 46, and SEQ ID NO: 22, respectively; preferably, the common light chain comprises LCDR1, LCDR2, and LCDR3 with amino acid sequences as set forth in SEQ ID NO: 43, SEQ ID NO: 46, and SEQ ID NO: 22, respectively. In some embodiments, the common light chain comprises a sequence as set forth in SEQ ID NO: 47, 51, 52, or 53, for example, a sequence as set forth in SEQ ID NO: 53.

In some embodiments, the multispecific antibody of the present invention is a bispecific antibody in the form of a DuoBody, comprising:
i) peptide chain I-A, which comprises a VL of the antigen-binding domain targeting CD3, and a first light chain constant region (CL); preferably, the first light chain constant region is kappa;
ii) peptide chain I-B, which comprises a VH of the antigen-binding domain targeting CD3, and a first heavy chain constant region (CH); preferably, the first heavy chain constant region is IgG, such as IgG1, IgG2, IgG3, or IgG4;
iii) peptide chain I-C, which comprises a VH of the antigen-binding domain targeting TAA or TSA, and a second heavy chain constant region; preferably, the second heavy chain constant region is IgG, such as IgG1, IgG2, IgG3, or IgG4; and
iv) peptide chain I-D, which comprises a VL of the antigen-binding domain targeting TAA or TSA, and a second light chain constant region; preferably, the second light chain constant region is kappa.

In some embodiments, the Fc domains comprised in the peptide chains I-B and I-C comprise modifications (e.g., knob-into-hole modifications) to promote dimerization of I-B and I-C, respectively.

In some embodiments, the peptide chain I-C comprises a VH sequence as set forth in SEQ ID NO: 70 or a variant thereof, and the peptide chain I-D comprises a VL sequence as set forth in SEQ ID NO: 71 or a variant thereof; preferably, the variant has at least 95%, 96%, 97%, 98%, or 99% sequence identity to the sequence from which it is derived.

In some embodiments, the peptide chain I-B comprises a VH sequence as set forth in SEQ ID NO: 58, 57, 60, or 61 or a variant thereof, and the peptide chain I-A comprises a VL sequence as set forth in SEQ ID NO: 51, 47, 52, or 53 or a variant thereof; preferably, the variant has at least 95%, 96%, 97%, 98%, or 99% sequence identity to the sequence from which it is derived.

In some embodiments, the peptide chain I-B comprises a VH sequence as set forth in SEQ ID NO: 58 or 57 or a variant thereof, and the peptide chain I-A comprises a VL sequence as set forth in SEQ ID NO: 51 or 47 or a variant thereof; preferably, the variant has at least 95%, 96%, 97%, 98%, or 99% sequence identity to the sequence from which it is derived.

In some embodiments, the multispecific antibody of the present invention is a common light chain bispecific antibody, comprising:
i) peptide chain II-A, which is a common light chain and comprises a VL of the antigen-binding domain targeting CD3, and a light chain constant region (CL); preferably, the light chain constant region is kappa;
ii) peptide chain II-B, which comprises a VH of the antigen-binding domain targeting CD3, and a first heavy chain constant region (CH); preferably, the first heavy chain constant region is IgG, such as IgG1, IgG2, IgG3, or IgG4; and
iii) peptide chain II-C, which comprises a VH of the antigen-binding domain targeting TAA or TSA, and a second CH; preferably, the second CH is IgG, such as IgG1, IgG2, IgG3, or IgG4.

In some embodiments, the Fc domains comprised in the peptide chains II-B and II-C respectively comprise modifications to promote the dimerization of II-B and II-C.

In some embodiments, in the common light chain bispecific antibody: (1) the peptide chain II-A comprises a VL sequence as set forth in SEQ ID NO: 47 or a variant thereof, and the peptide chain II-B comprises a VH sequence as set forth in SEQ ID NO: 57 or a variant thereof; or (2) the peptide chain II-A comprises a VL sequence as set forth in SEQ ID NO: 47 or a variant thereof, and the peptide chain II-B comprises a VH sequence as set forth in SEQ ID NO: 58 or a variant thereof; or (3) the peptide chain II-A comprises a VL sequence as set forth in SEQ ID NO: 51 or a variant thereof, and the peptide chain II-B comprises a VH sequence as set forth in SEQ ID NO: 58 or a variant thereof; or (4) the peptide chain II-A comprises a VL sequence as set forth in SEQ ID NO: 52 or a variant thereof, and the peptide chain II-B comprises a VH sequence as set forth in SEQ ID NO: 60 or a variant thereof; or (5) the peptide chain II-A comprises a VL sequence as set forth in SEQ ID NO: 53 or a variant thereof, and the peptide chain II-B comprises a VH sequence as set forth in SEQ ID NO: 60 or a variant thereof; or (6) the peptide chain II-A comprises a VL sequence as set forth in SEQ ID NO: 52 or a variant thereof, and the peptide chain II-B comprises a VH sequence as set forth in SEQ ID NO: 61 or a variant thereof; or (7) the peptide chain II-A comprises a VL sequence as set forth in SEQ ID NO: 53 or a variant thereof, and the peptide chain II-B comprises a VH sequence as set forth in SEQ ID NO: 61 or a variant thereof; the variant in any one of (1) to (7) has at least 95%, 96%, 97%, 98%, or 99% sequence identity to the sequence from which it is derived.

In some embodiments, the peptide chain II-C comprises a VH sequence as set forth in SEQ ID NO: 70 or a variant thereof; the variant has at least 95%, 96%, 97%, 98%, or 99% sequence identity to the sequence from which it is derived.

In yet another aspect, the present invention provides an isolated nucleic acid molecule, which encodes the antibody or antigen-binding fragment thereof as described in the present application, or the heavy chain variable region and/or the light chain variable region thereof.

In some embodiments, the present invention provides an isolated nucleic acid molecule, which encodes the multispecific antibody as described in the present application or the polypeptide chain thereof.

In another aspect, the present invention provides an expression vector, which comprises the isolated nucleic acid molecule as described herein.

In yet another aspect, the present invention provides a host cell, which comprises the isolated nucleic acid molecule as described herein or the vector (e.g., expression vector) as described herein.

In yet another aspect, the present invention provides a method for preparing the antibody or antigen-binding fragment thereof or the multispecific antibody as described herein, wherein the method comprises culturing the host cell as described herein under conditions allowing protein expression, and recollecting the antibody or antigen-binding fragment thereof, or the multispecific antibody, from a culture of the cultured host cell.

In yet another aspect, the present invention provides a pharmaceutical composition, which comprises the antibody or antigen-binding fragment thereof as described herein, the multispecific antibody as described herein, the isolated nucleic acid molecule as described herein, the vector as described herein, or the host cell as described herein, and a pharmaceutically acceptable carrier and/or excipient.

In yet another aspect, the present invention provides use of the antibody or antigen-binding fragment thereof as described herein, the isolated nucleic acid molecule as described herein, the vector as described herein, the host cell as described herein, or the pharmaceutical composition as described herein, in the manufacture of a medicament for preventing and/or treating a disease.

In yet another aspect, the present invention provides a method for preventing and/or treating a disease, which comprises administering to a subject in need an effective dose of the antibody or antigen-binding fragment thereof as described herein, the multispecific antibody as described herein, the isolated nucleic acid molecule as described herein, the vector as described herein, the host cell as described herein, or the pharmaceutical composition as described herein.

In some embodiments, in the uses or methods as described herein, the disease is a tumor, an inflammatory disease, or an autoimmune disease; preferably, the tumor is selected from the group consisting of ovarian cancer, breast cancer, gastric cancer, cholangiocarcinoma, colorectal cancer, lung cancer, acute myeloid leukemia, chronic lymphocytic leukemia, melanoma, and colorectal cancer.

### Detailed description of the invention

### Binding portion to CD3

The present invention provides an antibody or antigen-binding fragment thereof capable of specifically binding to CD3. The terms "anti-CD3 antibody," "anti-CD3," "CD3 antibody," or "CD3-binding" refer to an antibody capable of binding to the CD3 protein or fragment thereof with sufficient affinity such that the antibody can be used as a diagnostic and/or therapeutic agent targeting CD3.

In some embodiments, the present invention provides an antibody or antigen-binding fragment thereof capable of specifically binding to CD3, wherein the antibody or antigen-binding fragment thereof comprises: HCDR1 with an amino acid sequence as set forth in SEQ ID NO: 16, 54, 1, 9, or 29; HCDR2 with an amino acid sequence as set forth in SEQ ID NO: 55, 56, 17, 2, 10, 24, 30, or 37; HCDR3 with an amino acid sequence as set forth in SEQ ID NO: 18, 3, 11, 25, 31, or 38; LCDR1 with an amino acid sequence as set forth in SEQ ID NO: 42, 43, 20, 5, 13, or 33; LCDR2 with an amino acid sequence as set forth in SEQ ID NO: 21, 45, 46, 6, 14, 27, 34, or 44; and LCDR3 with an amino acid sequence as set forth in SEQ ID NO: 22, 7, 35, or 40.

In some embodiments, the present invention provides an antibody or antigen-binding fragment thereof capable of specifically binding to CD3, wherein the antibody or antigen-binding fragment thereof comprises a heavy chain variable region and/or a light chain variable region:
the heavy chain variable region comprises:
   (I) HCDR1, HCDR2, and HCDR3 with amino acid sequences as set forth in SEQ ID NO: 16, SEQ ID NO: 55, and SEQ ID NO: 18, respectively; or HCDR1, HCDR2, and HCDR3 having 1, 2, or 3 amino acid differences from the amino acid sequences as set forth in SEQ ID NO: 16, SEQ ID NO: 55, and SEQ ID NO: 18, respectively;
   (II) HCDR1, HCDR2, and HCDR3 with amino acid sequences as set forth in SEQ ID NO: 16, SEQ ID NO: 56, and SEQ ID NO: 18, respectively; or HCDR1, HCDR2, and HCDR3 having 1, 2, or 3 amino acid differences from the amino acid sequences as set forth in SEQ ID NO: 16, SEQ ID NO: 56, and SEQ ID NO: 18, respectively;
   (III) HCDR1, HCDR2, and HCDR3 with amino acid sequences as set forth in SEQ ID NO: 54, SEQ ID NO: 56, and SEQ ID NO: 18, respectively; or HCDR1, HCDR2, and HCDR3 having 1, 2, or 3 amino acid differences from the amino acid sequences as set forth in SEQ ID NO: 54, SEQ ID NO: 56, and SEQ ID NO: 18, respectively;
   (IV) HCDR1, HCDR2, and HCDR3 with amino acid sequences as set forth in SEQ ID NO: 16, SEQ ID NO: 17, and SEQ ID NO: 18, respectively; or HCDR1, HCDR2, and HCDR3 having 1, 2, or 3 amino acid differences from the amino acid sequences as set forth in SEQ ID NO: 16, SEQ ID NO: 17, and SEQ ID NO: 18, respectively;
   (V) HCDR1, HCDR2, and HCDR3 with amino acid sequences as set forth in SEQ ID NO: 1, SEQ ID NO: 2, and SEQ ID NO: 3, respectively; or HCDR1, HCDR2, and HCDR3 having 1, 2, or 3 amino acid differences from the amino acid sequences as set forth in SEQ ID NO: 1, SEQ ID NO: 2, and SEQ ID NO: 3, respectively;
   (VI) HCDR1, HCDR2, and HCDR3 with amino acid sequences as set forth in SEQ ID NO: 9, SEQ ID NO: 10, and SEQ ID NO: 11, respectively; or HCDR1, HCDR2, and HCDR3 having 1, 2, or 3 amino acid differences from the amino acid sequences as set forth in SEQ ID NO: 9, SEQ ID NO: 10, and SEQ ID NO: 11, respectively;
   (VII) HCDR1, HCDR2, and HCDR3 with amino acid sequences as set forth in SEQ ID NO: 9, SEQ ID NO: 24, and SEQ ID NO: 25, respectively; or HCDR1, HCDR2, and HCDR3 having 1, 2, or 3 amino acid differences from the amino acid sequences as set forth in SEQ ID NO: 9, SEQ ID NO: 24, and SEQ ID NO: 25, respectively;
   (VIII) HCDR1, HCDR2, and HCDR3 with amino acid sequences as set forth in SEQ ID NO: 29, SEQ ID NO: 30, and SEQ ID NO: 31, respectively; or HCDR1, HCDR2, and HCDR3 having 1, 2, or 3 amino acid differences from the amino acid sequences as set forth in SEQ ID NO: 29, SEQ ID NO: 30, and SEQ ID NO: 31, respectively; or
   (IX) HCDR1, HCDR2, and HCDR3 with amino acid sequences as set forth in SEQ ID NO: 9, SEQ ID NO: 37, and SEQ ID NO: 38, respectively; or HCDR1, HCDR2, and HCDR3 having 1, 2, or 3 amino acid differences from the amino acid sequences as set forth in SEQ ID NO: 9, SEQ ID NO: 37, and SEQ ID NO: 38, respectively;
the light chain variable region comprises:
   (I) LCDR1, LCDR2, and LCDR3 with amino acid sequences as set forth in SEQ ID NO: 42, SEQ ID NO: 21, and SEQ ID NO: 22, respectively; or LCDR1, LCDR2, and LCDR3 having 1, 2, or 3 amino acid differences from the amino acid sequences as set forth in SEQ ID NO: 42, SEQ ID NO: 21, and SEQ ID NO: 22, respectively;
   (II) LCDR1, LCDR2, and LCDR3 with amino acid sequences as set forth in SEQ ID NO: 42, SEQ ID NO: 44, and SEQ ID NO: 22, respectively; or LCDR1, LCDR2, and LCDR3 having 1, 2, or 3 amino acid differences from the amino acid sequences as set forth in SEQ ID NO: 42, SEQ ID NO: 44, and SEQ ID NO: 22, respectively;
   (III) LCDR1, LCDR2, and LCDR3 with amino acid sequences as set forth in SEQ ID NO: 43, SEQ ID NO: 45, and SEQ ID NO: 22, respectively; or LCDR1, LCDR2, and LCDR3 having 1, 2, or 3 amino acid differences from the amino acid sequences as set forth in SEQ ID NO: 43, SEQ ID NO: 45, and SEQ ID NO: 22, respectively;
   (IV) LCDR1, LCDR2, and LCDR3 with amino acid sequences as set forth in SEQ ID NO: 43, SEQ ID NO: 46, and SEQ ID NO: 22, respectively; or LCDR1, LCDR2, and LCDR3 having 1, 2, or 3 amino acid differences from the amino acid sequences as set forth in SEQ ID NO: 43, SEQ ID NO: 46, and SEQ ID NO: 22, respectively;
   (V) LCDR1, LCDR2, and LCDR3 with amino acid sequences as set forth in SEQ ID NO: 20, SEQ ID NO: 21, and SEQ ID NO: 22, respectively; or LCDR1, LCDR2, and LCDR3 having 1, 2, or 3 amino acid differences from the amino acid sequences as set forth in SEQ ID NO: 20, SEQ ID NO: 21, and SEQ ID NO: 22, respectively;
   (VI) LCDR1, LCDR2, and LCDR3 with amino acid sequences as set forth in SEQ ID NO: 5, SEQ ID NO: 6, and SEQ ID NO: 7, respectively; or LCDR1, LCDR2, and LCDR3 having 1, 2, or 3 amino acid differences from the amino acid sequences as set forth in SEQ ID NO: 5, SEQ ID NO: 6, and SEQ ID NO: 7, respectively;
   (VII) LCDR1, LCDR2, and LCDR3 with amino acid sequences as set forth in SEQ ID NO: 13, SEQ ID NO: 14, and SEQ ID NO: 7, respectively; or LCDR1, LCDR2, and LCDR3 having 1, 2, or 3 amino acid differences from the amino acid sequences as set forth in SEQ ID NO: 13, SEQ ID NO: 14, and SEQ ID NO: 7, respectively;
   (VIII) LCDR1, LCDR2, and LCDR3 with amino acid sequences as set forth in SEQ ID NO: 13, SEQ ID NO: 27, and SEQ ID NO: 7, respectively; or LCDR1, LCDR2, and LCDR3 having 1, 2, or 3 amino acid differences from the amino acid sequences as set forth in SEQ ID NO: 13, SEQ ID NO: 27, and SEQ ID NO: 7, respectively;
   (IX) LCDR1, LCDR2, and LCDR3 with amino acid sequences as set forth in SEQ ID NO: 33, SEQ ID NO: 34, and SEQ ID NO: 35, respectively; or LCDR1, LCDR2, and LCDR3 having 1, 2, or 3 amino acid differences from the amino acid sequences as set forth in SEQ ID NO: 33, SEQ ID NO: 34, and SEQ ID NO: 35, respectively; or
   (X) LCDR1, LCDR2, and LCDR3 with amino acid sequences as set forth in SEQ ID NO: 13, SEQ ID NO: 6, and SEQ ID NO: 40, respectively; or LCDR1, LCDR2, and LCDR3 having 1, 2, or 3 amino acid differences from the amino acid sequences from those as set forth in SEQ ID NO: 13, SEQ ID NO: 6, and SEQ ID NO: 40, respectively.

In some embodiments, the antibody or antigen-binding fragment thereof according to the present invention comprises:
(I) a heavy chain variable region, which comprises HCDR1, HCDR2, and HCDR3 with amino acid sequences as set forth in SEQ ID NO: 16, SEQ ID NO: 55, and SEQ ID NO: 18, respectively; and a light chain variable region, which comprises LCDR1, LCDR2, and LCDR3 with amino acid sequences as set forth in SEQ ID NO: 42, SEQ ID NO: 21, and SEQ ID NO: 22, respectively;
(II) a heavy chain variable region, which comprises HCDR1, HCDR2, and HCDR3 with amino acid sequences as set forth in SEQ ID NO: 16, SEQ ID NO: 56, and SEQ ID NO: 18, respectively; and a light chain variable region, which comprises LCDR1, LCDR2, and LCDR3 with amino acid sequences as set forth in SEQ ID NO: 43, SEQ ID NO: 45, and SEQ ID NO: 22, respectively;
(III) a heavy chain variable region, which comprises HCDR1, HCDR2, and HCDR3 with amino acid sequences as set forth in SEQ ID NO: 16, SEQ ID NO: 56, and SEQ ID NO: 18, respectively; and a light chain variable region, which comprises LCDR1, LCDR2, and LCDR3 with amino acid sequences as set forth in SEQ ID NO: 43, SEQ ID NO: 46, and SEQ ID NO: 22, respectively;
(IV) a heavy chain variable region, which comprises HCDR1, HCDR2, and HCDR3 with amino acid sequences as set forth in SEQ ID NO: 16, SEQ ID NO: 17, and SEQ ID NO: 18, respectively; and a light chain variable region, which comprises LCDR1, LCDR2, and LCDR3 with amino acid sequences as set forth in SEQ ID NO: 20, SEQ ID NO: 21, and SEQ ID NO: 22, respectively;
(V) a heavy chain variable region, which comprises HCDR1, HCDR2, and HCDR3 with amino acid sequences as set forth in SEQ ID NO: 1, SEQ ID NO: 2, and SEQ ID NO: 3, respectively; and a light chain variable region, which comprises LCDR1, LCDR2, and LCDR3 with amino acid sequences as set forth in SEQ ID NO: 5, SEQ ID NO: 6, and SEQ ID NO: 7, respectively;
(VI) a heavy chain variable region, which comprises HCDR1, HCDR2, and HCDR3 with amino acid sequences as set forth in SEQ ID NO: 9, SEQ ID NO: 10, and SEQ ID NO: 11, respectively; and a light chain variable region, which comprises LCDR1, LCDR2, and LCDR3 with amino acid sequences as set forth in SEQ ID NO: 13, SEQ ID NO: 14, and SEQ ID NO: 7, respectively;
(VII) a heavy chain variable region, which comprises HCDR1, HCDR2, and HCDR3 with amino acid sequences as set forth in SEQ ID NO: 9, SEQ ID NO: 24, and SEQ ID NO: 25, respectively; and a light chain variable region, which comprises LCDR1, LCDR2, and LCDR3 with amino acid sequences as set forth in SEQ ID NO: 13, SEQ ID NO: 27, and SEQ ID NO: 7, respectively;
(VIII) a heavy chain variable region, which comprises HCDR1, HCDR2, and HCDR3 with amino acid sequences as set forth in SEQ ID NO: 29, SEQ ID NO: 30, and SEQ ID NO: 31, respectively; and a light chain variable region, which comprises LCDR1, LCDR2, and LCDR3 with amino acid sequences as set forth in SEQ ID NO: 33, SEQ ID NO: 34, and SEQ ID NO: 35, respectively; or
(IX) a heavy chain variable region, which comprises HCDR1, HCDR2, and HCDR3 with amino acid sequences as set forth in SEQ ID NO: 9, SEQ ID NO: 37, and SEQ ID NO: 38, respectively; and a light chain variable region, which comprises LCDR1, LCDR2, and LCDR3 with amino acid sequences as set forth in SEQ ID NO: 13, SEQ ID NO: 6, and SEQ ID NO: 40, respectively.

In some embodiments, the antibody or antigen-binding fragment thereof according to the present invention comprises:
(I) a heavy chain variable region, which comprises an amino acid sequence as set forth in any one of SEQ ID NOs: 19, 4, 12, 26, 32, and 39; and a light chain variable region, which comprises an amino acid sequence as set forth in any one of SEQ ID NOs: 23, 8, 15, 28, 36, and 41; or
(II) a heavy chain variable region, which comprises an amino acid sequence having at least 95%, 96%, 97%, 98% or 99% sequence identity to the amino acid sequence as set forth in any one of SEQ ID NOs: 19, 4, 12, 26, 32, and 39; and a light chain variable region, which comprises an amino acid sequence having at least 95%, 96%, 97%, 98% or 99% sequence identity to the amino acid sequence as set forth in any one of SEQ ID NOs: 23, 8, 15, 28, 36, and 41.

In some embodiments, the antibody or antigen-binding fragment thereof according to the present invention comprises a heavy chain variable region and a light chain variable region, wherein:
(I) the heavy chain variable region comprises an amino acid sequence as set forth in SEQ ID NO: 19, or comprises an amino acid sequence having at least 95%, 96%, 97%, 98%, or 99% sequence identity to the amino acid sequence as set forth in SEQ ID NO: 19; and the light chain variable region comprises an amino acid sequence as set forth in SEQ ID NO: 23, or comprises an amino acid sequence having at least 95%, 96%, 97%, 98%, or 99% sequence identity to the amino acid sequence as set forth in SEQ ID NO: 23;
(II) the heavy chain variable region comprises an amino acid sequence as set forth in SEQ ID NO: 4, or comprises an amino acid sequence having at least 95%, 96%, 97%, 98%, or 99% sequence identity to the amino acid sequence as set forth in SEQ ID NO: 4; and the light chain variable region comprises an amino acid sequence as set forth in SEQ ID NO: 8, or comprises an amino acid sequence having at least 95%, 96%, 97%, 98%, or 99% sequence identity to the amino acid sequence as set forth in SEQ ID NO: 8;
(III) the heavy chain variable region comprises an amino acid sequence as set forth in SEQ ID NO: 12, or comprises an amino acid sequence having at least 95%, 96%, 97%, 98%, or 99% sequence identity to the amino acid sequence as set forth in SEQ ID NO: 12; and the light chain variable region comprises an amino acid sequence as set forth in SEQ ID NO: 15, or comprises an amino acid sequence having at least 95%, 96%, 97%, 98%, or 99% sequence identity to the amino acid sequence as set forth in SEQ ID NO: 15;
(IV) the heavy chain variable region comprises an amino acid sequence as set forth in SEQ ID NO: 26, or comprises an amino acid sequence having at least 95%, 96%, 97%, 98%, or 99% sequence identity to the amino acid sequence as set forth in SEQ ID NO: 26; and the light chain variable region comprises an amino acid sequence as set forth in SEQ ID NO: 28, or comprises an amino acid sequence having at least 95%, 96%, 97%, 98%, or 99% sequence identity to the amino acid sequence as set forth in SEQ ID NO: 28;
(V) the heavy chain variable region comprises an amino acid sequence as set forth in SEQ ID NO: 32, or comprises an amino acid sequence having at least 95%, 96%, 97%, 98%, or 99% sequence identity to the amino acid sequence as set forth in SEQ ID NO: 32; and the light chain variable region comprises an amino acid sequence as set forth in SEQ ID NO: 36, or comprises an amino acid sequence having at least 95%, 96%, 97%, 98%, or 99% sequence identity to the amino acid sequence as set forth in SEQ ID NO: 36; or
(VI) the heavy chain variable region comprises an amino acid sequence as set forth in SEQ ID NO: 39, or comprises an amino acid sequence having at least 95%, 96%, 97%, 98%, or 99% sequence identity to the amino acid sequence as set forth in SEQ ID NO: 39; and the light chain variable region comprises an amino acid sequence as set forth in SEQ ID NO: 41, or comprises an amino acid sequence having at least 95%, 96%, 97%, 98%, or 99% sequence identity to the amino acid sequence as set forth in SEQ ID NO: 41.

In some embodiments, the antibody or antigen-binding fragment thereof according to the present invention comprises:
a heavy chain variable region, which comprises an amino acid sequence as set forth in any one of SEQ ID NOs: 57, 58, 59, 60, and 61; and a light chain variable region, which comprises an amino acid sequence as set forth in any one of SEQ ID NOs: 47, 48, 49, 50, 51, 52, and 53.

In some embodiments, the antibody or antigen-binding fragment thereof according to the present invention comprises:
a heavy chain variable region, which comprises an amino acid sequence having at least 95%, 96%, 97%, 98%, or 99% sequence identity to the amino acid sequence as set forth in any one of SEQ ID NOs: 57, 58, 59, 60, and 61; and a light chain variable region, which comprises an amino acid sequence having at least 95%, 96%, 97%, 98%, or 99% sequence identity to the amino acid sequence as set forth in any one of SEQ ID NOs: 47, 48, 49, 50, 51, 52, and 53.

In some embodiments, the antibody or antigen-binding fragment thereof according to the present invention comprises:
a heavy chain variable region with an amino acid sequence as set forth in SEQ ID NO: 57 or 58, and a light chain variable region with an amino acid sequence as set forth in SEQ ID NO: 47;
a heavy chain variable region with an amino acid sequence as set forth in SEQ ID NO: 58, and a light chain variable region with an amino acid sequence as set forth in SEQ ID NO: 51;
a heavy chain variable region with an amino acid sequence as set forth in SEQ ID NO: 60, and a light chain variable region with an amino acid sequence as set forth in SEQ ID NO: 52 or 53; or
a heavy chain variable region with an amino acid sequence as set forth in SEQ ID NO: 61, and a light chain variable region with an amino acid sequence as set forth in SEQ ID NO: 52 or 53.

In some embodiments, the antibody or antigen-binding fragment thereof according to the present invention is selected from an antibody having the CDRs and/or variable region sequences of antibody NP010-018 or its humanized antibody 18, 18-5, 18-8, 18-11, 18-12, 18-13 or 18-14, an antibody having at least 80% (e.g., at least 85%, at least 90%, at least 91%, at least 92%, at least 93%, at least 94%, at least 95%, at least 96%, at least 97%, at least 98%, at least 99%, or 100%) identity to the CDRs or variable region sequences, and an antibody differing from the CDRs or variable region sequences only by the deletion, addition or substitution of one or several amino acids.

In some embodiments, the antibody or antigen-binding fragment thereof according to the present invention comprises a constant region, such as a heavy chain constant region derived from a human immunoglobulin (e.g., IgG, such as IgG1, IgG2, IgG3 or IgG4) and/or a light chain constant region (e.g., κ or λ). In some embodiments, the antibody or antigen-binding fragment thereof according to the present invention comprises: a heavy chain constant region as set forth in SEQ ID NO: 63 and/or a light chain constant region as set forth in SEQ ID NO: 62.

In some embodiments, the antibody or antigen-binding fragment thereof according to the present invention is a murine antibody, a chimeric antibody, a humanized antibody, or a fully human antibody.

In some embodiments, the antibody molecule of the present invention is humanized. Different methods for humanizing antibodies are known to those skilled in the art, as reviewed by Almagro & Fransson (Almagro JC and Fransson J (2008) Frontiers in Bioscience 13:1619-1633), the contents of which are incorporated by reference in its entirety. In some embodiments, the antibody of the present invention comprises a heavy chain framework region derived from a human immunoglobulin (e.g., a heavy chain framework region comprised in the amino acid sequence encoded by human heavy chain germline gene), and/or a light chain framework region (e.g., a light chain framework region comprised in the amino acid sequence encoded by human light chain germline gene). The heavy chain framework region and/or the light chain framework region optionally comprise one or more (e.g., 1, 2, 3, 4, 5, 6, 7, 8, 9, or 10) back mutations from human residues to murine residues.

In some embodiments, the antibody molecule of the present invention is a human antibody or a humanized antibody. Various techniques known in the art can be used to prepare human antibodies or humanized antibodies.

In one embodiment, the antibody molecule of the present invention also encompasses antigen-binding fragments thereof, such as the following antibody fragments: Fab, Fab', F(ab')₂, Fv, scFv, or sdAb.

The precise amino acid sequence boundaries of the variable region CDRs of the antibody of the present invention can be determined using any of a number of well-known schemes, including Chothia based on the three-dimensional structure of antibody and the topology of CDR loops (Chothia et al. (1989) Nature 342:877-883; Al-Lazikani et al., "Standard conformations for the canonical structures of immunoglobulins", Journal of Molecular Biology, Vol. 273, pp. 927-948 (1997)), Kabat based on antibody sequence variability (Kabat et al., Sequences of Proteins of Immunological Interest, 4th ed., U.S. Department of Health and Human Services, National Institutes of Health (1987)), AbM (University of Bath), Contact (University College London), the International ImMunoGeneTics database (IMGT) (1999 Nucleic Acids Research, 27, 209-212), and the North CDR definition based on affinity propagation clustering utilizing a large number of crystal structures.

Unless otherwise specified, the CDRs of the antibody of the present invention may have their boundaries determined by a person skilled in the art according to any scheme known in the art (e.g., different assignment systems or combinations thereof). In some embodiments, the CDRs of the antibody of the present invention are defined by Kabat, Chothia, AbM, Contact, North, or IMGT. In some embodiments, the CDRs of the antibody of the present invention are defined by Kabat.

It should be noted that the boundaries of the CDRs of the variable region of a given antibody, as determined by different assignment systems, may vary. That is, the CDR sequences of the variable region of the same antibody may differ under different assignment systems. Therefore, when an antibody is defined in terms of specific CDR sequences as defined in the present invention, the scope of the antibody also encompasses antibodies whose variable region sequences comprise the specific CDR sequences, but whose declared CDR boundaries differ from those specifically defined in the present invention due to the application of different schemes (e.g., different assignment systems or combinations thereof).

Antibodies with different specificities (i.e., different binding sites for different antigens) have different CDRs. However, although CDRs vary from antibody to antibody, only a limited number of amino acid positions within the CDRs are directly involved in antigen binding. Using at least two of the Kabat, Chothia, AbM, Contact, and North methods, a minimal overlapping region can be determined, thereby providing a "minimal binding unit" for antigen binding. The minimal binding unit may be a sub-portion of a CDR. As will be appreciated by those skilled in the art, the remaining residues of the CDR sequence can be determined through antibody structure and protein folding. Accordingly, the present invention also contemplates variants of any CDR presented herein. For example, in a variant of a CDR, the amino acid residues of the minimal binding unit may remain unchanged, while the remaining CDR residues as defined by Kabat or Chothia may be substituted with conservative amino acid residues.

The humanized antibodies of the present invention can be prepared by inserting murine CDR regions into human germline framework regions using methods known in the art. See, e.g., U.S. Patent No. 5,225,539 to Winter et al., and U.S. Patent Nos. 5,530,101; 5,585,089; 5,693,762; and 6,180,370 to Queen et al.

In some embodiments, the amino acid differences comprise amino acid deletions, insertions, or substitutions. In some embodiments, the anti-CD3 antibody or antigen-binding fragment thereof of the present invention comprises those antibodies having an amino acid sequence that has been mutated by amino acid deletions, insertions, or substitutions, yet still shares at least about 90%, 91%, 92%, 93%, 94%, 95%, 96%, 97%, 98%, 99%, or 100% identity to the above antibody (particularly in the CDR regions depicted in the above sequences). In some embodiments, compared to the CDR regions depicted in the specific sequences, the antibody of the present invention has no more than 1, 2, 3, 4, or 5 amino acid mutations in the CDR regions resulting from the amino acid deletions, insertions, or substitutions. In some embodiments, compared to the framework regions in the specific sequences, the antibody of the present invention has no more than 1, 2, 3, 4, or 5 amino acid mutations in the framework regions resulting from the amino acid deletions, insertions, or substitutions.

In some embodiments, the polynucleotide molecule encoding the antibody of the present invention comprises a polynucleotide molecule that has been mutated by nucleotide deletion, insertion, or substitution, but still has at least about 60%, 70%, 80%, 90%, 95%, or 100% identity to the coding region corresponding to the CDR depicted in the above sequence.

In some embodiments, the CD3 antibody is an IgG antibody, such as an IgG1, IgG2, IgG3, or IgG4 antibody or a modified form thereof, as described in the following sections.

In some embodiments, one or more amino acid modifications may be introduced into the Fc region of the antibody provided herein to produce an Fc region variant. The Fc region variant may comprise a human Fc region sequence (e.g., a human IgG1, IgG2, IgG3, or IgG4 Fc region) containing amino acid modifications (e.g., substitutions) at one or more amino acid positions.

In some embodiments, it may be necessary to generate a cysteine-engineered antibody, such as "thio-MAb," wherein one or more residues of the antibody are replaced with cysteine residues.

In some embodiments, the antibody provided herein may be further modified to contain other non-protein moieties that are known in the art and readily available. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymers, carboxymethyl cellulose, dextran, polyvinyl alcohol, polyvinylpyrrolidone, poly-1,3-diane, poly-1,3,6-triane, ethylene/maleic anhydride copolymers, poly(amino acids) (homogeneous or random copolymers), and dextran or poly(*N*-vinylpyrrolidone)-polyethylene glycol, propylene glycol homopolymers, polypropylene oxide/ethylene oxide copolymers, polyoxyethylated polyols (e.g., glycerol), polyvinyl alcohol, and mixtures thereof.

### Reference antibody

In some embodiments, the reference antibody SP34 (Teclistamab-CD3) is selected from the heavy chain as set forth in SEQ ID NO: 66 and the light chain as set forth in SEQ ID NO: 67.

In some embodiments, the reference antibody blinatumumab is selected from the heavy chain as set forth in SEQ ID NO: 68 and the light chain as set forth in SEQ ID NO: 69.

### Binding portion of multispecific antibody to tumor-associated antigen (TAA) or tumor-specific antigen (TSA)

The present invention provides a multispecific antibody, which comprises an antigen-binding domain targeting CD3 and at least one antigen-binding domain targeting other antigen, wherein the antigen-binding domain targeting CD3 is selected from the antibody or antigen-binding fragment thereof as described herein, and the other antigen is selected from tumor-associated antigen (TAA) or tumor-specific antigen (TSA).

In some embodiments, the multispecific antibody of the present invention is a bispecific antibody, a trispecific antibody, or a tetraspecific antibody.

In some embodiments, the multispecific antibody of the present invention comprises the antigen-binding domain targeting CD3 and the antigen-binding domain targeting the tumor-associated antigen or tumor-specific antigen.

In the multispecific antibody of the present invention, the tumor-associated antigen or tumor-specific antigen is selected from the group consisting of CD19, CD20, CD22, EGFR, HER2, HER3, PSMA, EpCAM, EphA2, CD30, CD33, CD38, CD79b, CD123, CLDN18.2, MSLN, GUCY2C, AFP, PAP, TROP2, LRRC15, gp100, 5T4, CEA, UPK2, DLL3, PRAME, CD H17, CDH19, GPA33, FAP, GPRC5D, GPC3, B7-H3, CLL-1, LIV-1, ACPP, CLDN6, PSCA, ENPP3, PRLR, MUC16, MUC17, CCR5, GD2, GD3, Ras, LewisY, BORIS, NY-ESO-1, OY-TES1, TSHR, LY6K, FLt3, IGFR-1, CAIX, c-MET, TROP2, and any combination thereof.

In some embodiments, in the multispecific antibody of the present invention, the tumor-associated antigen is selected from CD19.

In some embodiments, the multispecific antibody of the present invention comprises the antigen-binding domain targeting CD3 and an antigen-binding domain targeting CD19; the antigen-binding domain targeting CD19 comprises a light chain variable region and a heavy chain variable region, wherein the heavy chain variable region comprises a sequence as set forth in SEQ ID NO: 70 or a variant thereof, and the light chain variable region comprises a sequence as set forth in SEQ ID NO: 71 or a variant thereof; preferably, the variant has at least 95%, 96%, 97%, 98%, or 99% sequence identity to the sequence from which it is derived.

In some embodiments, the antigen-binding domain targeting CD3 of such multispecific antibody comprises HCDR1, HCDR2, and HCDR3 with amino acid sequences as set forth in SEQ ID NO: 16, SEQ ID NO: 56, and SEQ ID NO: 18, respectively, and LCDR1, LCDR2, and LCDR3 with amino acid sequences as set forth in SEQ ID NO: 43, SEQ ID NO: 46, and SEQ ID NO: 22, respectively.

In some embodiments, the multispecific antibody is a bispecific antibody in the form of a common light chain.

In some embodiments, the common light chain of the multispecific antibody comprises LCDR1, LCDR2, and LCDR3 with amino acid sequences as set forth in SEQ ID NO: 42, SEQ ID NO: 21, and SEQ ID NO: 22, respectively; or LCDR1, LCDR2, and LCDR3 with amino acid sequences as set forth in SEQ ID NO: 43, SEQ ID NO: 45, and SEQ ID NO: 22, respectively; or LCDR1, LCDR2, and LCDR3 with amino acid sequences as set forth in SEQ ID NO: 43, SEQ ID NO: 46, and SEQ ID NO: 22, respectively; preferably, the common light chain comprises LCDR1, LCDR2, and LCDR3 with amino acid sequences as set forth in SEQ ID NO: 43, SEQ ID NO: 46, and SEQ ID NO: 22, respectively.

In some embodiments, the common light chain of the multispecific antibody comprises a sequence as set forth in SEQ ID NO: 47, 51, 52, or 53.

In some embodiments, the antigen-binding domain targeting CD3 of the multispecific antibody of the present invention is Fab.

In some embodiments, the antigen-binding domain targeting TAA or TSA of the multispecific antibody of the present invention is Fab or scFv.

In some embodiments, the multispecific antibody of the present invention further comprises an Fc domain. In some embodiments, the Fc domain is IgG, such as IgG1, IgG2, IgG3, or IgG4. In some embodiments, the Fc domain comprises a modification to promote dimerization of a first Fc monomer and a second Fc monomer.

In some embodiments, the modification is selected from mutations that promote Fab-arm exchange. In some embodiments, the modification comprises a K409R mutation in one of the two monomers and an F405L mutation in the other of the two monomers to promote heterodimerization of the Fab arm. In some embodiments, the heavy chain constant region sequence containing K409R is as set forth in SEQ ID NO: 63. In some embodiments, the heavy chain constant region sequence containing F405L is as set forth in SEQ ID NO: 72.

In some embodiments, the modification comprises a knob modification in one of the two monomers and a hole modification in the other of the two monomers, thereby forming a knob-into-hole modification.

In some embodiments, the Fc domain may also be a natural Fc sequence.

In some embodiments, the multispecific antibody of the present invention is a DuoBody or a common light chain bispecific antibody.

In some embodiments, the multispecific antibody of the present invention is a DuoBody, which comprises:
(i) peptide chain I-A, which comprises a VL of the antigen-binding domain targeting CD3 and a first light chain constant region (CL); preferably, the first CL is kappa;
(ii) peptide chain I-B, which comprises a VH of the antigen-binding domain targeting CD3 and a first heavy chain constant region (CH); preferably, the first CH is IgG, such as IgG1, IgG2, IgG3, or IgG4;
(iii) peptide chain I-C, which comprises a VH of the antigen-binding domain targeting TAA or TSA and a second CH; preferably, the second CH is IgG, such as IgG1, IgG2, IgG3, or IgG4; and
(iv) peptide chain I-D, which comprises a VL of the antigen-binding domain targeting TAA or TSA and a second CL; preferably, the second CL is kappa.

In some embodiments, the Fc domains comprised in the peptide chains I-B and I-C comprise modifications to promote dimerization of I-B and I-C, respectively.

In some embodiments, the Fc domains of the peptide chains I-B and I-C comprise a mutation that promotes Fab arm exchange, for example, a K409R mutation in the CH3 region of one of the Fc domains and an F405L mutation in the CH3 region of the other of the Fc domains. In some embodiments, the peptide chains I-B and I-C comprise heavy chain constant region sequences as set forth in SEQ ID NO: 63 and 72, respectively. In some embodiments, the peptide chains I-B and I-C comprise heavy chain constant region sequences as set forth in SEQ ID NO: 72 and 63, respectively.

In some embodiments, the peptide chains I-A and I-D comprise a light chain constant region sequence as set forth in SEQ ID NO: 62.

In some embodiments, the antigen-binding domain targeting TAA or TSA is an antigen-binding domain targeting CD19, wherein the peptide chain I-C comprises a VH sequence as set forth in SEQ ID NO: 70 or a variant thereof, and the peptide chain I-D comprises a VL sequence as set forth in SEQ ID NO: 71 or a variant thereof; preferably, the variant has at least 95%, 96%, 97%, 98%, or 99% sequence identity to the sequence from which it derived.

In some embodiments, the peptide chain I-A comprises LCDR1, LCDR2, and LCDR3 with amino acid sequences as set forth in SEQ ID NO: 42, SEQ ID NO: 21, and SEQ ID NO: 22, respectively, and the peptide chain I-B comprises HCDR1, HCDR2, and HCDR3 with amino acid sequences as set forth in SEQ ID NO: 16, SEQ ID NO: 55, and SEQ ID NO: 18, respectively.

In some embodiments, the peptide chain I-B comprises a VH sequence as set forth in SEQ ID NO: 58, 57, 60, or 61, or a variant thereof, and the peptide chain I-A comprises a VL sequence as set forth in SEQ ID NO: 51, 47, 52, or 53, or a variant thereof; preferably, the variant has at least 95%, 96%, 97%, 98%, or 99% sequence identity to the sequence from which it is derived.

In some embodiments, the peptide chain I-A comprises a VL sequence as set forth in SEQ ID NO: 51 or 47, or a variant thereof; the peptide chain I-B comprises a VH sequence as set forth in SEQ ID NO: 58 or 57, or a variant thereof. In some embodiments, the peptide chain I-A comprises a VL sequence as set forth in SEQ ID NO: 51 or a variant thereof; the peptide chain I-B comprises a VH sequence as set forth in SEQ ID NO: 58 or a variant thereof. In some embodiments, the peptide chain I-A comprises a VL sequence as set forth in SEQ ID NO: 47 or a variant thereof; the peptide chain I-B comprises a VH sequence as set forth in SEQ ID NO: 57 or a variant thereof. Preferably, the variant has at least 95%, 96%, 97%, 98%, or 99% sequence identity to the sequence from which it is derived.

In some embodiments, the peptide chain I-C comprises a VH sequence as set forth in SEQ ID NO: 70, the peptide chain I-D comprises a VL sequence as set forth in SEQ ID NO: 71, the peptide chain I-A comprises a VL sequence as set forth in SEQ ID NO: 51, and the peptide chain I-B comprises a VH sequence as set forth in SEQ ID NO: 58.

In some embodiments, the peptide chain I-C comprises a VH sequence as set forth in SEQ ID NO: 70, the peptide chain I-D comprises a VL sequence as set forth in SEQ ID NO: 71, the peptide chain I-A comprises a VL sequence as set forth in SEQ ID NO: 47, and the peptide chain I-B comprises a VH sequence as set forth in SEQ ID NO: 57.

In some embodiments, the present invention provides the following exemplary DuoBody:
(1) the peptide chain I-A comprises a light chain sequence as set forth in SEQ ID NO: 65; the peptide chain I-B comprises a heavy chain sequence as set forth in SEQ ID NO: 64; the peptide chain I-C comprises a heavy chain sequence as set forth in SEQ ID NO: 68; the peptide chain I-D comprises a light chain sequence as set forth in SEQ ID NO: 69; or,
(2) the peptide chain I-A comprises a light chain sequence as set forth in SEQ ID NO: 84; the peptide chain I-B comprises a heavy chain sequence as set forth in SEQ ID NO: 83; the peptide chain I-C comprises a heavy chain sequence as set forth in SEQ ID NO: 68; the peptide chain I-D comprises a light chain sequence as set forth in SEQ ID NO: 69.

In some embodiments, the multispecific antibody of the present invention is a common light chain bispecific antibody, which comprises:
(i) peptide chain II-A, which is a common light chain and comprises a VL of the antigen-binding domain targeting CD3 and a light chain constant region (CL); preferably, the CL is kappa;
(ii) peptide chain II-B, which comprises a VH of the antigen-binding domain targeting CD3 and a first heavy chain constant region (CH); preferably, the first CH is IgG, such as IgG1, IgG2, IgG3, or IgG4; and
(iii) peptide chain II-C, which comprises a VH of the antigen-binding domain targeting TAA or TSA and a second CH; preferably, the second CH is IgG, such as IgG1, IgG2, IgG3, or IgG4;
wherein, the VL of the peptide chain II-A and the VH of the peptide chain II-B form a first binding site targeting CD3, and the VL of the peptide chain II-A and the VH of the peptide chain II-C form a second binding site targeting TAA or TSA.

In some embodiments, the VH of the peptide chain II-B and the VL of the peptide chain II-A in the common light chain bispecific antibody respectively comprise:
(I) HCDR1, HCDR2, and HCDR3 with amino acid sequences as set forth in SEQ ID NO: 16, SEQ ID NO: 55, and SEQ ID NO: 18, respectively; and LCDR1, LCDR2, and LCDR3 with amino acid sequences as set forth in SEQ ID NO: 42, SEQ ID NO: 21, and SEQ ID NO: 22, respectively;
(II) HCDR1, HCDR2, and HCDR3 with amino acid sequences as set forth in SEQ ID NO: 16, SEQ ID NO: 56, and SEQ ID NO: 18, respectively; and LCDR1, LCDR2, and LCDR3 with amino acid sequences as set forth in SEQ ID NO: 43, SEQ ID NO: 45, and SEQ ID NO: 22, respectively; or
(III) HCDR1, HCDR2, and HCDR3 with amino acid sequences as set forth in SEQ ID NO: 16, SEQ ID NO: 56, and SEQ ID NO: 18, respectively; and LCDR1, LCDR2, and LCDR3 with amino acid sequences as set forth in SEQ ID NO: 43, SEQ ID NO: 46, and SEQ ID NO: 22, respectively.

In some embodiments, the VH of the peptide chain II-B and the VL of the peptide chain II-A in the common light chain bispecific antibody respectively comprise: HCDR1, HCDR2, and HCDR3 with amino acid sequences as set forth in SEQ ID NO: 16, SEQ ID NO: 56, and SEQ ID NO: 18, respectively; and LCDR1, LCDR2, and LCDR3 with amino acid sequences as set forth in SEQ ID NO: 43, SEQ ID NO: 46, and SEQ ID NO: 22, respectively.

In some embodiments, the peptide chain II-A of the common light chain bispecific antibody comprises a VL sequence as set forth in SEQ ID NO: 47, 51, 52, or 53, or a variant thereof, preferably, the variant having at least 95%, 96%, 97%, 98%, or 99% sequence identity to the sequence from which it is derived. In some embodiments, the peptide chain II-B of the common light chain bispecific antibody comprises a VH sequence as set forth in SEQ ID NO: 57, 58, 60, or 61, or a variant thereof, preferably the variant having at least 95%, 96%, 97%, 98%, or 99% sequence identity to the sequence from which it is derived.

In some embodiments, the VH of the peptide chain II-B and the VL of the peptide chain II-A in the common light chain bispecific antibody respectively comprise: (1) a VH sequence as set forth in SEQ ID NO: 57 or a variant thereof, a VL sequence as set forth in SEQ ID NO: 47 or a variant thereof; (2) a VH sequence as set forth in SEQ ID NO: 58 or a variant thereof, a VL sequence as set forth in SEQ ID NO: 47 or a variant thereof; (3) a VH sequence as set forth in SEQ ID NO: 58 or a variant thereof, a VL sequence as set forth in SEQ ID NO: 51 or a variant thereof; (4) a VH sequence as set forth in SEQ ID NO: 60 or a variant thereof, a VL sequence as set forth in SEQ ID NO: 52 or a variant thereof; (5) a VH sequence as set forth in SEQ ID NO: 60 or a variant thereof, a VL sequence as set forth in SEQ ID NO: 53 or a variant thereof; (6) a VH sequence as set forth in SEQ ID NO: 61 or a variant thereof, a VL sequence as set forth in SEQ ID NO: 52 or a variant thereof; or (7) a VH sequence as set forth in SEQ ID NO: 61 or a variant thereof, a VL sequence as set forth in SEQ ID NO: 53 or a variant thereof. The variants described in any one of (1) to (7) have at least 95%, 96%, 97%, 98%, or 99% sequence identity to the sequences from which they are derived.

In some embodiments, the VH of the peptide chain II-B and the VL of the peptide chain II-A in the common light chain bispecific antibody respectively comprise: a VH sequence as set forth in SEQ ID NO: 61 and a VL sequence as set forth in SEQ ID NO: 53.

In some embodiments, the peptide chain II-C of the common light chain bispecific antibody comprises a VH of the antigen-binding domain targeting CD19. In some embodiments, the VH of the peptide chain II-C of the common light chain bispecific antibody comprises a sequence as set forth in SEQ ID NO: 70 or a variant thereof; preferably, the variant has at least 95%, 96%, 97%, 98%, or 99% sequence identity to the sequence from which it is derived.

In some embodiments, the Fc domains comprised in the peptide chains II-B and II-C of the common light chain bispecific antibody optionally comprises modifications to promote dimerization of I-B and I-C, respectively.

In some embodiments, the Fc domains comprised in the peptide chains II-B and II-C comprise a K409R mutation in the CH3 region of one of the Fc domains and an F405L mutation in the CH3 region of the other of the Fc domains. In some embodiments, the peptide chains II-B and II-C comprise heavy chain constant region sequences as set forth in SEQ ID NO: 63 and 72, respectively. In some embodiments, the peptide chains II-B and II-C comprise heavy chain constant region sequences as set forth in SEQ ID NO: 72 and 63, respectively.

In some embodiments, the peptide chain II-A of the common light chain bispecific antibody comprises a light chain constant region sequence as set forth in SEQ ID NO: 62.

### Antibody expression

In another aspect, the present invention provides an isolated nucleic acid molecule encoding any of the above-described antibodies or fragments thereof or any one of their chains. In one embodiment, the isolated nucleic acid molecule may comprise a polynucleotide encoding the amino acid sequence of the light chain variable region and/or the heavy chain variable region of the antibody, or comprise a polynucleotide encoding the amino acid sequence of the light chain and/or heavy chain of the antibody. In some embodiments, the isolated nucleic acid molecule comprises polynucleotides encoding the polypeptide chains of the multispecific antibody of the present invention.

In another aspect, the present invention provides a vector (e.g., an expression vector), which comprises the isolated nucleic acid molecule as described herein, preferably the vector is an eukaryotic expression vector. In some embodiments, the isolated nucleic acid molecule as described herein is comprised in one or more vectors. Vectors include, but are not limited to, viruses, plasmids, granules, λ phages, or yeast artificial chromosomes (YAC).

In yet another aspect, the present invention provides a host cell, which comprises the isolated nucleic acid molecule as described herein or the expression vector as described herein, preferably the host cell is an eukaryotic cell, more preferably a mammalian cell (e.g., a CHO cell or a 293 cell). In another embodiment, the host cell is prokaryotic.

In one embodiment, the present invention provides a method for preparing the antibody of the present invention, wherein the method comprises, when introducing the expression vector into a mammalian host cell, producing the antibody by culturing the host cell for a sufficient period of time to allow the antibody to be expressed in the host cell, or more preferably by secreting the antibody into a culture medium in which the host cell is grown. The antibody can be recovered from the culture medium using standard protein purification methods.

The present invention provides a mammalian host cell for expressing the recombinant antibody of the present invention, comprising a number of immortalized cell lines available from the American Type Culture Collection (ATCC). These particularly include Chinese hamster ovary (CHO) cells, NSO, SP2/0 cells, HeLa cells, young hamster kidney (BHK) cells, monkey kidney cells (COS), human hepatocellular carcinoma cells, A549 cells, 293T cells, and many other cell lines. Mammal host cells include human, mouse, rat, dog, monkey, pig, goat, cattle, horse, and hamster cells. Particularly preferred cell lines are selected by determining which cell lines exhibit high expression levels.

It is likely that antibodies expressed by different cell lines or in transgenic animals will have different glycosylation patterns from one another. However, all antibodies encoded by the nucleic acid molecules provided herein or comprising the amino acid sequences provided herein are part of the present invention, regardless of antibody glycosylation. Similarly, in some embodiments, non-fucosylated antibodies are advantageous because they generally exhibit more potent efficacy in vitro and in vivo than their fucosylated counterparts, and are unlikely to be immunogenic, as their glycan structures are normal components of natural human serum IgG.

### Pharmaceutical compositions and formulations

In another aspect, the present invention provides a pharmaceutical composition, which comprises the antibody or antigen-binding fragment thereof as described herein, the multispecific antibody as described herein, the isolated nucleic acid molecule as described herein, the vector as described herein, or the host cell as described herein, and a pharmaceutically acceptable carrier and/or excipient.

The term "pharmaceutical composition" refers to a formulation that allows the active ingredient contained therein to exist in an biologically effective form and that contains no additional components that are unacceptably toxic to the subject to whom the formulation is administered.

A pharmaceutical formulation comprising the antibody as described herein can be prepared, preferably in the form of an aqueous solution or a lyophilized formulation, by mixing the antibody of the present invention having the desired purity with one or more optional pharmaceutically acceptable excipients (Remington's Pharmaceutical Sciences, 16th edition, Osol, A. ed. (1980)).

### Medical use and therapeutic methods

Any of the antibodies provided herein can be used in therapeutic methods. It is also to be understood that when discussing an "antibody," compositions comprising the antibody are also encompassed. The antibody of the present invention may be used in a therapeutically effective amount or a prophylactically effective amount in the treatment or prevention methods described in any of the embodiments of the present invention.

In yet another aspect, the present invention provides the use of the antibody or antigen-binding fragment thereof as described herein, the isolated nucleic acid molecule as described herein, the vector as described herein, the host cell as described herein, or the pharmaceutical composition as described herein, in the manufacture of a medicament for the prevention and/or treatment of a disease.

In yet another aspect, the present invention provides a method for preventing and/or treating a disease, comprising administering to a subject in need thereof an effective dose of the antibody or antigen-binding fragment thereof as described herein, the multispecific antibody as described herein, the isolated nucleic acid molecule as described herein, the vector as described herein, the host cell as described herein, or the pharmaceutical composition as described herein.

In some embodiments, in the use or method as provided above, the disease is a tumor, an inflammatory disease, or an autoimmune disease; preferably, the tumor is selected from the group consisting of ovarian cancer, breast cancer, gastric cancer, cholangiocarcinoma, colorectal cancer, lung cancer, acute myeloid leukemia, chronic lymphocytic leukemia, melanoma, and colorectal cancer.

In some embodiments, the administration routes of the present invention include, but are not limited to, oral, intravenous, subcutaneous, intramuscular, intra-arterial, intra-articular (e.g., in an arthritic joint), by inhalation, aerosol delivery, or intratumoral administration, and the like.

### Definitions of terms

In the present invention, unless otherwise stated, scientific and technical terms used herein have the meanings commonly understood by those skilled in the art. Furthermore, the procedures used herein, such as those of cell culture, molecular biology (including recombinant technology), microbiology, cell biology, biochemistry, nucleic acid chemistry, and immunology, are all conventional procedures widely used in their respective fields. To better understand the present invention, definitions and explanations of relevant terms are provided below.

To facilitate the understanding of the present invention, certain scientific and technical terms are specifically defined as follows. Unless otherwise clearly defined elsewhere in the present application, all scientific and technical terms used herein have the meanings commonly understood by those of ordinary skill in the art to which the present invention belongs. For definitions and terms in the art, professionals may refer specifically to Current Protocols in Molecular Biology (Ausubel). The abbreviations for amino acid residues are the standard three-letter and/or one-letter codes used in the art to denote one of the twenty commonly occurring L-amino acids. As used herein (including the claims), the singular forms include their corresponding plural forms unless the context clearly dictates otherwise.

The term "about" when used in conjunction with a numerical value means to encompass numerical values within a range having a lower limit of 5% less than the specified numerical value and an upper limit of 5% greater than the specified numerical value, including but not limited to ±5%, ±2%, ±1%, and ±0.1%, as such variations are appropriate for carrying out the disclosed methods.

The term "and/or" should be understood to mean any one of the options or any combination of two or more of the options.

The term "CD3" refers to a part of the T cell receptor complex, and consists of three distinct chains: CD3ε, CD3δ, and CD3γ. Clustering of CD3 on T cells, for example by immobilization of anti-CD3 antibodies thereon, leads to T cell activation, which is similar to T cell receptor-mediated activation but independent of TCR clonal specificity. The vast majority of anti-CD3 antibodies recognize the CD3ε chain. The term refers to any native CD3 from any vertebrate source, including mammals such as primates (e.g., humans) and rodents (e.g., mice and rats), unless otherwise specified. The term encompasses "full-length," unprocessed CD3 as well as any form of CD3 or any fragment thereof resulting from intracellular processing. The term also includes naturally occurring variants of CD3, such as splice variants or allelic variants. In a preferred embodiment, CD3 refers to full-length CD3 or fragment thereof (e.g., a mature fragment lacking signal peptide) from human/cynomolgus monkey. In a preferred embodiment, CD3 refers to full-length CD3 or fragments thereof (e.g., a mature fragment lacking signal peptide) from mouse/rat.

The term "CD19" refers to the Cluster of Differentiation 19 protein (CD19), which is an antigenic determinant detectable on precursor cells of leukemia. The amino acid and nucleic acid sequences for human and mouse can be found in public databases, such as GenBank, UniProt, and Swiss-Prot. For example, the amino acid sequence of human CD19 can be found under UniProt/Swiss-Prot accession number P15391, and the nucleotide sequence encoding human CD19 can be found under accession number NM_001178098. CD19 is expressed on most B-lineage cancers, such as acute lymphoblastic leukemia, chronic lymphocytic leukemia, and non-Hodgkin lymphoma.

As used herein, the term "or" should be understood to have the same meaning as "and/or" as defined above. For example, when separating items in a list, "or" or "and/or" shall be interpreted as inclusive, i.e., including at least one from the list of numbers or elements, but also including more than one, and optionally, additional unlisted items. Only when explicitly indicated to the contrary, such as "only one" or "exactly one," or when "consisting of" is used in a claim, shall it refer to only a single number or a single element from the list.

Unless expressly stated to the contrary, as used herein, the words "a" and "an" should be understood to mean "at least one."

The term "percent (%) amino acid sequence identity" or simply "identity" is defined as the percentage of amino acid residues in a candidate amino acid sequence that are identical to the amino acid residues in a reference amino acid sequence, after aligning the sequences (and introducing gaps if necessary) to achieve the maximum percent sequence identity, and without considering any conservative substitutions as part of the sequence identity. Sequence alignment to determine percent amino acid sequence identity can be performed using various methods known in the art, for example, using publicly available computer software such as BLAST, BLAST-2, ALIGN, or MEGALIGN (DNASTAR) software. Those skilled in the art can determine appropriate parameters for measuring alignment, including any algorithms required to obtain maximal alignment over the full length of the sequences being compared.

The term "immune response" refers to the action of, for example, lymphocytes, antigen-presenting cells, phagocytes, granulocytes, and soluble macromolecules (including antibodies, cytokines, and complement) produced by the above cells or the liver, which action leads to the selective damage, destruction, or elimination of invading pathogens, cells or tissues infected with pathogens, cancer cells, or, in the case of autoimmunity or pathological inflammation, normal human cells or tissues.

The term "signal transduction pathway" or "signal transduction activity" refers to a biochemical causal relationship, typically initiated by protein-protein interactions such as the binding of a growth factor to a receptor, which relationship leads to the transmission of a signal from one part of a cell to another part of the cell. Generally, transmission involves the specific phosphorylation of one or more tyrosine, serine, or threonine residues on one or more proteins in a series of reactions that result in signal transduction. The penultimate process typically includes nuclear events, leading to changes in gene expression.

The terms "activity" or "biological activity," or "biological property" or "biological characteristic" are used interchangeably herein and include, but are not limited to, epitope/antigen affinity and specificity, the ability to neutralize or antagonize CD3 activity in vivo or in vitro, IC₅₀, in vivo stability of antibody, and the immunogenic properties of antibody. Other identifiable biological properties or characteristics of antibodies known in the art include, for example, cross-reactivity (i.e., cross-reactivity generally with non-human homologs of target peptide, or with other proteins or tissues), and the ability to maintain high expression levels of proteins in mammalian cells. The aforementioned properties or characteristics are observed, measured, or assessed using techniques well known in the art, including but not limited to ELISA, FACS, or BIACORE surface plasmon resonance analysis, unrestricted in vitro or in vivo neutralization assays, receptor binding, cytokine or growth factor production and/or secretion, signal transduction, and immunohistochemistry of tissue sections from different sources (including human, primate, or any other source).

The term "antibody" refers to any form of antibody that possesses the desired biological activity. Therefore, it is used in the broadest sense and specifically includes, but is not limited to, monoclonal antibodies (including full-length monoclonal antibodies), polyclonal antibodies, multispecific antibodies (e.g., bispecific antibodies), humanized antibodies, fully human antibodies, chimeric antibodies, and camelized single-domain antibodies.

The term "isolated antibody" refers to a purified state of a binding compound and in this context means that the molecule is substantially free of other biomolecules, such as nucleic acids, proteins, lipids, sugars, or other substances such as cell debris and growth medium. The term "isolated" does not imply the complete absence of such substances or the absence of water, buffers, or salts, unless they are present in amounts that significantly interfere with the experimental or therapeutic application of the binding compounds described herein.

The term "monoclonal antibody" refers to an antibody obtained from a substantially homogeneous population of antibodies, i.e., the individual antibodies comprising the population are identical except for possible naturally occurring mutations that may be present in minor amounts. Monoclonal antibodies are highly specific, being directed against a single antigenic epitope. In contrast, conventional (polyclonal) antibody preparations typically include a large number of antibodies directed against (or specific for) different epitopes. The modifier "monoclonal" indicates the character of the antibody as obtained from a substantially homogeneous population of antibodies and shall not be construed as requiring the antibody to be produced by any particular method.

The term "full-length antibody" refers to an immunoglobulin molecule that, when naturally occurring, comprises at least four peptide chains: two heavy (H) chains and two light (L) chains interconnected by disulfide bonds. Each heavy chain is composed of a heavy chain variable region (abbreviated herein as VH) and a heavy chain constant region (abbreviated herein as CH). The heavy chain constant region is composed of three domains, CH1, CH2, and CH3. Each light chain is composed of a light chain variable region (abbreviated herein as VL) and a light chain constant region. The light chain constant region is composed of one domain, CL. The VH and VL regions can be further subdivided into regions of hypervariability, termed complementarity determining regions (CDRs), interspersed with regions that are more conserved, termed framework regions (FRs). Each VH or VL region is composed of three CDRs and four FRs arranged from amino-terminus to carboxyl-terminus in the following order: FR1, CDR1, FR2, CDR2, FR3, CDR3, FR4. The variable regions of the heavy and light chains contain a binding domain that interacts with an antigen. The constant region of an antibody may mediate the binding of the immunoglobulin to host tissues or factors, including various cells of the immune system (e.g., effector cells) and the first component (Clq) of the classical complement system.

The term "antigen-binding fragment" of an antibody ("parent antibody") includes fragments or derivatives of the antibody, typically comprising at least one fragment of the antigen-binding region or variable region (e.g., one or more CDRs) of the parent antibody, which retain at least some of the binding specificity of the parent antibody. Examples of antibody-binding fragments include, but are not limited to, Fab, Fab', F(ab')₂, and Fv fragments; diabodies; linear antibodies; single-chain antibody molecules, such as sc-Fv; nanobodies and multispecific antibodies formed from antibody fragments. When the binding activity of an antigen is expressed on a molar basis, the binding fragment or derivative typically retains at least 10% of its antigen-binding activity. Preferably, the binding fragment or derivative retains at least 20%, 50%, 70%, 80%, 90%, 95%, or 100% or more of the antigen-binding affinity of the parent antibody. It is also contemplated that an antigen-binding fragment of an antibody may comprise a conservative or non-conservative amino acid substitution that does not significantly alter its biological activity (referred to as "conservative variant" or "functionally conservative variant" of the antibody). The term "binding compound" refers to both an antibody and its binding fragment.

The term "single-chain Fv" or "scFv" antibody refers to an antibody fragment comprising the VH and VL domains of an antibody, wherein these domains are present in a single polypeptide chain. The Fv polypeptide generally further comprises a polypeptide linker between the VH and VL domains that enables the scFv to form the desired structure for antigen binding.

The term "domain antibody" is an immunologically functional immunoglobulin fragment containing only a heavy chain variable region or a light chain variable region. In some cases, two or more VH regions are covalently linked with peptide linkers to form a bivalent domain antibody. The two VH regions of a bivalent domain antibody can target the same or different antigens.

The term "bivalent antibody" comprises two antigen-binding sites. In some cases, the two binding sites have the same antigen specificity. However, a bivalent antibody can be bispecific.

The term "diabody" refers to a small antibody fragment with two antigen-binding sites, the fragment comprising a heavy chain variable domain (VH) linked to a light chain variable domain (VL) in the same polypeptide chain (VH-VL or VL-VH). By using a linker that is too short to allow pairing between the two domains on the same chain, the domains are forced to pair with the complementary domains of another chain and create two antigen-binding sites.

The term "murine antibody" or "hybridoma antibody" in the present disclosure refers to a monoclonal antibody against human CD3 prepared according to the knowledge and skills in the art. During preparation, a test subject is injected with CD3 antigen, and then hybridomas expressing antibodies with the desired sequences or functional properties are isolated.

The term "chimeric antibody" is an antibody having the variable domains of a first antibody and the constant domains of a second antibody, wherein the first and second antibodies are from different species. Typically, the variable domains are obtained from an antibody from a rodent or the like (the "parent antibody"), while the constant domain sequences are obtained from a human antibody, such that the resulting chimeric antibody is less likely to induce an adverse immune response in a human subject compared to the parent rodent antibody.

The term "humanized antibody" refers to a form of antibody that contains sequences from both human and non-human (e.g., mouse, rat) antibodies. Generally, a humanized antibody comprises substantially all of at least one, and typically two, variable domains, in which all or substantially all of the hypervariable loops correspond to those of a non-human immunoglobulin, and all or substantially all of the framework (FR) regions are those of a human immunoglobulin sequence. The humanized antibody optionally may comprise at least a portion of a human immunoglobulin constant region (Fc).

The term "fully human antibody" refers to an antibody that comprises only human immunoglobulin protein sequences. If produced in mice, in mouse cells, or in hybridomas derived from mouse cells, a fully human antibody may contain murine carbohydrate chains. Likewise, "mouse antibody" refers to an antibody that comprises only mouse immunoglobulin sequences. Alternatively, if produced in rats, in rat cells, or in hybridomas derived from rat cells, a fully human antibody may contain rat carbohydrate chains. Likewise, "rat antibody" refers to an antibody that comprises only rat immunoglobulin sequences.

"Isotype" antibodies refer to antibody classes provided by the heavy chain constant region gene (e.g., IgM, IgE, IgG such as IgG1, IgG2, or IgG4). Isotypes also include modified forms of one of these classes, wherein modifications have been made to alter Fc function, e.g., to enhance or weaken effector function or binding to Fc receptors.

The term "Fc region" as used herein is used to define the C-terminal region of an immunoglobulin heavy chain that comprises at least a portion of the constant region. The term includes native sequence Fc regions and variant Fc regions. In some embodiments, a human IgG heavy chain Fc region extends from Cys226 or Pro230 to the carboxyl-terminus of the heavy chain. However, the C-terminal lysine (Lys447) of the Fc region may be present or absent (numbering in this paragraph is according to the EU numbering system, also known as the EU index, as in Rabat et al., Sequences of Proteins of Immunological Interest, 5th Ed. Public Health Service, National Institutes of Health, Bethesda, MD, 1991).

The term "Knob-into-Hole structure" refers to the mutation of a hydrophobic amino acid in the CH3 domain of an antibody Fc. A side chain amino acid in the CH3 of one chain is mutated into a larger hydrophobic amino acid (knob) to enhance hydrophobic interactions; a side chain amino acid in another CH3 is mutated into a smaller amino acid (hole) to reduce steric hindrance; after mutation, the CH3 with the knob and the CH3 with the hole form a Knob-into-Hole structure (KiH) through hydrophobic interactions, facilitating the formation of heavy chain heterodimers; KiH mutations primarily occur at internal hydrophobic amino acids within the three-dimensional structure of the CH3 domain, with almost no change in exposed amino acids, thus not affecting Fc effector functions or immunogenicity.

The term "epitope" refers to a protein determinant capable of specifically binding to an antibody. Epitopes usually consist of chemically active surface molecules such as amino acids or sugar side chains and usually have specific three-dimensional structural characteristics as well as specific charge characteristics. The difference between conformational and non-conformational epitopes lies in the loss of binding to the former, but not the latter, in the presence of denaturing solvents.

The term "common light chain" refers to a light chain comprising the same light chain variable region and light chain constant region, which is capable of pairing with the heavy chain of a first antibody that binds to a first antigen to form a binding site that specifically binds to the first antigen, and is also capable of pairing with the heavy chain of a second antibody that binds to a second antigen to form a binding site that specifically binds to the second antigen. Further, the light chain variable region of the common light chain forms a first antigen-binding site with the heavy chain variable region of the first antibody, and the light chain variable region of the common light chain forms a second antigen-binding site with the heavy chain variable region of the second antibody.

The term "Duobody" refers to a heterodimer comprising one heavy chain and one light chain from a first antibody paired with one heavy chain and one light chain from a second antibody. Typically, a Duobody can be prepared by combining one half of a first monospecific antibody containing two heavy chains and two light chains with one half of a second monospecific antibody containing two heavy chains and two light chains. When the two monospecific antibodies recognize different epitopes on different antigens, the resulting heterodimer is a bispecific antibody. In some embodiments, each monospecific antibody comprises a heavy chain constant region with a single point mutation in the CH3 domain. These point mutations allow the interaction between the CH3 domains in the resulting bispecific antibody to be stronger than the interaction between CH3 domains in either monospecific antibody without the mutation. In some embodiments, the single point mutation in each monospecific antibody may be located at residue 366, 368, 370, 399, 405, 407, or 409 (according to EU numbering) in the CH3 domain of the heavy chain constant region. In some embodiments, the single point mutation is at a different residue in one monospecific antibody relative to the other monospecific antibody. For example, one monospecific antibody may comprise the mutation F405L (EU numbering), while the other monospecific antibody may comprise the mutation K409R (EU numbering).

The term "cross-reactivity" as described herein refers to the binding to antigenic fragments of the same target molecule in human, monkey, and/or murine (mouse or rat) species. Therefore, "cross-reactivity" is to be understood as an interspecies reaction with the same molecule X expressed in different species. The cross-reactivity specificity of a monoclonal antibody that recognizes human CD3, monkey, and/or murine CD3 (mouse or rat) can be determined by FACS analysis.

"Affinity" or "binding affinity" refers to the intrinsic binding affinity that reflects the interaction between members of a binding pair. The affinity of molecule X for its partner Y can generally be represented by the equilibrium dissociation constant (KD), which is the ratio of the dissociation rate constant to the association rate constant (kdis and kon, respectively). Affinity can be measured by common methods known in the art. One specific method for measuring affinity is the ForteBio kinetic binding assay described herein.

The term "not binding" to a protein or cell means not binding to the protein or cell, or not binding to it with high affinity, i.e., the KD for binding to the protein or cell is 1.0×10⁻⁶ M or higher, more preferably 1.0×10⁻⁵ M or higher, even more preferably 1.0×10⁻⁴ M or higher, 1.0×10⁻³ M or higher, and most preferably 1.0×10⁻² M or higher.

The term "high affinity" for an IgG antibody means a KD of 1.0×10⁻⁶ M or lower, preferably 5.0×10⁻⁸ M or lower, more preferably 1.0×10⁻⁸ M or lower, 5.0×10⁻⁹ M or lower, and even more preferably 1.0×10⁻⁹ M or lower, for the antigen. For other antibody isotypes, "high affinity" binding may vary. For example, "high affinity" binding for an IgM isotype means a KD of 10⁻⁶ M or lower, preferably 10⁻⁷ M or lower, more preferably 10⁻⁸ M or lower.

The ability to "compete for binding" refers to the ability of an antibody or antigen-binding fragment to inhibit the binding interaction between two molecules (e.g., human CD3 and anti-CD3 antibody) to any detectable degree (e.g., inhibition by at least 85%, or at least 90%, or at least 95%).

The term "antibody-dependent cellular cytotoxicity," "antibody-dependent cell-mediated cytotoxicity," or "ADCC" refers to a cell-mediated immune defense, in which immune system effector cells actively target cells whose membrane-surface antigens are bound by antibodies.

The term "complement-dependent cytotoxicity" or "CDC" refers to the effector function of IgG and IgM antibodies that, upon binding to surface antigens, trigger the classical complement pathway, including the formation of the membrane attack complex and target cell lysis.

The terms "nucleic acid," "polynucleotide," "nucleic acid molecule," and "polynucleotide molecule" refer to deoxyribonucleic acid (DNA) or ribonucleic acid (RNA) and a polymer thereof in either single-stranded or double-stranded form. Unless explicitly limited, the terms include nucleic acids containing known analogs of natural nucleotides that have similar binding properties as the reference nucleic acid and are metabolized in a manner similar to naturally occurring nucleotides (see, e.g., U.S. Patent No. 8,278,036 to Kariko et al., which discloses mRNA molecules in which uridine is replaced by pseudouridine, methods of synthesizing such mRNA molecules, and methods for in vivo delivery of therapeutic proteins). Unless otherwise indicated, a particular nucleic acid sequence also implicitly includes its conservatively modified variants (e.g., degenerate codon substitutions), alleles, orthologs, SNPs, and complementary sequences, as well as the explicitly indicated sequence. Specifically, degenerate codon substitutions may be achieved by generating sequences in which the third position of one or more selected (or all) codons is substituted with mixed bases and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

"Construct" refers to any recombinant polynucleotide molecule (e.g., plasmid, cosmid, virus, autonomously replicating polynucleotide molecule, phage, or linear or circular single-stranded or double-stranded DNA or RNA polynucleotide molecule), derived from any source, capable of genomic integration or autonomous replication, constituting a polynucleotide molecule in which one or more polynucleotide molecules have been functionally and operatively linked (i.e., operably linked). A recombinant construct typically will comprise a polynucleotide of the present invention operably linked to a transcription initiation regulatory sequence that directs transcription of the polynucleotide in a host cell. Both heterologous and non-heterologous (i.e., endogenous) promoters may be used to direct expression of the nucleic acid of the present invention.

"Vector" refers to any recombinant polynucleotide construct that may be used for transformation purposes (i.e., introduction of heterologous DNA into a host cell). One type of vector is a "plasmid," which refers to a circular double-stranded DNA loop into which an additional DNA segment may be ligated. Another type of vector is a viral vector, in which an additional DNA segment may be ligated into the viral genome. Certain vectors are capable of autonomous replication in the host cell into which they are introduced (e.g., bacterial vectors having a bacterial origin of replication and episomal mammalian vectors). Other vectors (e.g., non-episomal mammalian vectors) are integrated into the genome of the host cell upon introduction into the host cell, and are thereby replicated along with the host genome. Moreover, certain vectors are capable of directing the expression of operably linked genes. Such vectors are referred to herein as "expression vectors."

As used herein, the term "expression vector" refers to a nucleic acid molecule capable of replicating and expressing a gene of interest when transformed, transfected, or transduced into a host cell. Expression vectors comprise one or more phenotypic selection markers and an origin of replication to ensure the maintenance of the vector and, if desired, to provide amplification within the host.

"Activation," "stimulation," and "treatment" of a cell or receptor may have the same meaning, e.g., activation, stimulation, or treatment of a cell or receptor with a ligand, unless the context dictates otherwise or is explicitly specified. "Ligand" comprises natural and synthetic ligands, such as cytokines, cytokine variants, analogs, mutant proteins, and binding compounds derived from antibodies. "Ligand" also comprises small molecules, such as peptidomimetics of cytokines and peptidimimetics of antibodies. "Activation" may refer to cell activation regulated by internal mechanisms as well as external or environmental factors. "Response/reaction," e.g., the response of cells, tissues, organs, or organisms, comprises a change in biochemical or physiological behaviors (e.g., concentration, density, adhesion, or migration within a biological compartment, gene expression rate, or differentiation state), wherein the change is associated with activation, stimulation, or treatment, or with internal mechanisms such as genetic programming.

As used herein, the term "treatment" or "therapy" of any disease or disorder, in one embodiment, refers to ameliorating the disease or disorder (i.e., slowing or arresting or reducing the progression of the disease or at least one of its clinical symptoms). In another embodiment, "treatment" or "therapy" refers to alleviating or ameliorating at least one physical parameter, including those physical parameters that may not be discernible by the patient. In another embodiment, "treatment" or "therapy" refers to modulating the disease or disorder either physically (e.g., stabilization of a discernible symptom), physiologically (e.g., stabilization of a physical parameter), or both. Unless explicitly described herein, methods for assessing the treatment and/or prevention of a disease are generally known in the art.

"Subject" comprises any human or non-human animal. The term "non-human animal" comprises all vertebrates, e.g., mammals and non-mammals, such as non-human primates, sheep, dogs, cats, horses, cows, chickens, amphibians, reptiles, etc. As used herein, the term "cyno" or "cynomolgus monkey" refers to the cynomolgus monkey (*Macaca fascicularis*).

"In combination with" administration of one or more other therapeutic agents comprises simultaneous (concurrent) administration and sequential administration in any order.

"Therapeutically effective amount," "therapeutically effective dose," and "effective amount" refer to an amount of the anti-CD3 antibody or an antigen-binding fragment thereof of the present invention, that, when administered alone or in combination with other therapeutic agents to a cell, tissue, or subject, is effective to prevent or ameliorate one or more symptoms of a disease or condition or the progression of the disease or condition. A therapeutically effective dose also refers to an amount of the antibody or antigen-binding fragment thereof sufficient to result in symptom improvement, e.g., an amount that treats, cures, prevents, or ameliorates a related medical condition, or increases the rate of treatment, cure, prevention, or amelioration of such a condition. When administered to an individual as a single active ingredient, a therapeutically effective dose refers to only that ingredient. When administered in combination, a therapeutically effective dose refers to the combined amount of active ingredients that results in a therapeutic effect, whether administered in combination, sequentially, or simultaneously. An effective amount of a therapeutic agent will result in an improvement in a diagnostic criterion or parameter by at least 10%, typically at least 20%, preferably at least about 30%, more preferably at least 40%, and most preferably at least 50%.

"Pharmaceutically acceptable carrier" refers to an ingredient in a pharmaceutical formulation or composition, other than the active ingredient, that is non-toxic to a subject. Pharmaceutically acceptable carriers include, but are not limited to, buffers, excipients, stabilizers, or preservatives.

The term "cancer" is used herein to refer to a group of cells that exhibit abnormally high levels of proliferation and growth. Cancer may be benign (also known as a benign tumor), premalignant, or malignant. Cancer cells may be solid cancer cells or leukemic cancer cells. The term "tumor" as used herein refers to one or more cells comprising a cancer. The term "tumor growth" is used herein to refer to the proliferation or growth of one or more cells comprising a cancer, resulting in a corresponding increase in the size or extent of the cancer.

### Beneficial effects of the invention

In the present invention, multiple anti-human CD3 antibodies were thoroughly analyzed and compared, and their sequences were optimized and humanized to generate humanized CD3 antibodies.

The humanized anti-CD3 antibodies of the present invention, when used in the preparation of bispecific antibodies, exhibited enhanced tumor-killing activity and safety. The humanized sequences disclosed in the present invention possesses more advantages and features compared to humanized sequences from other companies. When used in the manufacture of novel anti-tumor drugs, they demonstrated superior tumor-killing activity and significantly improved physicochemical stability. Moreover, they exhibited markedly reduced cytokine release, thereby providing significantly enhanced safety, making it more suitable for the screening and development of anti-tumor drugs. This inventive achievement constitutes the beneficial effects of the present invention and offers high medical application value.

The embodiments of the present invention will be described in detail below with reference to the accompanying drawings and examples. However, those skilled in the art will understand that the following drawings and examples are for illustrative purposes only and are not intended to limit the scope of the present invention. Various objects and advantages of the present invention will become apparent to those skilled in the art from the following detailed description of the drawings and preferred embodiments.

### Brief Description of the Drawings

Figures 1A to 1C show the binding experiment of anti-human CD3E antibodies on Jurkat cells.
Figures 2A to 2E show the detection of activation activity of anti-human CD3E antibodies on Jurkat-NFAT cells.
Figures 3A to 3B show the binding experiment of humanized anti-CD3 antibodies on Jurkat cells.
Figure 4 shows the binding experiment of humanized anti-CD3 antibodies on cynomolgus monkey CD3.
Figures 5A to 5E show the validation of CD3xCD19 bispecific antibodies in Duobody form and its in vitro killing assay.
Figures 6A to 6F show the validation of CD3xBCMA bispecific antibodies in Duobody form and its in vitro killing assay.
Figures 7A to 7E show the cytokine release assay of CD3 bispecific antibodies in Duobody form.
Figures 8A to 8B show the in vitro binding assay of CD3xBCMA common light chain bispecific antibodies.
Figures 9A to 9E show the in vitro killing assay of CD3xBCMA common light chain bispecific antibodies.
Figure 10 shows the in vivo antitumor activity assay of CD3xBCMA common light chain bispecific antibodies.

### Sequence Information

**Table 1: Information on the sequences involved in the present invention is described in the table below:**

| SEQ ID NO | Description | Sequence |
|---|---|---|
| 1 | NP010-007 HCDR1 (Kabat numbering scheme) | RYGMH |
| 2 | NP010-007 HCDR2 (Kabat numbering scheme) | MIYYDSSKVYYADTVKG |
| 3 | NP010-007 HCDR3 (Kabat numbering scheme) | WDYTSGFAY |
| 4 | NP010-007 VH | DMKLVESGGGLVQPGNSLTLSCVASGFTFRR YGMHWIRQAPKQGLEWITMIYYDSSKVYYA DTVKGRFTISRDNSKNTLYLEMTSLRSDDTA MYYCAAWDYTSGFAYWGPGTLVTVSS |
| 5 | NP010-007 LCDR1 (Kabat numbering scheme) | QSSKSLLRSDGKTYLN |
| 6 | NP010-007/NP010-030 LCDR2 (Kabat | WMSTRAS |
| | numbering scheme) | |
| 7 | NP010-007/ NP010-012/ NP010-023 LCDR3 (Kabat numbering scheme) | QQDHEFPRT |
| 8 | NP010-007 VL | DIVMTQGALPNPVPSGESVSITCQSSKSLLRS DGKTYLNWYLQRPGQSPQFLIYWMSTRASG VSDRFSGSGSGTDFTLKISRVEAGDVGVYYC QQDHEFPRTFGPGTKLELR |
| 9 | NP010-012/ NP010-023/ NP010-030 HCDR1 (Kabat numbering scheme) | KYGMH |
| 10 | NP010-012 HCDR2 (Kabat numbering scheme) | MIYFDSSQMYYADTVKG |
| 11 | NP010-012 HCDR3 (Kabat numbering scheme) | WDYSRGFAY |
| 12 | NP010-012 VH | EVKLVESGGGLIQPGDSLTLSCVAPGFTFSKY GMHWIRQAPKKGLEWIAMIYFDSSQMYYAD TVKGRFTISRDNSKNTLYLEMNNLRSEDTAM YYCAAWDYSRGFAYWGQGTLVTVSS |
| 13 | NP010-012/ NP010-023/ NP010-030 LCDR1 (Kabat numbering scheme) | QSSKSLLRSNGKTYLN |
| 14 | NP010-012 LCDR2 (Kabat numbering scheme) | WMSARAS |
| 15 | NP010-012 VL | AIVMTQGALPNPVPSGESVSITCQSSKSLLRS NGKTYLNWYLQRPGQSPQLLIYWMSARASG VSDRFSGSGSGTDFTLKISRVEAEDVGVYYC QQDHEFPRTFGGGTKLELK |
| 16 | NP010-018 HCDR1 (Kabat numbering scheme) | NAAMY |
| 17 | NP010-018 HCDR2 (Kabat numbering scheme) | RIRTKPNNYETFYAESVKG |
| 18 | NP010-018 HCDR3 (Kabat numbering scheme) | AGTIPTMFMTLPPFAY |
| 19 | NP010-018 VH | AVQLVESGGGLVQPKESLRISCSASGFSFTNA AMYWVRQAPGKGLEWVARIRTKPNNYETFY AESVKGRFTISRDDSNSMVYLQMDNLKTADT AMYYCTTAGTIPTMFMTLPPFAYWGQGTLV TVSS |
| 20 | NP010-018 LCDR1 (Kabat numbering scheme) | KASQNIDRYLN |
| 21 | NP010-018 LCDR2 (Kabat numbering scheme) | NTNNLQT |
| 22 | NP010-018 LCDR3 (Kabat numbering scheme) | LQHRNWYT |
| 23 | NP010-018 VL | DIQMTQSPSFLSASVGDRVTINCKASQNIDRY LNWYQQKLGEPPKLLIYNTNNLQTGIPSRFSG SGSGTDFTLTISSLQPEDVASYSCLQHRNWYT FGAGTKLELK |
| 24 | NP010-023 HCDR2 (Kabat numbering scheme) | MIYYDSSQMYYADTVKG |
| 25 | NP010-023 HCDR3 (Kabat numbering scheme) | WDYSGGGSAD |
| 26 | NP010-023 VH | EVKLVESGGGLVQPGNSLTLSCVASGLTFRK YGMHWIRQAPKKGLEWISMIYYDSSQMYYA DTVKGRFTISRDNSKSTLYLEMNSLRSEDTAR YYCAAWDYSGGGSADWGQGTLVTVSS |
| 27 | NP010-023 LCDR2 (Kabat numbering scheme) | WMSSRAS |
| 28 | NP010-023 VL | EIVMTQGALPNPVPSGESASITCQSSKSLLRSN GKTYLNWYLQRPGQSPQLLIYWMSSRASGV SDRFSGSGSGTDFTLKISRVEAEDVGVYYCQ QDHEFPRTFGGGTKLELK |
| 29 | NP010-025 HCDR1 (Kabat numbering scheme) | DYYIH |
| 30 | NP010-025 HCDR2 (Kabat numbering scheme) | YINPYNNYIHYNAKFMN |
| 31 | NP010-025 HCDR3 (Kabat numbering scheme) | AARSTYHSVMDA |
| 32 | NP010-025 VH | QVSLLQSGAALVKPGASVKLSCRGSGYTFTD YYIHWVKQSHRKSLEWIGYINPYNNYIHYNA KFMNEATLTVDKSTNTAYLELSRLTSEGSAT YYCRTAARSTYHSVMDAWGQGVSVTVSS |
| 33 | NP010-025 LCDR1 (Kabat numbering scheme) | KSSQSLVHSDGKTYLI |
| 34 | NP010-025 LCDR2 (Kabat numbering scheme) | LVSKLDS |
| 35 | NP010-025 LCDR3 (Kabat numbering scheme) | LQGTHSPLT |
| 36 | NP010-025 VL | DVVMTQTPVSLSVAIGQPASISCKSSQSLVHS DGKTYLIWFLQRPGQSPKRLIYLVSKLDSGIP DRFSGSGSETDFTLKISRVETDDLGVYYCLQG THSPLTFGSGTKLEIK |
| 37 | NP010-030 HCDR2 (Kabat numbering | MIYYDSSQMYYVGTVKG |
| | scheme) | |
| 38 | NP010-030 HCDR3 (Kabat numbering scheme) | WDYNSGFAY |
| 39 | NP010-030 VH | EVKLVESGGGLVQPGNSLTLSCVASGFTFRK YGMHWIRQAPKKGLEWIAMIYYDSSQMYYV GTVKGRFTISRDNSKNTLYLEMNSLRSEDTA MYYCAAWDYNSGFAYWGQGTLVTVSS |
| 40 | NP010-030 LCDR3 (Kabat numbering scheme) | QQDLEFPRT |
| 41 | NP010-030 VL | DIVMTQGALPNPVPSGESASITCQSSKSLLRS NGKTYLNWYLQRPGQSPQFLIYWMSTRASG VSDRFSGSGSGTDFTLKISRVEAEDVGVYYC QQDLEFPRTFGGGTKLELK |
| 42 | NP010-Hz018-VL/VL1/VL2/VL3/VL4 LCDR1 (Kabat numbering scheme) | QASQNIDRYLN |
| 43 | NP010-Hz018-VL/VL5/VL6 LCDR1 (Kabat numbering scheme) | NTNSLQS |
| 44 | NP010-Hz018-VL1/VL3 LCDR2 (Kabat numbering scheme) | NTNNLET |
| 45 | NP010-Hz018-VL5 LCDR2 (Kabat numbering scheme) | NTNSLQS |
| 46 | NP010-Hz018-VL6 LCDR2 (Kabat numbering scheme) | NTNNLQS |
| 47 | NP010-Hz018-VL | DIQMTQSPSSLSASVGDRVTITCQASQNIDRY LNWYQQKPGKPPKLLIYNTNNLQTGVPSRFS GSGSGTDFTFTISSLQPEDIATYSCLQHRNWY TFGQGTKLEIK |
| 48 | NP010-Hz018-VL1 | DIQMTQSPSSLSASVGDRVTITCQASQNIDRY LNWYQQKPGKAPKLLIYNTNNLETGVPSRFS GSGSGTDFTFTISSLQPEDIATYYCLQHRNWY TFGQGTKLEIK |
| 49 | NP010-Hz018-VL2 | DIQMTQSPSSLSASVGDRVTITCQASQNIDRY LNWYQQKPGKAPKLLIYNTNNLQTGVPSRFS GSGSGTDFTFTISSLQPEDIATYYCLQHRNWY TFGQGTKLEIK |
| 50 | NP010-Hz018-VL3 | DIQMTQSPSSLSASVGDRVTITCQASQNIDRY LNWYQQKPGKPPKLLIYNTNNLETGVPSRFS GSGSGTDFTFTISSLQPEDIATYYCLQHRNWY TFGQGTKLEIK |
| 51 | NP010-Hz018-VL4 | DIQMTQSPSSLSASVGDRVTITCQASQNIDRY LNWYQQKPGKPPKLLIYNTNNLQTGVPSRFS GSGSGTDFTFTISSLQPEDIATYYCLQHRNWY |
| | | TFGQGTKLEIK |
| 52 | NP010-Hz018-VL5 | DIQMTQSPSFLSASVGDRVTITCRASQNIDRY LNWYQQKPGKAPKLLIYNTNSLQSGVPSRFS GSGSGTDFTLTISSLQPEDFATYYCLQHRNW YTFGQGTKLEIK |
| 53 | NP010-Hz018-VL6 | DIQMTQSPSFLSASVGDRVTITCRASQNIDRY LNWYQQKPGKPPKLLIYNTNNLQSGVPSRFS GSGSGTDFTLTISSLQPEDFATYYCLQHRNW YTFGQGTKLEIK |
| 54 | NP010-Hz018-VH3 HCDR1 (Kabat numbering scheme) | NAAMD |
| 55 | NP010-Hz018-VH/VH1 HCDR2 (Kabat numbering scheme) | RIRTKPNNYETDYAAPVKG |
| 56 | NP010-Hz018-VH3/VH4/VH5 HCDR2 (Kabat numbering scheme) | RIRTKPNNYETEYAASVKG |
| 57 | NP010-Hz018-VH | EVQLVESGGGLVKPGGSLRLSCAASGFSFTN AAMYWVRQAPGKGLEWVARIRTKPNNYET DYAAPVKGRFTISRDDSKNTVYLQMNSLKTE DTAVYYCTTAGTIPTMFMTLPPFAYWGQGTL VTVSS |
| 58 | NP010-Hz018-VH1 | EVQLVESGGGLVKPGGSLRLSCAASGFSFTN AAMYWVRQAPGKGLEWVGRIRTKPNNYET DYAAPVKGRFTISRDDSKNTLYLQMNSLKTE DTAVYYCTTAGTIPTMFMTLPPFAYWGQGTL VTVSS |
| 59 | NP010-Hz018-VH3 | EVQLVESGGGLVQPGGSLRLSCAASGFSFTN AAMDWVRQAPGKGLEWVGRIRTKPNNYETE YAASVKGRFTISRDDSKNSLYLQMNSLKTED TAVYYCTTAGTIPTMFMTLPPFAYWGQGTLV TVSS |
| 60 | NP010-Hz018-VH4 | EVQLVESGGGLVQPGGSLRLSCAASGFSFTN AAMYWVRQAPGKGLEWVGRIRTKPNNYETE YAASVKGRFTISRDDSKNSLYLQMNSLKTED TAVYYCTTAGTIPTMFMTLPPFAYWGQGTLV TVSS |
| 61 | NP010-Hz018-VH5 | EVQLVESGGGLVQPGGSLRLSCAASGFSFTN AAMYWVRQAPGKGLEWVARIRTKPNNYETE YAASVKGRFTISRDDSKNSVYLQMNSLKTED TAVYYCTTAGTIPTMFMTLPPFAYWGQGTLV TVSS |
| 62 | Kappa constant region sequence | |
| 63 | IgG4 constant region sequence (K409R) | ASTKGPSVFPLAPCSRSTSESTAALGCLVKDY FPEPVTVSWNSGALTSGVHTFPAVLQSSGLYS LSSVVTVPSSSLGTKTYTCNVDHKPSNTKVD KRVESKYGPPCPPCPAPEFLGGPSVFLFPPKP KDTLMISRTPEVTCVVVDVSQEDPEVQFNWY VDGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLGK |
| 64 | NP010-Hz018-8 HC | EVQLVESGGGLVKPGGSLRLSCAASGFSFTN AAMYWVRQAPGKGLEWVGRIRTKPNNYET DYAAPVKGRFTISRDDSKNTLYLQMNSLKTE DTAVYYCTTAGTIPTMFMTLPPFAYWGQGTL VTVSSASTKGPSVFPLAPCSRSTSESTAALGC LVKDYFPEPVTVSWNSGALTSGVHTFPAVLQ SSGLYSLSSVVTVPSSSLGTKTYTCNVDHKPS NTKVDKRVESKYGPPCPPCPAPEFLGGPSVFL FPPKPKDTLMISRTPEVTCVVVDVSQEDPEVQ FNWYVDGVEVHNAKTKPREEQFNSTYRVVS VLTVLHQDWLNGKEYKCKVSNKGLPSSIEKT ISKAKGQPREPQVYTLPPSQEEMTKNQVSLTC LVKGFYPSDIAVEWESNGQPENNYKTTPPVL DSDGSFFLYSRLTVDKSRWQEGNVFSCSVMH EALHNHYTQKSLSLSLGK |
| 65 | NP010-Hz018-8 LC | DIQMTQSPSSLSASVGDRVTITCQASQNIDRY LNWYQQKPGKPPKLLIYNTNNLQTGVPSRFS GSGSGTDFTFTISSLQPEDIATYYCLQHRNWY TFGQGTKLEIKRTVAAPSVFIFPPSDEQLKSGT ASVVCLLNNFYPREAKVQWKVDNALQSGNS QESVTEQDSKDSTYSLSSTLTLSKADYEKHK VYACEVTHQGLSSPVTKSFNRGEC |
| 66 | SP34(Teclistamab-CD3)-HC | EVQLVESGGGLVQPGGSLRLSCAASGFTFNT YAMNWVRQAPGKGLEWVARIRSKYNNYAT YYAASVKGRFTISRDDSKNSLYLQMNSLKTE DTAVYYCARHGNFGNSYVSWFAYWGQGTL VTVSSASTKGPSVFPLAPCSRSTSESTAALGC LVKDYFPEPVTVSWNSGALTSGVHTFPAVLQ SSGLYSLSSVVTVPSSSLGTKTYTCNVDHKPS NTKVDKRVESKYGPPCPPCPAPEAAGGPSVF LFPPKPKDTLMISRTPEVTCVVVDVSQEDPEV QFNWYVDGVEVHNAKTKPREEQFNSTYRVV SVLTVLHQDWLNGKEYKCKVSNKGLPSSIEK TISKAKGQPREPQVYTLPPSQEEMTKNQVSLT CLVKGFYPSDIAVEWESNGQPENNYKTTPPV LDSDGSFFLYSRLTVDKSRWQEGNVFSCSVM HEALHNHYTQKSLSLSLGK |
| 67 | SP34(Teclistamab-CD3)-LC | QTVVTQEPSLTVSPGGTVTLTCRSSTGAVTTS NYANWVQQKPGQAPRGLIGGTNKRAPGTPA RFSGSLLGGKAALTLSGVQPEDEAEYYCALW YSNLWVFGGGTKLTVLGQPKAAPSVTLFPPS SEELQANKATLVCLISDFYPGAVTVAWKADS |
| | | SPVKAGVETTTPSKQSNNKYAASSYLSLTPEQ WKSHRSYSCQVTHEGSTVEKTVAPTECS |
| 68 | Blinatumumab-CD19 terminal-HC | QVQLQQSGAELVRPGSSVKISCKASGYAFSS YWMNWVKQRPGQGLEWIGQIWPGDGDTNY NGKFKGKATLTADESSSTAYMQLSSLASEDS AVYFCARRETTTVGRYYYAMDYWGQGTTV TVSSASTKGPSVFPLAPCSRSTSESTAALGCL VKDYFPEPVTVSWNSGALTSGVHTFPAVLQS SGLYSLSSVVTVPSSSLGTKTYTCNVDHKPSN TKVDKRVESKYGPPCPPCPAPEFLGGPSVFLF PPKPKDTLMISRTPEVTCVVVDVSQEDPEVQF NWYVDGVEVHNAKTKPREEQFNSTYRVVSV LTVLHQDWLNGKEYKCKVSNKGLPSSIEKTI SKAKGQPREPQVYTLPPSQEEMTKNQVSLTC LVKGFYPSDIAVEWESNGQPENNYKTTPPVL DSDGSFLLYSKLTVDKSRWQEGNVFSCSVM HEALHNHYTQKSLSLSLGK |
| 69 | Blinatumumab-CD19 terminal-LC | DIQLTQSPASLAVSLGQRATISCKASQSVDYD GDSYLNWYQQIPGQPPKLLIYDASNLVSGIPP RFSGSGSGTDFTLNIHPVEKVDAATYHCQQS TEDPWTFGGGTKLEIKRTVAAPSVFIFPPSDE QLKSGTASVVCLLNNFYPREAKVQWKVDNA LQSGNSQESVTEQDSKDSTYSLSSTLTLSKAD YEKHKVYACEVTHQGLSSPVTKSFNRGEC |
| 70 | Blinatumumab-CD19 terminal-VH | QVQLQQSGAELVRPGSSVKISCKASGYAFSS YWMNWVKQRPGQGLEWIGQIWPGDGDTNY NGKFKGKATLTADESSSTAYMQLSSLASEDS AVYFCARRETTTVGRYYYAMDYWGQGTTV TVSS |
| 71 | Blinatumumab-CD19 terminal-VL | DIQLTQSPASLAVSLGQRATISCKASQSVDYD GDSYLNWYQQIPGQPPKLLIYDASNLVSGIPP RFSGSGSGTDFTLNIHPVEKVDAATYHCQQS TEDPWTFGGGTKLEIK |
| 72 | IgG4 constant region sequence (F405L) | ASTKGPSVFPLAPCSRSTSESTAALGCLVKDY FPEPVTVSWNSGALTSGVHTFPAVLQSSGLYS LSSVVTVPSSSLGTKTYTCNVDHKPSNTKVD KRVESKYGPPCPPCPAPEFLGGPSVFLFPPKP KDTLMISRTPEVTCVVVDVSQEDPEVQFNWY VDGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFLLYSKLTVDKSRWQEGNVFSCSVMHEALH NHYTQKSLSLSLGK |
| 73 | BCMA-005/ BCMA-41 HCDR1 (Kabat numbering scheme) | NFDMA |
| 74 | BCMA-005/ BCMA-41 HCDR2 (Kabat numbering scheme) | SIVTRGDDTYYRDSVRG |
| 75 | BCMA-005-HCDR3 (Kabat numbering | HDYGGPSGWFGY |
| | scheme) | |
| 76 | BCMA-005-LCDR1 (Kabat numbering scheme) | RASQGFSNNLN |
| 77 | BCMA-005-LCDR2 (Kabat numbering scheme) | YTSNLHS |
| 78 | BCMA-005-LCDR3 (Kabat numbering scheme) | QQDASFPPT |
| 79 | BCMA-005-VH | EVQLVESGGGLVKPGGSLRLSCAASGFTFSNF DMAWVRQAPGKGLEWVASIVTRGDDTYYR DSVRGRFTISRDNAKNTQYLQMNSLRAEDTA VYYCARHDYGGPSGWFGYWGQGTLVTVSS |
| 80 | BCMA-005-HC | EVQLVESGGGLVKPGGSLRLSCAASGFTFSNF DMAWVRQAPGKGLEWVASIVTRGDDTYYR DSVRGRFTISRDNAKNTQYLQMNSLRAEDTA VYYCARHDYGGPSGWFGYWGQGTLVTVSS ASTKGPSVFPLAPCSRSTSESTAALGCLVKDY FPEPVTVSWNSGALTSGVHTFPAVLQSSGLYS LSSVVTVPSSSLGTKTYTCNVDHKPSNTKVD KRVESKYGPPCPPCPAPEFLGGPSVFLFPPKP KDTLMISRTPEVTCVVVDVSQEDPEVQFNWY VDGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFLLYSKLTVDKSRWQEGNVFSCSVMHEALH NHYTQKSLSLSLGK |
| 81 | BCMA-005-VL | DIQMTQSPSSLSASVGDRVTITCRASQGFSNN LNWYQQKPGKTPKPLIYYTSNLHSGVPSRFS GSGSGTDYSLTISSLQPEDFATYYCQQDASFP PTFGQGTKLEIK |
| 82 | BCMA-005-LC | DIQMTQSPSSLSASVGDRVTITCRASQGFSNN LNWYQQKPGKTPKPLIYYTSNLHSGVPSRFS GSGSGTDYSLTISSLQPEDFATYYCQQDASFP PTFGQGTKLEIKRTVAAPSVFIFPPSDEQLKSG TASVVCLLNNFYPREAKVQWKVDNALQSGN SQESVTEQDSKDSTYSLSSTLTLSKADYEKHK VYACEVTHQGLSSPVTKSFNRGEC |
| 83 | BCMA-41 HCDR3 (Kabat numbering scheme) | HDYGGDAFWFGY |
| 84 | BCMA-41 VH | EVQLVESGGGLVKPGGSLRLSCAASGFTFSNF DMAWVRQAPGKGLEWVASIVTRGDDTYYR DSVRGRFTISRDNAKNTQYLQMNSLRAEDTA VYYCARHDYGGDAFWFGYWGQGTLVTVSS |
| 85 | HXT4s-BCMA-005-mut41-HC-FLRK | EVQLVESGGGLVKPGGSLRLSCAASGFTFSNF DMAWVRQAPGKGLEWVASIVTRGDDTYYR DSVRGRFTISRDNAKNTQYLQMNSLRAEDTA VYYCARHDYGGDAFWFGYWGQGTLVTVSS |
| | | ASTKGPSVFPLAPCSRSTSESTAALGCLVKDY FPEPVTVSWNSGALTSGVHTFPAVLQSSGLYS LSSVVTVPSSSLGTKTYTCNVDHKPSNTKVD KRVESKYGPPCPPCPAPEFLGGPSVFLFPPKP KDTLMISRTPEVTCVVVDVSQEDPEVQFNWY VDGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFLLYSKLTVDKSRWQEGNVFSCSVMHEALH NHYTQKSLSLSLGK |

### Specific Models for Carrying Out the present invention

The present invention comprises all combinations of the specific embodiments described herein. Further embodiments of the present invention and the full scope of its applicability will become apparent from the detailed description provided below. However, it should be understood that although the detailed description and specific examples indicate preferred embodiments of the present invention, these descriptions and examples are provided by way of illustration only, as various changes and modifications within the spirit and scope of the present invention will become apparent to those skilled in the art from these detailed descriptions. For all purposes, all disclosures, patents, and patent applications cited herein, including citations, are incorporated herein by reference in their entirety.

The antibodies of the present invention can be prepared by various methods well known to those skilled in the art, including the specific embodiments listed below, embodiments formed by combining them with other methods, and equivalent substitutions well known to those skilled in the art, with preferred embodiments including but not limited to the examples of the present invention.

The present invention is illustrated by the following examples, but is not intended to limit the present invention in any way. The invention has been described in detail herein, and specific embodiments thereof have also been disclosed. Various changes and modifications to the specific embodiments of the present invention will be apparent to those skilled in the art without departing from the spirit and scope of the present invention.

### Example 1: Hybridoma method for generating monoclonal antibodies against human CD3E

The heterodimeric protein CD3E&G (UniProt database, P07766-1 & P09693), which corresponded to the extracellular domain of the recombinant human CD3 antigen protein and was purchased from Acro Biosystems (Cat. No.: CDG-H52W5), or a peptide segment of KLH- conjugated CD3E (sequence: DGNEEMGGITQTPYKVSISGTTVILTC-KLH), was used to immunize 6- to 8-week-old Balb/c mice (purchased from Vital River) or Sprague-Dawley rats (purchased from Vital River). The first immunization was performed with complete Freund's adjuvant at a dose of 50 µg of antigen per mouse (or rat). The second and subsequent immunizations were performed with incomplete Freund's adjuvant mixed with 25 µg of protein antigen per mouse (or rat). Immunization time points for mice were day 0, day 14, day 28, and day 49.

After four immunizations, tail blood was collected from the mice, and the serum titer of anti-CD3E antibodies (Acro Biosystems, Cat. No.: H5223) was measured. The mouse (or rat) with the highest serum titer of anti-CD3E antibody was selected, and its spleen cells were isolated, then fused with mouse myeloma cell line SP2/0. After plating and culturing in HAT selection medium for 10 to 14 days, wells showing positive binding of culture supernatant to human CD3E were subcloned. Monoclonal clones that remained positive for human CD3E binding after subcloning were selected, and the variable region sequences of the anti-human CD3E antibodies were obtained by Sanger sequencing. The sequences of the light chain and heavy chain variable regions and CDRs (according to Kabat numbering scheme) of the anti-human CD3E antibodies are shown in Table 2 below. For the specific amino acid sequences, please refer to Table 1.

**Table 2: Sequence composition of light and heavy chain variable regions of anti-human CD3E antibodies**

| | HCDR1 | HCDR2 | HCDR3 | VH | LCDR1 | LCDR2 | LCDR3 | VL |
|---|---|---|---|---|---|---|---|---|
| | (SEQ ID NO: ) | (SEQ ID NO: ) | (SEQ ID NO: ) | (SEQ ID NO: ) | (SEQ ID NO: ) | (SEQ ID NO: ) | (SEQ ID NO: ) | (SEQ ID NO: ) |
| NP010-00 7 | 1 | 2 | 3 | 4 | 5 | 6 | 7 | 8 |
| NP010-01 2 | 9 | 10 | 11 | 12 | 13 | 14 | 7 | 15 |
| NP010-01 8 | 16 | 17 | 18 | 19 | 20 | 21 | 22 | 23 |
| NP010-02 3 | 9 | 24 | 25 | 26 | 13 | 27 | 7 | 28 |
| NP010-02 5 | 29 | 30 | 31 | 32 | 33 | 34 | 35 | 36 |
| NP010-03 0 | 9 | 37 | 38 | 39 | 13 | 6 | 40 | 41 |

### Example 2: Jurkat binding detection of anti-human CD3E antibodies

The variable region sequences of the anti-human CD3E antibody were subcloned into the backbone of human IgG4 (SEQ ID NO: 63)/Kappa (SEQ ID NO: 62). The constructs were expressed in the HEK293 system, and purified with Protein A affinity chromatography to obtain the human-mouse chimeric anti-human CD3E antibodies NP010-007, NP010-012, NP010-018, NP010-023, NP010-025, and NP010-030.

The binding of anti-human CD3E antibodies to Jurkat cells was determined by flow cytometry. The specific method was as follows: Jurkat cells (Clone E6-1, ATCC, Cat. No.: TIB-152) were washed twice with DPBS (Dulbecco's phosphate-buffered saline), centrifuged at 1000 rpm, and harvested. The cell density was adjusted to 50,000 cells per well using FACS buffer (DPBS + 2% FBS (fetal bovine serum)). Serially diluted chimeric anti-human CD3E antibodies (starting concentration 15 µg/mL, 3-fold serial dilutions) were incubated with Jurkat cells on ice for 60 minutes. The cells were washed twice with pre-chilled FACS buffer, followed by incubation with a 1:1000 dilution of PE (fluorescein)-labeled anti-human IgG secondary antibody on ice for 30 minutes. After two additional washes, the cells were resuspended and fluorescence signals were detected using a Sartorius IntelliCyt^{®} iQue3 flow cytometer.

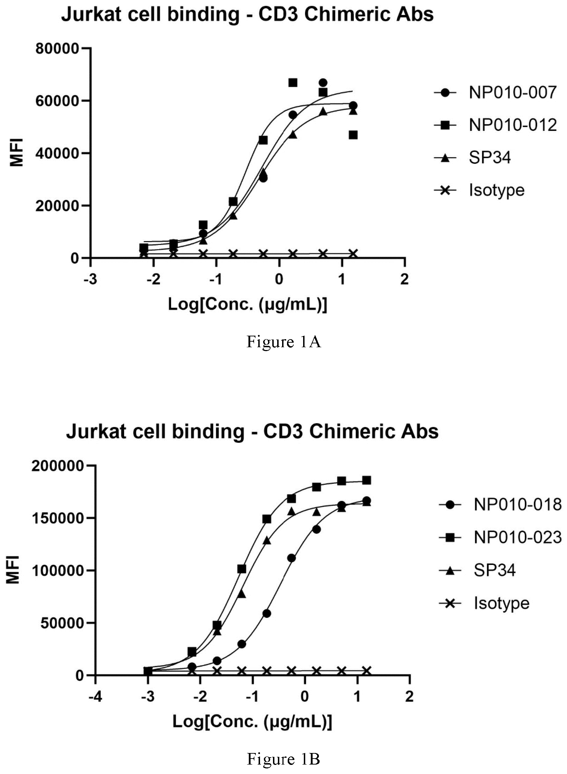

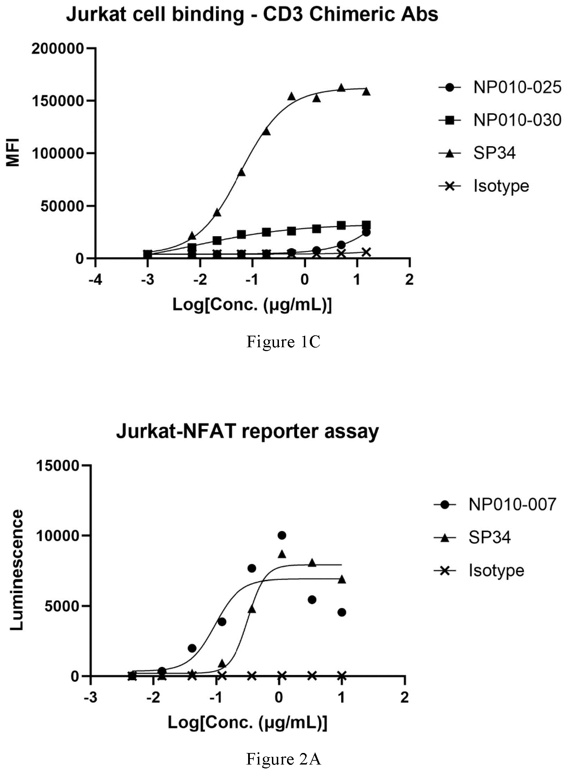

SP34 (heavy chain sequence as set forth in SEQ ID NO: 66, light chain sequence as set forth in SEQ ID NO: 67), internally expressed by Junshi Biosciences, was used as a positive control antibody. Figures 1A to 1C show the binding of anti-human CD3E antibodies to Jurkat cells.

### Example 3: Detection of activation activity of anti-human CD3E antibodies on Jurkat-NFAT cells

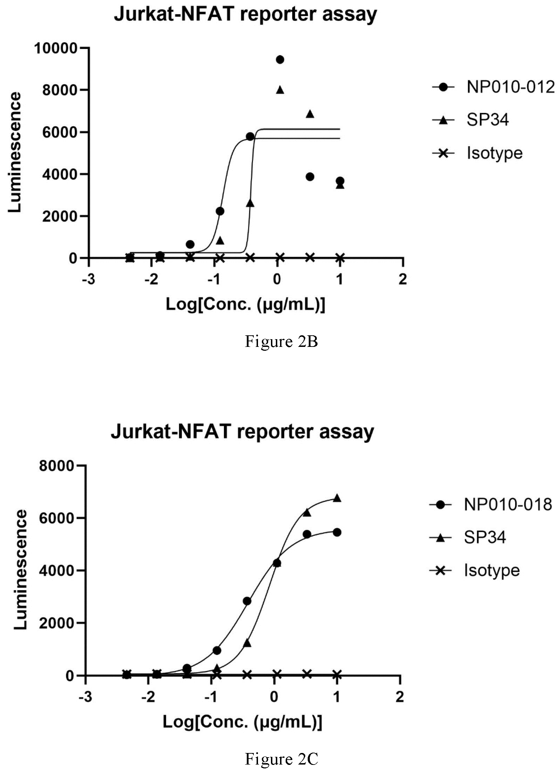

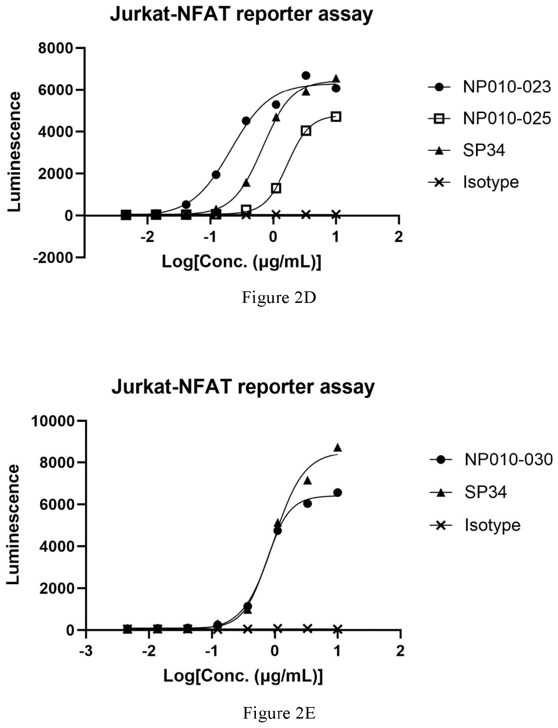

In this experiment, the principle is that the expression of NFAT reporter gene could be induced in the Jurkat-NFAT-Luc cell line under stimulation with anti-CD3 antibody was utilized to assess the extent of T cell activation by the CD3 antibodies. 40 µl of Jurkat-NFAT cells (Cell Resource Center, Shanghai Institutes for Biological Sciences, Chinese Academy of Sciences) at 10,000 cells per well were added to a 96-well cell culture plate (Corning, Cat. No.: 3917). Subsequently, 40 µl of serially diluted CD3 chimeric antibodies (starting concentration of 10 µg/ml, 3-fold dilution, 8 concentration gradients in total) were added and co-incubated for 6 hours at 37°C. Next, 50 µl of the luciferase substrate One-Lite (Vazyme, DD1203-01) was added, mixed, and incubated at room temperature for 6 minutes. Readings were then performed using an M5 microplate reader (Molecular Devices). As shown in Figures 2A-E, different CD3 chimeric antibodies all activated Jurkat-NFAT cells to some extent.

### Example 4: Humanization of anti-human CD3E antibody

The anti-human CD3E antibody NP010-018 was selected for humanization. The CDR grafting method used for humanization is briefly described as follows: the variable regions of the heavy and light chains of the murine antibody are compared with germline sequences, and sequences with high similarity are selected as humanization templates for the heavy and light chains, respectively, for CDR grafting. At the same time, homology modeling is performed using the VH and VL of known antibody structures with high homology to identify key amino acids in the framework regions that maintain the binding activity of the murine antibody, from which reference sites for back mutations are selected.

By searching and comparing NP010-018 against the human germline database, based on the degree of sequence similarity, the light chains IGKV1-33*01 and IGKV1-39*02 were selected as templates for humanization, and the light chain back mutation sites were at positions 43P, 53N, 55Q, and 87S. The heavy chains IGHV3-15*01 and IGHV3-72*01 were selected as templates for humanization, and the heavy chain back mutation sites were at positions 35Y, 49A, and 81V.

The humanized light chains after CDR grafting were: NP010-Hz018-VL (IGKV1-33*01), NP010-Hz018-VL1 (IGKV1-33*01), NP010-Hz018-VL2 (IGKV1-33*01), NP010-Hz018-VL3 (IGKV1-33*01), NP010-Hz018-VL4 (IGKV1-33*01), NP010-Hz018-VL5 (IGKV1-39*02), and NP010-Hz018-VL6 (IGKV1-39*02). Their sequence compositions are shown in Table 3 below.

**Table 3: Light chain sequence compositions of humanized anti-human CD3E antibodies**

| | LCDR1 (SEQ ID NO: ) | LCDR2 (SEQ ID NO: ) | LCDR3 (SEQ ID NO: ) | VL (SEQ ID NO: ) |
|---|---|---|---|---|
| NP010-Hz018-VL | 42 | 21 | 22 | 47 |
| NP010-Hz018-VL1 | 42 | 44 | 22 | 48 |
| NP010-Hz018-VL2 | 42 | 21 | 22 | 49 |
| NP010-Hz018-VL3 | 42 | 44 | 22 | 50 |
| NP010-Hz018-VL4 | 42 | 21 | 22 | 51 |
| NP010-Hz018-VL5 | 43 | 45 | 22 | 52 |
| NP010-Hz018-VL6 | 43 | 46 | 22 | 53 |

The humanized heavy chains after CDR grafting were: NP010-Hz018-VH (IGHV3-15*01), NP010-Hz018-VH1 (IGHV3-15*01), NP010-Hz018-VH3 (IGHV3-72*01), NP010-Hz018-VH4 (IGHV3-72*01), and NP010-Hz018-VH5 (IGHV3-72*01). Their sequence compositions are shown in Table 3 below.

**Table 4: Heavy chain sequence compositions of humanized anti-human CD3E antibodies**

| | HCDR1 (SEQ ID NO: ) | HCDR2 (SEQ ID NO: ) | HCDR3 (SEQ ID NO: ) | VH (SEQ ID NO: ) |
|---|---|---|---|---|
| NP010-Hz018-VH | 16 | 55 | 18 | 57 |
| NP010-Hz018-VH1 | 16 | 55 | 18 | 58 |
| NP010-Hz018-VH3 | 54 | 55 | 18 | 59 |
| NP010-Hz018-VH4 | 16 | 56 | 18 | 60 |
| NP010-Hz018-VH5 | 16 | 56 | 18 | 61 |

The humanized antibody is designated as NP010-Hz018, where the number following the designation represents the serial number of the humanized antibody, e.g., NP010-Hz018-5, NP010-Hz018-8, NP010-Hz018-14. The humanized antibody sequences were cloned into the framework of human IgG4 (SEQ ID NO: 63)/kappa (SEQ ID NO: 62) (sequences shown below), and the humanized antibodies were expressed and purified using the HEK293 expression system.

The heavy and light chain pairings of the series of antibodies generated after humanization of the NP010-018 chimeric antibody are shown in Table 5 below.

**Table 5: Heavy and light chain (variable region) pairings of humanized antibodies**

| | VH | VH1 | VH3 | VH4 | VH5 |
|---|---|---|---|---|---|
| VL | 18 | 18-5 | | | |
| VL1 | | | | | |
| VL2 | | | | | |
| VL3 | | | | | |
| VL4 | | 18-8 | | | |
| VL5 | | | | 18-11 | 18-13 |
| VL6 | | | | 18-12 | 18-14 |

The specific compositions of the light and heavy chain sequences of the humanized antibodies are shown in Tables 6 and 7 below.

**Table 6: Heavy chain sequence compositions of humanized antibodies**

| | HCDR1 | HCDR2 | HCDR3 | VH | CH |
|---|---|---|---|---|---|
| | (SEQ ID NO: ) | (SEQ ID NO: ) | (SEQ ID NO: ) | (SEQ ID NO: ) | (SEQ ID NO: ) |
| NP010-Hz018 | 16 | 55 | 18 | 57 | 63 |
| NP010-Hz018-5 | 16 | 55 | 18 | 58 | 63 |
| NP010-Hz018-8 | 16 | 55 | 18 | 58 | 63 |
| NP010-Hz018-11 | 16 | 56 | 18 | 60 | 63 |
| NP010-Hz018-12 | 16 | 56 | 18 | 60 | 63 |
| NP010-Hz018-13 | 16 | 56 | 18 | 61 | 63 |
| NP010-Hz018-14 | 16 | 56 | 18 | 61 | 63 |

**Table 7: Light chain sequence compositions of humanized antibodies**

| | LCDR1 | LCDR2 | LCDR3 | VL | CL |
|---|---|---|---|---|---|
| | (SEQ ID NO: ) | (SEQ ID NO: ) | (SEQ ID NO: ) | (SEQ ID NO: ) | (SEQ ID NO: ) |
| NP010-Hz018 | 42 | 21 | 22 | 47 | 62 |
| NP010-Hz018-5 | 42 | 21 | 22 | 47 | 62 |
| NP010-Hz018-8 | 42 | 21 | 22 | 51 | 62 |
| NP010-Hz018-11 | 43 | 45 | 22 | 52 | 62 |
| NP010-Hz018-12 | 43 | 46 | 22 | 53 | 62 |
| NP010-Hz018-13 | 43 | 45 | 22 | 52 | 62 |
| NP010-Hz018-14 | 43 | 46 | 22 | 53 | 62 |

### Example 5: Binding activity of humanized anti-CD3 antibody on Jurkat cells

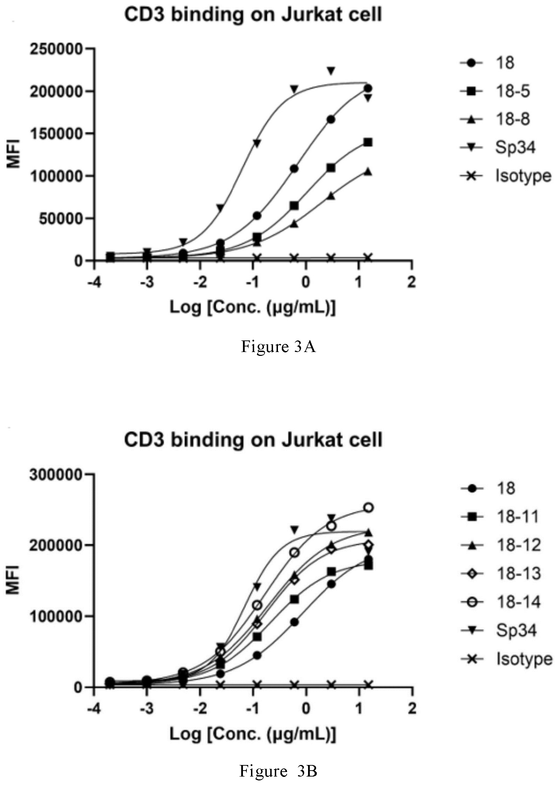

In a 96-well U-bottom cell culture plate, 100 µl of Jurkat cells (total cell number: 5×10⁴ cells) were added, followed by 100 µl of serially diluted CD3 chimeric and humanized monoclonal antibodies (starting concentration: 15 µg/ml, 5-fold dilution, 8 concentration gradients in total). The plate was incubated at 4°C for 30 minutes. After incubation, the cells were washed twice with flow cytometry staining buffer (BD, Cat. No.: 554658) to remove unbound antibodies. Then, 50 µl of Alexa Fluor 488-labeled Goat Anti-Human IgG secondary antibody (Jackson Lab, Cat. No.: 109-546-098, 1:1000 dilution) was added, and the mixture was incubated at 4°C for 1 hour. Finally, the cells were washed twice with flow cytometry staining buffer, resuspended in 100 µl of the same buffer, and analyzed by flow cytometry. The data were analyzed using FlowJo software. As shown in Figures 3A and 3B, a series of antibodies derived from the humanization of the NP010-018 chimeric antibody exhibited varying degrees of binding activity to Jurkat cells. Among them, 18-14 showed the strongest cell binding activity, while 18-8 showed the weakest.

### Example 6: Binding activity of humanized anti-CD3 antibodies on cynomolgus CD3

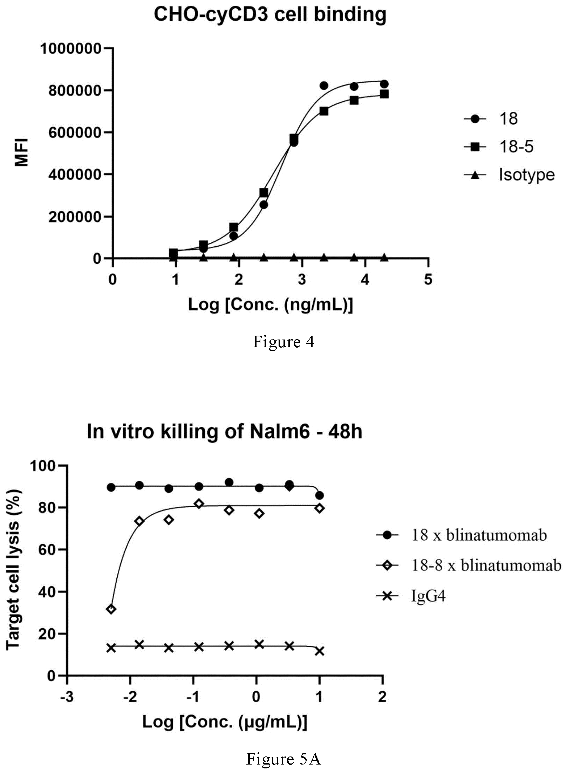

CHO-Cyno CD3e cells (CHO cells obtained from Beijing Zhongyuan Heju Economic and Trade Co., Ltd., with surface overexpression of cynomolgus monkey CD3e) were incubated with various concentrations of humanized anti-CD3 antibodies 18 and 18-5, as well as the control molecule Sp34 (starting concentration 20 µg/mL, 3-fold serial dilution, 8 concentration gradients in total) at 4°C for 30 minutes. The cells were then washed and incubated with a fluorescence-labeled secondary antibody for 30 minutes at 4°C in the dark. Finally, the plates were washed twice with flow cytometry staining buffer, and the cells were resuspended in 100 µL of staining buffer for flow cytometry analysis. Raw data were analyzed using FlowJo software to obtain MFI values, and antibody dose-dependent binding curves were fitted using GraphPad. As shown in Figure 4, both 18 and 18-5 bound to cynomolgus monkey CD3.

### Example 7: Preparation of CD3xCD19 bispecific antibodies using Duobody method and verification of their in vitro killing activity

In the present invention, the DuoBody in vitro recombination method (see patent CN103097417B, Example 1 for details) was used to prepare CD3xCD19 bispecific antibodies. For the CD3 end, clones 18 and 18-8 were selected; for the CD19 end, the CD19 end from Amgen's marketed bispecific antibody blinatumomab was used (the sequences of the CD19 end antibody were as follows: the heavy chain variable region sequence was SEQ ID NO: 70, the light chain variable region sequence was SEQ ID NO: 71, the heavy chain constant region sequence was SEQ ID NO: 72, the light chain constant region sequence was SEQ ID NO: 62, the heavy chain sequence was SEQ ID NO: 68, and the light chain sequence was SEQ ID NO: 69). This yielded two bispecific antibodies: 18×blinatumomab and 18-8×blinatumomab.

Human peripheral blood mononuclear cells (PBMCs, Stemexpress, Cat. No.: PBMNC50C) were resuscitated, and total human T cells were isolated and purified using a total T cell isolation kit (Miltenyi Biotec, Cat. No.: 130-096-535). The purified total human T cells (100,000 cells per well) and Nalm-6 cells (20,000 cells per well, derived from ATCC, Cat. No.: CRL-3273) pre-labeled with cell-trace violet (Invitrogen, Cat. No. C34557), along with serially diluted CD3xCD19 bispecific antibodies (starting concentration 10 µg/ml, 3-fold serial dilutions, 8 concentration gradients in total), were co-incubated in 96-well plates at 37°C for 48 hours. After incubation, the cells were collected and stained with FITC-labeled anti-CD4 (BD, Cat. No.: 550628), BV711-labeled anti-CD8 (BD, Cat. No.: 563677), APC-labeled anti-CD69 (BD, Cat. No.: 555533), and APC-Cy7-labeled anti-CD25 (BD, Cat. No.: 557753) at 4°C for 30 minutes. Following incubation, the cells were washed with flow cytometry staining buffer, and dead cells were labeled with propidium iodide (PI) staining solution (Sigma, Cat. No. P4864) at a final concentration of 1 µg/ml. Cells were then analyzed on a flow cytometer (BD, Fortessa). FlowJo software was used to analyze the killing rate of target Nalm-6 cells and the activation of CD4+ and CD8+ T cells.

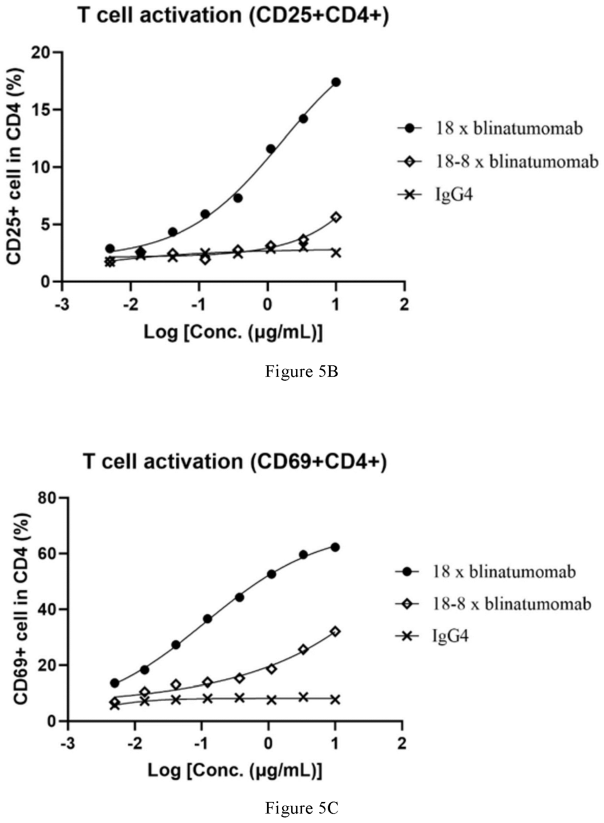

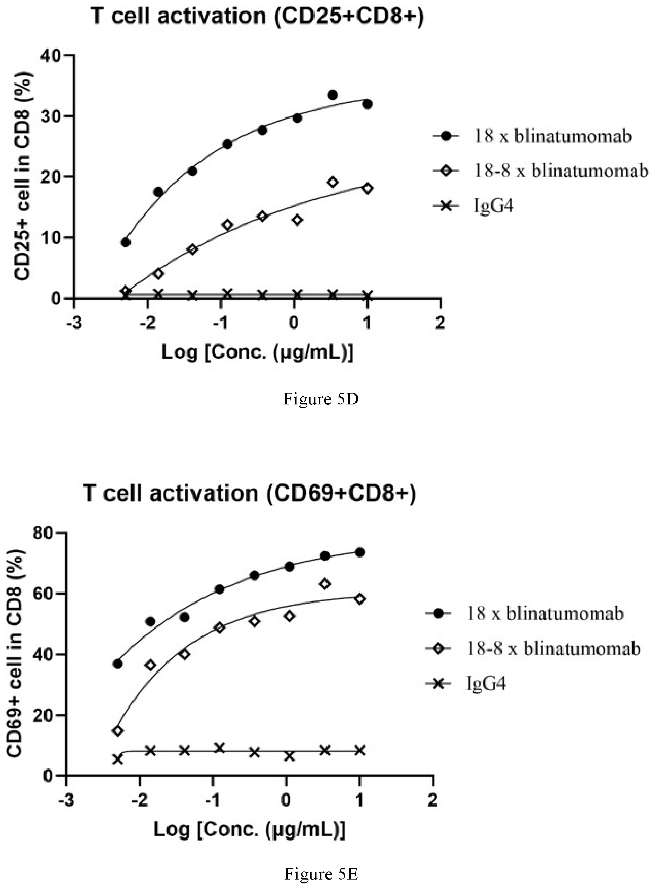

As shown in Figure 5A, after 48 hours of co-culture, both CD3xCD19 bispecific antibodies of the present invention mediated the killing of target cells, with a maximum killing rate approaching 90%. Compared to 18xblinatumomab, the killing activity of the 18-8 bispecific antibody was significantly weaker, and its killing potency correlated with the T cell binding activity at the CD3 terminus. The CD3xCD19 bispecific antibodies effectively stimulated CD4+ and CD8+ T cells to upregulate the membrane surface activation molecules CD25 and CD69, among which 18xblinatumomab exhibited stronger T cell activation (Figures 5B to 5E). These results indicate that the CD3 bispecific antibodies in Duobody form of the present invention possessed moderate T cell activation activity, ensuring efficient killing while reducing the risk of cytokine storm-related side effects.

### Example 8: Preparation of CD3xBCMA bispecific antibodies using Duobody method and verification of their in vitro cytotoxic activity

To verify the biological activity of the anti-CD3 antibody, the present invention employed Genmab's Duobody in vitro reassembly method (see details in Patent CN103097417B, Example 1) to generate CD3xBCMA bispecific antibodies. For the CD3 end, clone 18-8 was selected, with Sp34 serving as a potent control. For the BCMA end, an antibody developed by Junshi Biosciences, designated BCMA-005, was used (wherein the heavy chain variable region sequence was SEQ ID NO: 79, in which the HCDR1 sequence of the heavy chain variable region was SEQ ID NO: 73, the HCDR2 sequence of the heavy chain variable region was SEQ ID NO: 74, and the HCDR3 sequence of the heavy chain variable region was SEQ ID NO: 75; the light chain variable region sequence was SEQ ID NO: 80, in which the LCDR1 sequence of the light chain variable region was SEQ ID NO: 76, the LCDR2 sequence of the light chain variable region was SEQ ID NO: 77, and the LCDR3 sequence of the light chain variable region was SEQ ID NO: 79; the heavy chain constant region sequence was SEQ ID NO: 72; and the light chain constant region sequence was SEQ ID NO: 62). This resulted in two bispecific antibodies: 18-8xBCMA-005 and sp34xBCMA-005.

Human peripheral blood mononuclear cells (PBMCs, Stemexpress, Cat. No.: PBMNC50C) were resuscitated, and total human T cells were isolated and purified using a total T cell isolation kit (Miltenyi Biotec, Cat. No.: 130-096-535). The purified total human T cells (100,000 cells/well) and NCI-H929 cells (obtained from ATCC, Cat. No.: CRL-3580) pre-labeled with cell-trace violet (Invitrogen, Cat. No.: C34557) (20,000 cells/well) were co-incubated with serially diluted CD3xBCMA bispecific antibodies (starting concentration 10 µg/ml, 3-fold serial dilution, 8 concentration gradients in total) in 96-well plates at 37°C for 24 or 48 hours. Finally, cells were collected and stained for flow cytometry with FITC-labeled anti-CD4 (BD, Cat. No.: 550628), BV711-labeled anti-CD8 (BD, Cat. No.: 563677), APC-labeled anti-CD69 (BD, Cat. No.: 555533), and APC-Cy7-labeled anti-CD25 (BD, Cat. No.: 557753) for 30 minutes at 4°C. After incubation, cells were washed with PBS, then dead cells were stained with propidium iodide (PI) solution (Sigma, Cat. No.: P4864) at a final concentration of 1 µg/ml. Cells were analyzed on a flow cytometer (BD, Fortessa). FlowJo software was used to analyze the killing rate of target NCI-H929 cells and the activation of CD4+ and CD8+ T cells.

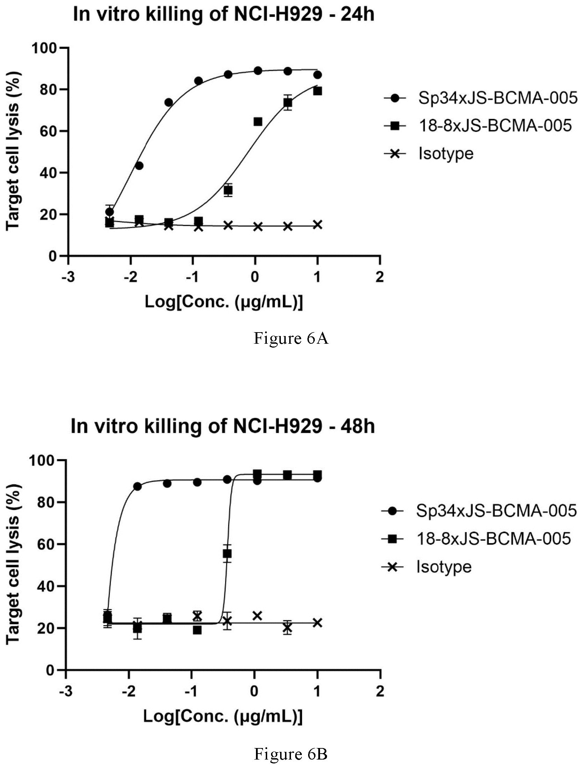

As shown in Figure 6A, after 24 hours of co-incubation, the 18-8xBCMA-005 of the present invention mediated the killing of target NCI-H929 cells by T cells, with a maximum killing rate of 80%. Compared to the potent CD3 control molecule sp34xBCMA-005, the killing activity of 18-8xBCMA-005 was significantly weaker, with EC₅₀ values of 9.6 ng/mL and 780 ng/mL, respectively. As shown in Figure 6B, after 48 hours of co-incubation, 18-8xBCMA-005 achieved the same maximum killing rate as sp34xBCMA-005 (>90%).

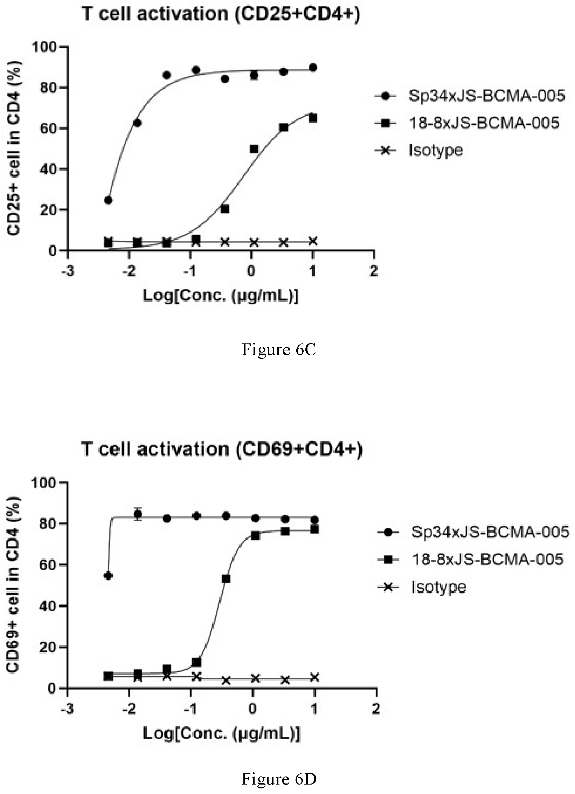

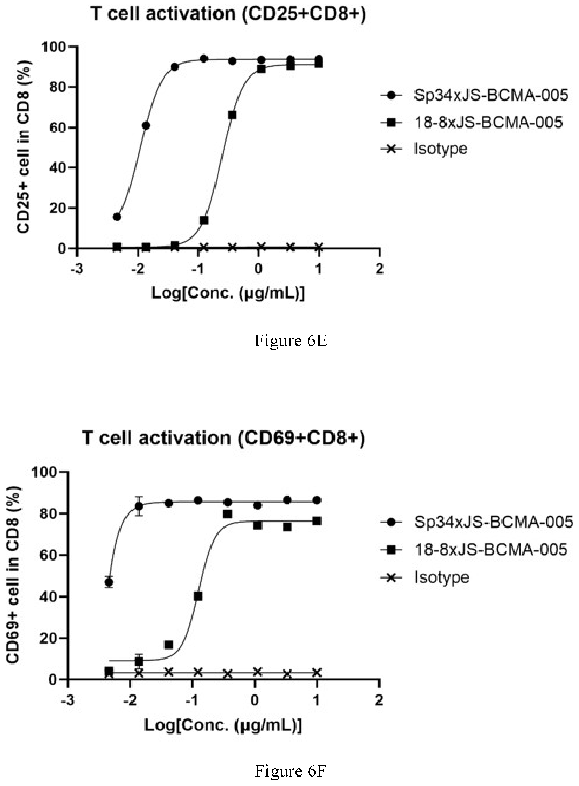

CD69 is a marker for early T cell activation, while CD25 is a marker for late T cell activation. Analysis of these two T cell activation markers reveals the degree of T cell activation induced by the CD3 bispecific antibodies. As shown in Figures 6C and 6D, after 48 hours of co-incubation with target cells, 18-8xBCMA-005 upregulated CD25 and CD69 expression on CD4+ T cells in a dose-dependent manner. Similarly, for CD8+ T cells, 18-8xBCMA-005 induced upregulation of CD25 and CD69 expression (see Figures 6E and 6F). Compared to sp34xBCMA-005, the T cell activation effect of the 18-8xBCMA-005 bispecific antibody of the present invention was significantly weaker. These results indicate that the CD3 bispecific antibodies in Duobody form of the present invention exhibited moderate T cell activation activity, ensuring efficient cell killing while reducing the side effects of cytokine storm.

### Example 9: Cytokine release induced by CD3 bispecific antibody in Duobody form

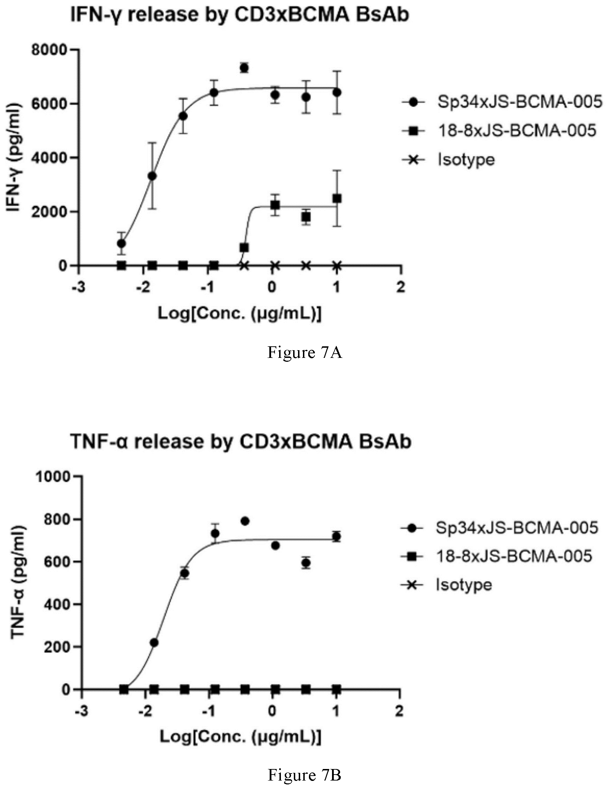

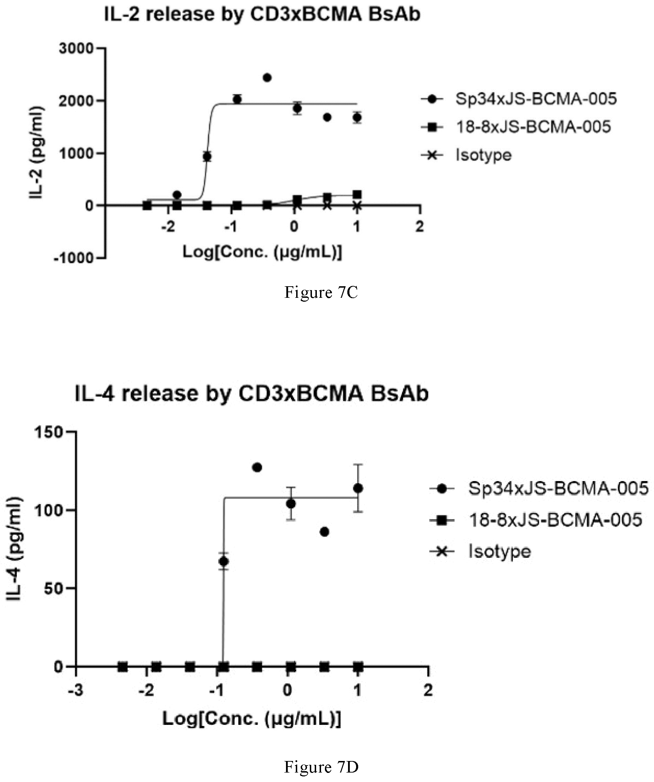

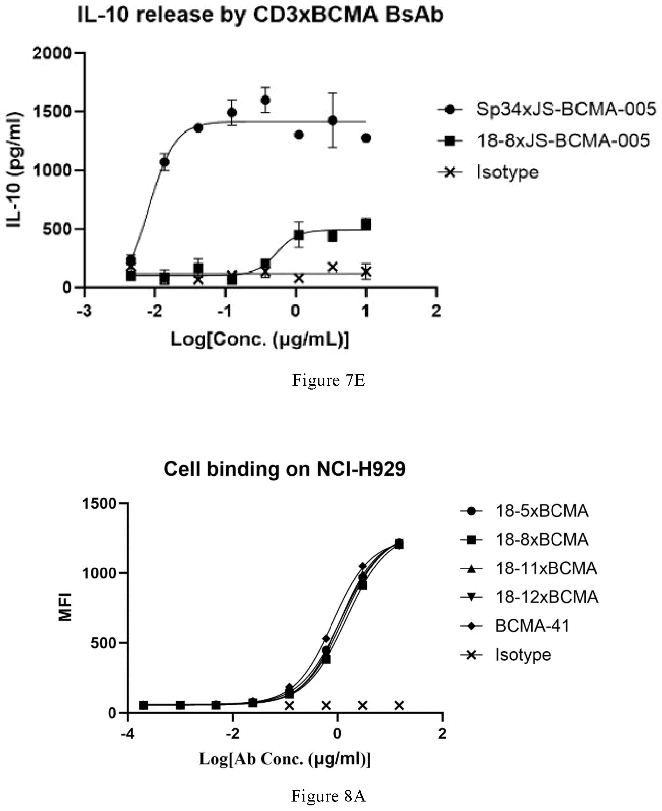

T cells were isolated and purified from PBMCs using Miltenyi's magnetic bead-based cell separation kit. The T cells and NCI-H929 target cells were then co-cultured in 96-well U-bottom plates at an effector-to-target ratio of 5:1 (50,000 T cells and 10,000 target cells per well, respectively). At the same time, serially diluted CD3xBCMA bispecific antibodies (starting concentration 10 µg/ml, 3-fold serial dilutions, 8 concentration gradients in total) were added, and the plates were incubated at 37°C for 24 hours. After incubation, the cell culture supernatant was collected by centrifugation, and the levels of cytokines were measured using a CBA multi-cytokine detection kit (BD, Cat. No.: 551809). As shown in Figures 7A-E, compared to the control molecule sp34xBCMA-005, the 18-8xBCMA-005 antibody of the present invention induced significantly lower release levels of cytokines, including INF-γ, TNF-α, IL-2, IL-4, and IL-10. While achieving the comparable maximum killing rate, 18-8xBCMA-005 exhibited significantly lower release levels of cytokines, potentially demonstrating better safety profile in clinical applications.

### Example 10: In vitro binding activity of common light chain CD3xBCMA bispecific antibodies

To prepare common light chain bispecific antibodies, the light chains of CD3-humanized molecules (18, 18-5, 18-8, 18-11, 18-12, 18-13, 18-14) were paired with the heavy chain of the anti-BCMA antibody (BCMA-41) (whose sequence composition is shown in Table 8), resulting in seven anti-BCMA antibodies. The CD3-humanized molecules and the anti-BCMA antibody were reassembled in vitro to ultimately generate seven CD3xBCMA bispecific antibodies sharing common light chains. Since the light chains of 18 and 18-5 were the same, the light chains of 18-11 and 18-13 were the same, and the light chains of 18-12 and 18-14 were the same, one molecule from each pair was selected for the assessment of binding activity of the common light chain CD3xBCMA bispecific antibodies to the BCMA end.

**Table 8: BCMA-41 heavy chain sequence composition**

| | HCDR1 | HCDR2 | HCDR3 | VH | CH | HC |
|---|---|---|---|---|---|---|
| | (SEQ ID NO: ) | (SEQ ID NO: ) | (SEQ ID NO: ) | (SEQ ID NO: ) | (SEQ ID NO: ) | (SEQ ID NO: ) |
| NP010-Hz018-VH | 73 | 74 | 83 | 84 | 72 | 85 |

In a 96-well U-bottom cell culture plate, 50 µl of NCI-H929 cells (1x10⁵ cells in total) with high BCMA expression were added, followed by 50 µl of serially diluted CD3xBCMA bispecific antibodies (starting concentration 15 µg/ml, 5-fold dilution, 8 concentration gradients in total). The mixture was co-incubated in the 96-well plate at 4°C for 30 minutes. After incubation, the plate was washed twice with flow cytometry staining buffer (BD, Cat. No.: 554658) to remove unbound antibodies. Then, 50 µl of Alexa Fluor 488-labeled Goat Anti-Human IgG secondary antibody (Jackson Lab, Cat. No.: 109-546-098, 1:1000 dilution) was added and incubated at 4°C for 1 hour. Finally, the plate was washed twice with flow cytometry staining buffer, and the cells were resuspended in 150 µl of staining buffer for flow cytometry analysis. Data were analyzed using Flowjo software. As shown in Figure 8A, using the light chain of the CD3 antibody as the common light chain, after generating the CD3xBCMA common light chain bispecific antibodies with the BCMA-41 antibody, the binding activity of their BCMA end was well maintained, comparable to that of the bivalent BCMA-41 antibody.

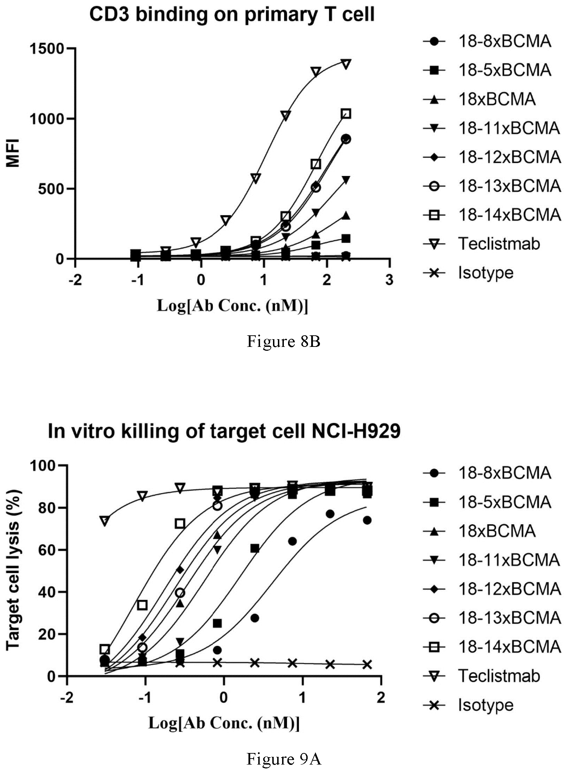

Human peripheral blood mononuclear cells (PBMCs, Stemexpress, Cat. No.: PBMNC50C) were resuscitated, and total human T cells were isolated and purified using a total T cell isolation kit (Miltenyi Biotec, Cat. No.: 130-096-535). Then, 50 µl of purified human T cells (1x10⁵ cells per well) were added to a 96-well U-bottom cell culture plate, followed by 50 µl of serially diluted CD3xBCMA common light chain bispecific antibodies (starting concentration 200 nM, 3-fold dilution, 8 concentration gradients in total). The mixture was co-incubated in the 96-well plate at 4°C for 30 minutes. After incubation, the plate was washed twice with flow cytometry staining buffer (BD, Cat. No.: 554658) to remove unbound antibodies. Then, 50 µl of PE-labeled Goat Anti-Human IgG secondary antibody (Biolegend, Cat. No.: 398004, 1:1000 dilution) was added and incubated at 4°C for 1 hour. Finally, the plate was washed twice with flow cytometry staining buffer, and the cells were resuspended in 150 µl of staining buffer for flow cytometry analysis. Data were analyzed using Flowjo software. As shown in Figure 8B, compared to the marketed Teclistamab from Johnson & Johnson, the CD3xBCMA bispecific antibodies of the present invention all exhibited weaker binding to T cells, with binding strengths in descending order of: 18-14 > 18-12 = 18-13 > 18-11 > 18 > 18-5 > 18-8, indicating that they could effectively reduce the occurrence of cytokine storm.

### Example 11: In vitro cytotoxic activity of common light chain CD3xBCMA bispecific antibodies

Human peripheral blood mononuclear cells (PBMCs, Stemexpress, Cat. No.: PBMNC50C) were resuscitated. Total human T cells were isolated and purified using a total T cell isolation kit (Miltenyi Biotec, Cat. No.: 130-096-535). The purified total human T cells (100,000 cells per well) and NCI-H929 cells pre-labeled with cell-trace violet (Invitrogen, Cat. No.: C34557) (20,000 cells per well) were co-incubated with serially diluted common light chain CD3xBCMA bispecific antibodies (starting concentration 66.7 nM, 3-fold serial dilutions, 8 concentration points in total) in 96-well plates at 37°C for 24 hours. After incubation, cells were collected and stained with FITC-labeled anti-CD4 (BD, Cat. No.: 550628), BV711-labeled anti-CD8 (BD, Cat. No.: 563677), APC-labeled anti-CD69 (BD, Cat. No.: 555533), and APC-Cy7-labeled anti-CD25 (BD, Cat. No.: 557753) for 30 minutes at 4°C. Following incubation, cells were washed with flow cytometry staining buffer, then dead cells were stained with propidium iodide (PI) staining solution (Sigma, Cat. No.: P4864) at a final concentration of 1 µg/mL. Cells were analyzed on a flow cytometer (BD, Fortessa). FlowJo software was used to analyze the killing rate of target NCI-H929 cells and the activation of CD4+ and CD8+ T cells.

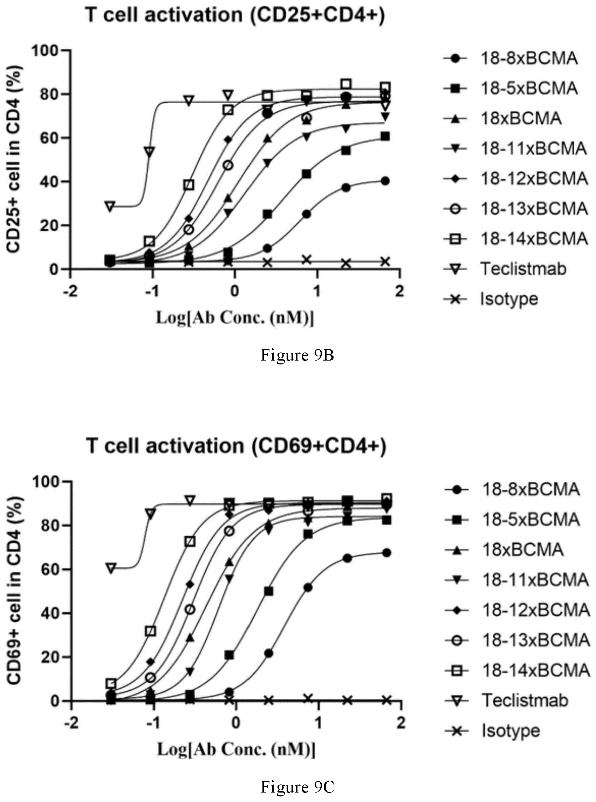

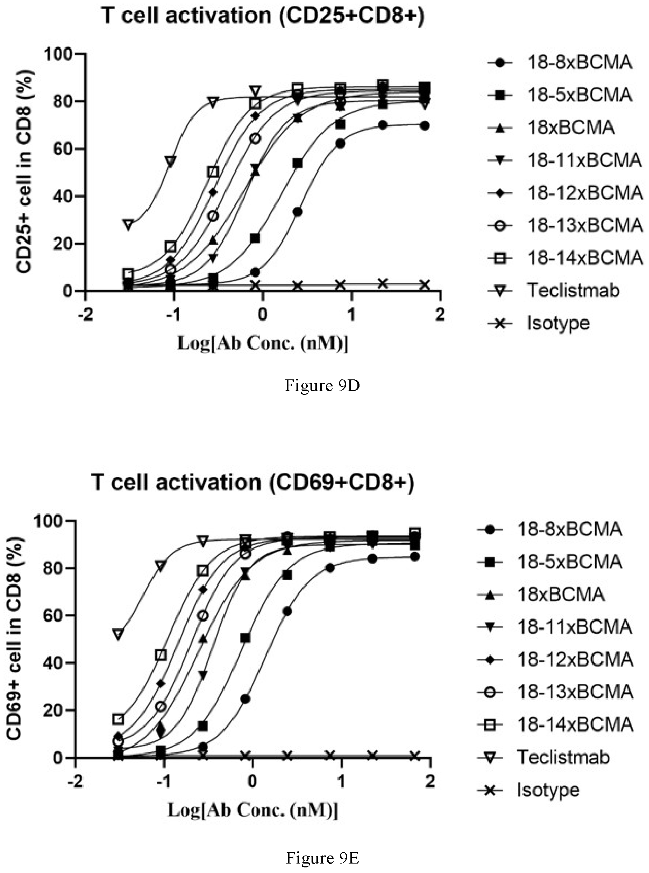

As shown in Figure 9A, after 24 hours of co-incubation, the common light chain CD3xBCMA bispecific antibodies of the present invention mediated the killing of target NCI-H929 cells, with a maximum killing rate approaching 90%. Compared to Teclistamab, the killing activity of the common light chain bispecific antibodies was noticeably weaker, and the strength of killing activity correlated with the T cell binding activity of the CD3 domain. Among them, the bispecific antibody with the weakest activity was 18-8xBCMA; nevertheless, under high concentration conditions, it still achieved the same maximum killing rate after 24 hours.

The common light chain bispecific antibodies could effectively stimulate CD4+ and CD8+ T cells to upregulate the membrane surface activation molecules CD25 and CD69. Among them, 18-8xBCMA showed the weakest T cell activation, while 18-14xBCMA exhibited the strongest T cell stimulation, though still weaker than the control molecule Teclistamab (Figures 9B to 9E). These results indicate that the common light chain format CD3 bispecific antibodies of the present invention possessed moderate T cell activation activity, ensuring efficient killing while potentially reducing the side effects of cytokine storm.

### Example 12: In vivo antitumor activity of common light chain CD3xBCMA bispecific antibodies

Female immunodeficient NDG mice, aged 6 to 8 weeks (purchased from Biocytogen Biotechnology Co., Ltd.), were injected via the tail vein with 5×10⁶ NCI-H929-Luc cells (purchased from Nanjing Cobioer Biotechnology Co., Ltd., Cat. No.: CBP30061L). This time point was recorded as Day 0. Thirteen days later, 1×10⁷ PBMCs (Stemexpress, Cat. No.: PBMNC50C) were injected via the tail vein. After 4 days, each mouse was injected via the tail vein with 3 mg of luciferase substrate D-luciferin potassium salt (Fluorescence Biotechnology Co. Ltd., Cat. No.: 12505). Tumor size was determined by detecting the luciferase substrate signal using a small animal in vivo imaging system (Guangzhou Boluteng Biotechnology Co., Ltd., Model: Aniview100). Based on tumor size, the mice were randomly divided into 5 groups, each containing 5 animals, as follows:
G1: Saline control group
G2: 18-8xBCMA41
G3: 18xBCMA41
G4: 18-14xBCMA41
G5: Teclistamab.

The common light chain bispecific antibodies 18-8xBCMA41, 18xBCMA41, and 18-14xBCMA41 were administered via tail vein injection at a dose of 10 µg per mouse. Teclistamab was used as the control antibody in this experiment. Subsequently, the antibodies were administered once every 5 days for a total of 4 doses. Tumor growth was monitored by tail vein injection of D-luciferin potassium salt (3 mg per animal), followed by detection of the luciferase substrate signal using the AniView100 small animal in vivo imaging system. Higher signal values indicated faster tumor growth.

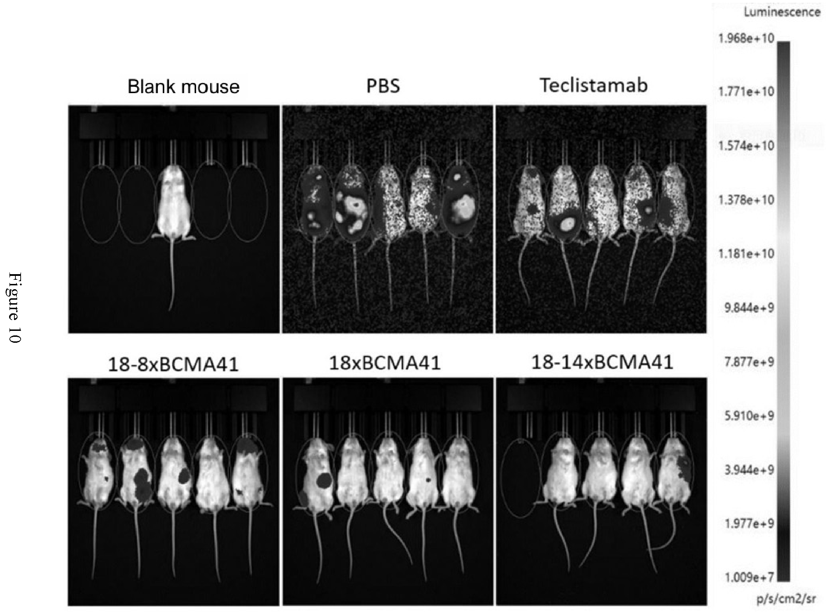

As shown in Figure 10, compared with the control antibody Teclistamab, the common light chain CD3×BCMA antibodies of the present invention all demonstrated favorable anti-tumor efficacy. Notably, the two bispecific antibodies 18xBCMA41 and 18-14xBCMA41 achieved complete or near-complete tumor regression in 4 mice and 3 mice, respectively.

Although specific embodiments of the present invention have been described in detail, those skilled in the art will understand that various modifications and variations can be made to the details based on all the disclosed teachings, and all such changes are within the scope of protection of the present invention. The full scope of the present invention is given by the appended claims and any equivalents thereof.

## Claims

1. An antibody or antigen-binding fragment thereof capable of specifically binding to CD3, wherein the antibody or antigen-binding fragment thereof comprises: HCDR1 with an amino acid sequence as set forth in SEQ ID NO: 16, 54, 1, 9, or 29; HCDR2 with an amino acid sequence as set forth in SEQ ID NO: 55, 56, 17, 2, 10, 24, 30, or 37; HCDR3 with an amino acid sequence as set forth in SEQ ID NO: 18, 3, 11, 25, 31, or 38; LCDR1 with an amino acid sequence as set forth in SEQ ID NO: 42, 43, 20, 5, 13, or 33; LCDR2 with an amino acid sequence as set forth in SEQ ID NO: 21, 45, 46, 6, 14, 27, 34, or 44; and LCDR3 with an amino acid sequence as set forth in SEQ ID NO: 22, 7, 35, or 40.

2. An antibody or antigen-binding fragment thereof capable of specifically binding to CD3, wherein the antibody or antigen-binding fragment thereof comprises a heavy chain variable region and/or a light chain variable region:
the heavy chain variable region comprises:
(I) HCDR1, HCDR2, and HCDR3 with amino acid sequences as set forth in SEQ ID NO: 16, SEQ ID NO: 55, and SEQ ID NO: 18, respectively; or HCDR1, HCDR2, and HCDR3 having 1, 2, or 3 amino acid differences from the amino acid sequences as set forth in SEQ ID NO: 16, SEQ ID NO: 55, and SEQ ID NO: 18, respectively;
(II) HCDR1, HCDR2, and HCDR3 with amino acid sequences as set forth in SEQ ID NO: 16, SEQ ID NO: 56, and SEQ ID NO: 18, respectively; or HCDR1, HCDR2, and HCDR3 having 1, 2, or 3 amino acid differences from the amino acid sequences as set forth in SEQ ID NO: 16, SEQ ID NO: 56, and SEQ ID NO: 18, respectively;
(III) HCDR1, HCDR2, and HCDR3 with amino acid sequences as set forth in SEQ ID NO: 54, SEQ ID NO: 56, and SEQ ID NO: 18, respectively; or HCDR1, HCDR2, and HCDR3 having 1, 2, or 3 amino acid differences from the amino acid sequences as set forth in SEQ ID NO: 54, SEQ ID NO: 56, and SEQ ID NO: 18, respectively;
(IV) HCDR1, HCDR2, and HCDR3 with amino acid sequences as set forth in SEQ ID NO: 16, SEQ ID NO: 17, and SEQ ID NO: 18, respectively; or HCDR1, HCDR2, and HCDR3 having 1, 2, or 3 amino acid differences from the amino acid sequences as set forth in SEQ ID NO: 16, SEQ ID NO: 17, and SEQ ID NO: 18, respectively;
(V) HCDR1, HCDR2, and HCDR3 with amino acid sequences as set forth in SEQ ID NO: 1, SEQ ID NO: 2, and SEQ ID NO: 3; or HCDR1, HCDR2, and HCDR3 having 1, 2, or 3 amino acid differences from the amino acid sequences as set forth in SEQ ID NO: 1, SEQ ID NO: 2, and SEQ ID NO: 3, respectively;
(VI) HCDR1, HCDR2, and HCDR3 with amino acid sequences as set forth in SEQ ID NO: 9, SEQ ID NO: 10, and SEQ ID NO: 11, respectively; or HCDR1, HCDR2, and HCDR3 having 1, 2, or 3 amino acid differences from the amino acid sequences as set forth in SEQ ID NO: 9, SEQ ID NO: 10, and SEQ ID NO: 11, respectively;
(VII) HCDR1, HCDR2, and HCDR3 with amino acid sequences as set forth in SEQ ID NO: 9, SEQ ID NO: 24, and SEQ ID NO: 25, respectively; or HCDR1, HCDR2, and HCDR3 having 1, 2, or 3 amino acid differences from the amino acid sequences as set forth in SEQ ID NO: 9, SEQ ID NO: 24, and SEQ ID NO: 25, respectively;
(VIII) HCDR1, HCDR2, and HCDR3 with amino acid sequences as set forth in SEQ ID NO: 29, SEQ ID NO: 30, and SEQ ID NO: 31, respectively; HCDR1, HCDR2, and HCDR3 having 1, 2, or 3 amino acid differences from the amino acid sequences as set forth in SEQ ID NO: 29, SEQ ID NO: 30, and SEQ ID NO: 31, respectively; or
(IX) HCDR1, HCDR2, and HCDR3 with amino acid sequences as set forth in SEQ ID NO: 9, SEQ ID NO: 37, and SEQ ID NO: 38, respectively; or HCDR1, HCDR2, and HCDR3 having 1, 2, or 3 amino acid differences from the amino acid sequences as set forth in SEQ ID NO: 9, SEQ ID NO: 37, and SEQ ID NO: 38, respectively;
the light chain variable region comprises:
(I) LCDR1, LCDR2, and LCDR3 with amino acid sequences as set forth in SEQ ID NO: 42, SEQ ID NO: 21, and SEQ ID NO: 22, respectively; or LCDR1, LCDR2, and LCDR3 having 1, 2, or 3 amino acid differences from the amino acid sequences as set forth in SEQ ID NO: 42, SEQ ID NO: 21, and SEQ ID NO: 22, respectively;
(II) LCDR1, LCDR2, and LCDR3 with amino acid sequences as set forth in SEQ ID NO: 42, SEQ ID NO: 44, and SEQ ID NO: 22, respectively; or LCDR1, LCDR2, and LCDR3 having 1, 2, or 3 amino acid differences from the amino acid sequences as set forth in SEQ ID NO: 42, SEQ ID NO: 44, and SEQ ID NO: 22, respectively;
(III) LCDR1, LCDR2, and LCDR3 with amino acid sequences as set forth in SEQ ID NO: 43, SEQ ID NO: 45, and SEQ ID NO: 22, respectively; or LCDR1, LCDR2, and LCDR3 having 1, 2, or 3 amino acid differences from the amino acid sequences as set forth in SEQ ID NO: 43, SEQ ID NO: 45, and SEQ ID NO: 22, respectively;
(IV) LCDR1, LCDR2, and LCDR3 with amino acid sequences as set forth in SEQ ID NO: 43, SEQ ID NO: 46, and SEQ ID NO: 22, respectively; or LCDR1, LCDR2, and LCDR3 having 1, 2, or 3 amino acid differences from the amino acid sequences as set forth in SEQ ID NO: 43, SEQ ID NO: 46, and SEQ ID NO: 22, respectively;
(V) LCDR1, LCDR2, and LCDR3 with amino acid sequences as set forth in SEQ ID NO: 20, SEQ ID NO: 21, and SEQ ID NO: 22, respectively; or LCDR1, LCDR2, and LCDR3 having 1, 2, or 3 amino acid differences from the amino acid sequences as set forth in SEQ ID NO: 20, SEQ ID NO: 21, and SEQ ID NO: 22, respectively;
(VI) LCDR1, LCDR2, and LCDR3 with amino acid sequences as set forth in SEQ ID NO: 5, SEQ ID NO: 6, and SEQ ID NO: 7, respectively; or LCDR1, LCDR2, and LCDR3 having 1, 2, or 3 amino acid differences from the amino acid sequences as set forth in SEQ ID NO: 5, SEQ ID NO: 6, and SEQ ID NO: 7, respectively;
(VII) LCDR1, LCDR2, and LCDR3 with amino acid sequences as set forth in SEQ ID NO: 13, SEQ ID NO: 14, and SEQ ID NO: 7, respectively; or LCDR1, LCDR2, and LCDR3 having 1, 2, or 3 amino acid differences from the amino acid sequences as set forth in SEQ ID NO: 13, SEQ ID NO: 14, and SEQ ID NO: 7, respectively;
(VIII) LCDR1, LCDR2, and LCDR3 with amino acid sequences as set forth in SEQ ID NO: 13, SEQ ID NO: 27, and SEQ ID NO: 7, respectively; or LCDR1, LCDR2, and LCDR3 having 1, 2, or 3 amino acid differences from the amino acid sequences as set forth in SEQ ID NO: 13, SEQ ID NO: 27, and SEQ ID NO: 7, respectively;
(IX) LCDR1, LCDR2, and LCDR3 with amino acid sequences as set forth in SEQ ID NO: 33, SEQ ID NO: 34, and SEQ ID NO: 35, respectively; or LCDR1, LCDR2, and LCDR3 having 1, 2, or 3 amino acid differences from the amino acid sequences as set forth in SEQ ID NO: 33, SEQ ID NO: 34, and SEQ ID NO: 35, respectively; or
(X) LCDR1, LCDR2, and LCDR3 with amino acid sequences as set forth in SEQ ID NO: 13, SEQ ID NO: 6, and SEQ ID NO: 40, respectively; or LCDR1, LCDR2, and LCDR3 having 1, 2, or 3 amino acid differences from the amino acid sequences as set forth in SEQ ID NO: 13, SEQ ID NO: 6, and SEQ ID NO: 40, respectively;
preferably, the antibody or antigen-binding fragment thereof comprises:
(I) a heavy chain variable region, which comprises HCDR1, HCDR2, and HCDR3 with amino acid sequences as set forth in SEQ ID NO: 16, SEQ ID NO: 55, and SEQ ID NO: 18, respectively; and a light chain variable region, which comprises LCDR1, LCDR2, and LCDR3 with amino acid sequences as set forth in SEQ ID NO: 42, SEQ ID NO: 21, and SEQ ID NO: 22, respectively;
(II) a heavy chain variable region, which comprises HCDR1, HCDR2, and HCDR3 with amino acid sequences as set forth in SEQ ID NO: 16, SEQ ID NO: 56, and SEQ ID NO: 18, respectively; and a light chain variable region, which comprises LCDR1, LCDR2, and LCDR3 with amino acid sequences as set forth in SEQ ID NO: 43, SEQ ID NO: 45, and SEQ ID NO: 22, respectively;
(III) a heavy chain variable region, which comprises HCDR1, HCDR2, and HCDR3 with amino acid sequences as set forth in SEQ ID NO: 16, SEQ ID NO: 56, and SEQ ID NO: 18, respectively; and a light chain variable region, which comprises LCDR1, LCDR2, and LCDR3 with amino acid sequences as set forth in SEQ ID NO: 43, SEQ ID NO: 46, and SEQ ID NO: 22, respectively;
(IV) a heavy chain variable region, which comprises HCDR1, HCDR2, and HCDR3 with amino acid sequences as set forth in SEQ ID NO: 16, SEQ ID NO: 17, and SEQ ID NO: 18, respectively; and a light chain variable region, which comprises LCDR1, LCDR2, and LCDR3 with amino acid sequences as set forth in SEQ ID NO: 20, SEQ ID NO: 21, and SEQ ID NO: 22, respectively;
(V) a heavy chain variable region, which comprises HCDR1, HCDR2, and HCDR3 with amino acid sequences as set forth in SEQ ID NO: 1, SEQ ID NO: 2, and SEQ ID NO: 3, respectively; and a light chain variable region, which comprises LCDR1, LCDR2, and LCDR3 with amino acid sequences as set forth in SEQ ID NO: 5, SEQ ID NO: 6, and SEQ ID NO: 7, respectively;
(VI) a heavy chain variable region, which comprises HCDR1, HCDR2, and HCDR3 with amino acid sequences as set forth in SEQ ID NO: 9, SEQ ID NO: 10, and SEQ ID NO: 11, respectively; and a light chain variable region, which comprises LCDR1, LCDR2, and LCDR3 with amino acid sequences as set forth in SEQ ID NO: 13, SEQ ID NO: 14, and SEQ ID NO: 7, respectively;
(VII) a heavy chain variable region, which comprises HCDR1, HCDR2, and HCDR3 with amino acid sequences as set forth in SEQ ID NO: 9, SEQ ID NO: 24, and SEQ ID NO: 25, respectively; and a light chain variable region, which comprises LCDR1, LCDR2, and LCDR3 with amino acid sequences as set forth in SEQ ID NO: 13, SEQ ID NO: 27, and SEQ ID NO: 7, respectively;
(VIII) a heavy chain variable region, which comprises HCDR1, HCDR2, and HCDR3 with amino acid sequences as set forth in SEQ ID NO: 29, SEQ ID NO: 30, and SEQ ID NO: 31, respectively; and a light chain variable region, which comprises LCDR1, LCDR2, and LCDR3 with amino acid sequences as set forth in SEQ ID NO: 33, SEQ ID NO: 34, and SEQ ID NO: 35, respectively; or
(IX) a heavy chain variable region, which comprises HCDR1, HCDR2, and HCDR3 with amino acid sequences as set forth in SEQ ID NO: 9, SEQ ID NO: 37, and SEQ ID NO: 38, respectively; and a light chain variable region, which comprises LCDR1, LCDR2, and LCDR3 with amino acid sequences as set forth in SEQ ID NO: 13, SEQ ID NO: 6, and SEQ ID NO: 40, respectively.
preferably, the antibody or antigen-binding fragment thereof comprises:
(I) a heavy chain variable region, which comprises an amino acid sequence as set forth in any one of SEQ ID NOs: 19, 4, 12, 26, 32, and 39; and a light chain variable region, which comprises an amino acid sequence as set forth in any one of SEQ ID NOs: 23, 8, 15, 28, 36, and 41; or
(II) a heavy chain variable region, which comprises an amino acid sequence having at least 95%, 96%, 97%, 98%, or 99% sequence identity to the amino acid sequence as set forth in any one of SEQ ID NOs: 19, 4, 12, 26, 32, and 39; and a light chain variable region, which comprises an amino acid sequence having at least 95%, 96%, 97%, 98%, or 99% sequence identity to the amino acid sequence as set forth in any one of SEQ ID NOs: 23, 8, 15, 28, 36, and 41;
preferably, the antibody or antigen-binding fragment thereof comprises a heavy chain variable region and a light chain variable region, wherein:
(I) the heavy chain variable region comprises an amino acid sequence as set forth in SEQ ID NO: 19, or comprises an amino acid sequence having at least 95%, 96%, 97%, 98%, or 99% sequence identity to the amino acid sequence as set forth in SEQ ID NO: 19; and the light chain variable region comprises an amino acid sequence as set forth in SEQ ID NO: 23, or comprises an amino acid sequence having at least 95%, 96%, 97%, 98%, or 99% sequence identity to the amino acid sequence as set forth in SEQ ID NO: 23;
(II) the heavy chain variable region comprises an amino acid sequence as set forth in SEQ ID NO: 4, or comprises an amino acid sequence having at least 95%, 96%, 97%, 98%, or 99% sequence identity to the amino acid sequence as set forth in SEQ ID NO: 4; and the light chain variable region comprises an amino acid sequence as set forth in SEQ ID NO: 8, or comprises an amino acid sequence having at least 95%, 96%, 97%, 98%, or 99% sequence identity to the amino acid sequence as set forth in SEQ ID NO: 8;
(III) the heavy chain variable region comprises an amino acid sequence as set forth in SEQ ID NO: 12, or comprises an amino acid sequence having at least 95%, 96%, 97%, 98%, or 99% sequence identity to the amino acid sequence as set forth in SEQ ID NO: 12; and the light chain variable region comprises an amino acid sequence as set forth in SEQ ID NO: 15, or comprises an amino acid sequence having at least 95%, 96%, 97%, 98%, or 99% sequence identity to the amino acid sequence as set forth in SEQ ID NO: 15;
(IV) the heavy chain variable region comprises an amino acid sequence as set forth in SEQ ID NO: 26, or comprises an amino acid sequence having at least 95%, 96%, 97%, 98%, or 99% sequence identity to the amino acid sequence as set forth in SEQ ID NO: 26; and the light chain variable region comprises an amino acid sequence as set forth in SEQ ID NO: 28, or comprises an amino acid sequence having at least 95%, 96%, 97%, 98%, or 99% sequence identity to the amino acid sequence as set forth in SEQ ID NO: 28;
(V) the heavy chain variable region comprises an amino acid sequence as set forth in SEQ ID NO: 32, or comprises an amino acid sequence having at least 95%, 96%, 97%, 98%, or 99% sequence identity to the amino acid sequence as set forth in SEQ ID NO: 32; and the light chain variable region comprises an amino acid sequence as set forth in SEQ ID NO: 36, or comprises an amino acid sequence having at least 95%, 96%, 97%, 98%, or 99% sequence identity to the amino acid sequence as set forth in SEQ ID NO: 36; or
(VI) the heavy chain variable region comprises an amino acid sequence as set forth in SEQ ID NO: 39, or comprises an amino acid sequence having at least 95%, 96%, 97%, 98%, or 99% sequence identity to the amino acid sequence as set forth in SEQ ID NO: 39; and the light chain variable region comprises an amino acid sequence as set forth in SEQ ID NO: 41, or comprises an amino acid sequence having at least 95%, 96%, 97%, 98%, or 99% sequence identity to the amino acid sequence as set forth in SEQ ID NO: 41.

3. The antibody or antigen-binding fragment thereof according to claim 1 or 2, wherein the antibody or antigen-binding fragment thereof comprises:
(I) a heavy chain variable region, which comprises an amino acid sequence as set forth in any one of SEQ ID NOs: 57, 58, 59, 60, and 61; and a light chain variable region, which comprises an amino acid sequence as set forth in any one of SEQ ID NOs: 47, 48, 49, 50, 51, 52, and 53;
(II) a heavy chain variable region, which comprises an amino acid sequence having at least 95%, 96%, 97%, 98% or 99% sequence identity to the amino acid sequence as set forth in any one of SEQ ID NOs: 57, 58, 59, 60, and 61; and a light chain variable region, which comprises an amino acid sequence having at least 95%, 96%, 97%, 98% or 99% sequence identity to the amino acid sequence as set forth in any one of SEQ ID NOs: 47, 48, 49, 50, 51, 52, and 53; or
(III) a heavy chain variable region with an amino acid sequence as set forth in SEQ ID NO: 57 or 58, and a light chain variable region with an amino acid sequence as set forth in SEQ ID NO: 47; a heavy chain variable region with an amino acid sequence as set forth in SEQ ID NO: 58, and a light chain variable region with an amino acid sequence as set forth in SEQ ID NO: 51; a heavy chain variable region with an amino acid sequence as set forth in SEQ ID NO: 60, and a light chain variable region with an amino acid sequence as set forth in SEQ ID NO: 52 or 53; or a heavy chain variable region with an amino acid sequence as set forth in SEQ ID NO: 61, and a light chain variable region with an amino acid sequence as set forth in SEQ ID NO: 52 or 53.

4. The antibody or antigen-binding fragment thereof according to any one of claims 1 to 3, wherein the antibody is selected from the group consisting of murine antibody, chimeric antibody, humanized antibody, or fully human antibody, and the antigen-binding fragment is selected from the group consisting of Fab, Fab', F(ab')₂, Fv, scFv, and sdAb.

5. The antibody or antigen-binding fragment thereof according to any one of claims 1 to 4, wherein the antibody or antigen-binding fragment thereof is any IgG subtype, such as IgG1, IgG2, IgG3, or IgG4, preferably IgG4 subtype.

6. The antibody or antigen-binding fragment thereof according to any one of claims 1 to 5, wherein the antibody comprises a heavy chain constant region as set forth in SEQ ID NO: 63 and/or a light chain constant region as set forth in SEQ ID NO: 62.

7. A multispecific antibody, which comprises an antigen-binding domain targeting CD3 and at least one antigen-binding domain targeting other antigen, wherein the antigen-binding domain targeting CD3 is selected from the antibody or antigen-binding fragment thereof according to any one of claims 1 to 6, and the other antigen is selected from a tumor-associated antigen (TAA) or a tumor-specific antigen (TSA).

8. The multispecific antibody according to claim 7, wherein the multispecific antibody is a bispecific antibody, a trispecific antibody, or a tetraspecific antibody; preferably, the multispecific antibody is a bispecific antibody, which comprises the antigen-binding domain targeting CD3 and the antigen-binding domain targeting the tumor-associated antigen or tumor-specific antigen.

9. The multispecific antibody according to any one of claims 7 to 8, wherein the tumor-associated antigen or tumor-specific antigen is selected from the group consisting of CD19, CD20, CD22, EGFR, HER2, HER3, PSMA, EpCAM, EphA2, CD30, CD33, CD38, CD79b, CD123, CLDN18.2, MSLN, GUCY2C, AFP, PAP, TROP2, LRRC15, gp100, 5T4, CEA, UPK2, DLL3, PRAME, CDH17, CDH19, GPA33, FAP, GPRC5D, GPC3, B7-H3, CLL-1, LIV-1, ACPP, CLDN6, PSCA, ENPP3, PRLR, MUC16, MUC17, CCR5, GD2, GD3, Ras, LewisY, BORIS, NY-ESO-1, OY-TES1, TSHR, LY6K, FLt3, IGFR-1, CAIX, c-MET, TROP2, and any combination thereof; preferably, the tumor-associated antigen is selected from CD19.

10. The multispecific antibody according to any one of claims 7 to 8, comprising the antigen-binding domain targeting CD3 and the antigen-binding domain targeting CD19; wherein the antigen-binding domain targeting CD19 comprises a light chain variable region and a heavy chain variable region, the heavy chain variable region comprises a sequence as set forth in SEQ ID NO: 70 or a variant thereof, and the light chain variable region comprises a sequence as set forth in SEQ ID NO: 71 or a variant thereof.

11. The multispecific antibody according to any one of claims 7 to 10, wherein the multispecific antibody is a bispecific antibody in the form of a DuoBody, comprising:
i) peptide chain I-A, which comprises a VL of the antigen-binding domain targeting CD3, and a first light chain constant region (CL); preferably, the first light chain constant region is kappa;
ii) peptide chain I-B, which comprises a VH of the antigen-binding domain targeting CD3, and a first heavy chain constant region (CH); preferably, the first heavy chain constant region is IgG, such as IgG1, IgG2, IgG3, or IgG4;
iii) peptide chain I-C, which comprises a VH of the antigen-binding domain targeting TAA or TSA, and a second heavy chain constant region; preferably, the second heavy chain constant region is IgG, such as IgG1, IgG2, IgG3, or IgG4; and
iv) peptide chain I-D, which comprises a VL of the antigen-binding domain targeting TAA or TSA, and a second light chain constant region; preferably, the second light chain constant region is kappa;
preferably, the Fc domains comprised in the peptide chains I-B and I-C comprise modifications to promote dimerization of I-B and I-C, respectively;
preferably, the peptide chain I-C comprises a VH sequence as set forth in SEQ ID NO: 70 or a variant thereof, and the peptide chain I-D comprises a VL sequence as set forth in SEQ ID NO: 71 or a variant thereof;
preferably, the peptide chain I-B comprises a VH sequence as set forth in SEQ ID NO: 58, 57, 60, or 61, or a variant thereof, and the peptide chain I-A comprises a VL sequence as set forth in SEQ ID NO: 51, 47, 52, or 53, or a variant thereof;
preferably, the peptide chain I-B comprises a VH sequence as set forth in SEQ ID NO: 58 or 57, or a variant thereof, and the peptide chain I-A comprises a VL sequence as set forth in SEQ ID NO: 51 or 47, or a variant thereof.

12. The multispecific antibody according to any one of claims 7 to 8, wherein the antigen-binding domain targeting CD3 comprises HCDR1, HCDR2, and HCDR3 with amino acid sequences as set forth in SEQ ID NO: 16, SEQ ID NO: 56, and SEQ ID NO: 18, respectively, and LCDR1, LCDR2, and LCDR3 with amino acid sequences as set forth in SEQ ID NO: 43, SEQ ID NO: 46, and SEQ ID NO: 22, respectively.

13. The multispecific antibody according to claim 12, which is a bispecific antibody in the form of a common light chain.

14. The multispecific antibody according to claim 13, wherein the common light chain comprises LCDR1, LCDR2, and LCDR3 with amino acid sequences as set forth in SEQ ID NO: 42, SEQ ID NO: 21, and SEQ ID NO: 22, respectively; or LCDR1, LCDR2, and LCDR3 with amino acid sequences as set forth in SEQ ID NO: 43, SEQ ID NO: 45, and SEQ ID NO: 22, respectively; or LCDR1, LCDR2, and LCDR3 with amino acid sequences as set forth in SEQ ID NO: 43, SEQ ID NO: 46, and SEQ ID NO: 22, respectively; preferably, the common light chain comprises LCDR1, LCDR2, and LCDR3 with amino acid sequences as set forth in SEQ ID NO: 43, SEQ ID NO: 46, and SEQ ID NO: 22, respectively.

15. The multispecific antibody according to claim 13 or 14, wherein the common light chain comprises a sequence as set forth in SEQ ID NO: 47, 51, 52, or 53.

16. The multispecific antibody according to any one of claims 7 to 9, 12 to 15, wherein the multispecific antibody is a common light chain bispecific antibody, comprising:
i) peptide chain II-A, which is a common light chain and comprises a VL of the antigen-binding domain targeting CD3 and a light chain constant region (CL); preferably, the light chain constant region is kappa;
ii) peptide chain II-B, which comprises a VH of the antigen-binding domain targeting CD3 and a first heavy chain constant region (CH); preferably, the first heavy chain constant region is IgG, such as IgG1, IgG2, IgG3 or IgG4; and
iii) peptide chain II-C, which comprises a VH of the antigen-binding domain targeting TAA or TSA and a second CH; preferably, the second CH is IgG, such as IgG1, IgG2, IgG3 or IgG4;
preferably, the Fc domains comprised in the peptide chains II-B and II-C comprise modifications to promote dimerization of II-B and II-C, respectively;
preferably, (1) the peptide chain II-A comprises a VL sequence as set forth in SEQ ID NO: 47, or a variant thereof, and the peptide chain II-B comprises a VH sequence as set forth in SEQ ID NO: 57, or a variant thereof;
(2) the peptide chain II-A comprises a VL sequence as set forth in SEQ ID NO: 47, or a variant thereof, and the peptide chain II-B comprises a VH sequence as set forth in SEQ ID NO: 58, or a variant thereof;
(3) the peptide chain II-A comprises a VL sequence as set forth in SEQ ID NO: 51, or a variant thereof, and the peptide chain II-B comprises a VH sequence as set forth in SEQ ID NO: 58, or a variant thereof;
(4) the peptide chain II-A comprises a VL sequence as set forth in SEQ ID NO: 52, or a variant thereof, and the peptide chain II-B comprises a VH sequence as set forth in SEQ ID NO: 60, or a variant thereof;
(5) the peptide chain II-A comprises a VL sequence as set forth in SEQ ID NO: 53, or a variant thereof, and the peptide chain II-B comprises a VH sequence as set forth in SEQ ID NO: 60, or a variant thereof;
(6) the peptide chain II-A comprises a VL sequence as set forth in SEQ ID NO: 52, or a variant thereof, and the peptide chain II-B comprises a VH sequence as set forth in SEQ ID NO: 61, or a variant thereof; or
(7) the peptide chain II-A comprises a VL sequence as set forth in SEQ ID NO: 53, or a variant thereof, and the peptide chain II-B comprises a VH sequence as set forth in SEQ ID NO: 61, or a variant thereof;
the variant in any one of (1) to (7) has at least 95%, 96%, 97%, 98%, or 99% sequence identity to the sequence from which it is derived;
more preferably, the peptide chain II-C comprises a VH sequence as set forth in SEQ ID NO: 70 or a variant thereof; and the variant has at least 95%, 96%, 97%, 98%, or 99% sequence identity to the sequence from which it is derived.

17. An isolated nucleic acid molecule, which encodes:
- the antibody or antigen-binding fragment thereof according to any one of claims 1 to 6, or the heavy chain variable region and/or light chain variable region thereof; or
- the multispecific antibody according to any one of claims 7 to 16, or the polypeptide chain thereof.

18. A vector, which comprises the isolated nucleic acid molecule according to claim 17.

19. A host cell, which comprises the isolated nucleic acid molecule according to claim 17 or the vector according to claim 18.

20. A method for preparing an antibody or antigen-binding fragment thereof, or a multispecific antibody, comprising culturing the host cell according to claim 19 under conditions allowing protein expression, and recollecting the antibody or antigen-binding fragment thereof, or the multispecific antibody, from a culture of the cultured host cell.

21. A pharmaceutical composition, which comprises the antibody or antigen-binding fragment thereof according to any one of claims 1 to 6, the multispecific antibody according to any one of claims 7 to 16, the isolated nucleic acid molecule according to claim 17, the vector according to claim 18, or the host cell according to claim 19, and a pharmaceutically acceptable carrier and/or excipient.

22. Use of the antibody or antigen-binding fragment thereof according to any one of claims 1 to 6, the multispecific antibody according to any one of claims 7 to 16, the isolated nucleic acid molecule according to claim 17, the vector according to claim 18, the host cell according to claim 19, or the pharmaceutical composition according to claim 21, in the manufacture of a medicament for the prevention and/or treatment of a disease.

23. A method for preventing and/or treating a disease, comprising administering to a subject in need an effective dose of the antibody or antigen-binding fragment thereof according to any one of claims 1 to 6, the multispecific antibody according to any one of claims 7 to 16, the isolated nucleic acid molecule according to claim 17, the vector according to claim 18, or the host cell according to claim 19, or the pharmaceutical composition according to claim 21.

24. The use according to claim 22 or the method according to claim 23, wherein the disease is a tumor, an inflammatory disease, or an autoimmune disease; preferably, the tumor is selected from the group consisting of ovarian cancer, breast cancer, gastric cancer, cholangiocarcinoma, colorectal cancer, lung cancer, acute myeloid leukemia, chronic lymphocytic leukemia, melanoma, and colorectal cancer.
